# EUROPEAN PATENT SPECIFICATION

(11) **EP 1 781 297 B1**
(45) Date of publication and mention of the grant of the patent: **15.10.2008**
(21) Application number: 05783919.3
(22) Date of filing: 12.08.2005
(51) Int. Cl.: A61K 31/5375, C07C 275/28, C07D 295/14, A61P 17/00

(54) **NOVEL BIAROMATIC COMPOUNDS THAT ACTIVATE PPAR TYPE RECEPTORS, AND USE THEREOF IN COSMETIC OR PHARMACEUTICAL COMPOSITIONS**
NEUARTIGE BIAROMATISCHE PPAR-REZEPTOREN AKTIVIERENDE ZUSAMMENSETZUNGEN UND IHRE VERWENDUNG IN KOSMETISCHEN ODER PHARMAZEUTISCHEN ZUSAMMENSETZUNGEN
NOUVEAUX COMPOSES BIOAROMATIQUES ACTIVANT DES RECEPTEURS DE TYPE PPAR, ET LEUR UTILISATION DANS DES COMPOSITIONS PHARMACEUTIQUES OU COSMETIQUES

(30) Priority: 17.08.2004 FR 0408933; 08.09.2004 US 607781 P
(43) Date of publication of application: 09.05.2007
(73) Proprietor: Galderma Research & Development, 06410 Biot (FR)
(72) Inventor: CLARY, Laurence, F-06480 La Colle sur Loup (FR); BOITEAU, Jean-Guy, F-34130 Saint-Aunes (FR); MILLOIS BARBUIS, Corinne, 83700 Saint Raphael (FR)
(74) Representative: Allab, Myriam
(86) International application number: PCT/EP2005/009990
(87) International publication number: WO 2006/018326

(56) References cited:
- WO-A-01/16120
- WO-A-02/12210
- WO-A-03/055867
- DATABASE BEILSTEIN BEILSTEIN INSTITUTE FOR ORGANIC CHEMISTRY, FRANKFURT-MAIN, DE; XP002358643 & K. URBAHNS ET AL.: BIOORG.MED.CHEM.LETT., vol. 13, no. 6, 2003, pages 1071-1074,
- DATABASE BEILSTEIN BEILSTEIN INSTITUTE FOR ORGANIC CHEMISTRY, FRANKFURT-MAIN, DE; XP002358644 & KLAUS URBAHNS ET ALL.: BIOORG.MED.CHEM.LETT., vol. 13, no. 6, 2003, pages 1071-1074,
- DATABASE BEILSTEIN BEILSTEIN INSTITUTE FOR ORGANIC CHEMISTRY, FRANKFURT-MAIN, DE; XP002358645 & KRISTJAN S., GUDMUNDSSON: BIOORG. MED.CHEM, vol. 10, no. 6, 2002, pages 2051-2066,

## Description

The invention concerns, as novel and useful industrial products, a novel class of biaromatic compounds that activate the Peroxisome Proliferator-Activated Receptor type receptors of subtype γ (PPARγ). The invention also concerns the process for preparing them and their use in pharmaceutical compositions for use in human or veterinary medicine, or alternatively in cosmetic compositions.

The activity of receptors of PPAR type has been the subject of numerous studies. Mention may be made, as a guide, of the publication entitled "Differential Expression of Peroxisome Proliferator-Activated Receptor Subtypes During the Differentiation of Human Keratinocytes", Michel Rivier et al., J. Invest. Dermatol. 111, 1998, pp. 1116-1121, in which is listed a large number of bibliographic references relating to PPAR type receptors. Mention may also be made, as a guide, of the file entitled "The PPARs: From orphan receptors to Drug Discovery", Timothy M. Willson, Peter J. Brown, Daniel D. Sternbach, and Brad R. Henke, J. Med. Chem., 2000, Vol. 43, pp. 527-550.

The PPAR receptors activate transcription by binding to DNA sequence elements known as the peroxisome proliferator response elements (PPRE), in the form of a heterodimer with the retinoid X receptors (known as RXRs).

Three subtypes of human PPAR have been identified and described: PPARα, PPARγ and PPARδ (or NUC1).

PPARα is mainly expressed in the liver, whereas PPARδ is ubiquitous.

Of the three subtypes, PPARγ is the one that has been the most extensively studied. All the references suggest a critical role of PPARγ in the regulation of differentiation of adipocytes, where it is strongly expressed. It also plays a key role in systemic lipid homeostasis.

It has especially been described in patent application WO 96/33724 that PPARγ-selective compounds, such as a prostaglandin-J2 or -D2, are potential active agents for the treatment of obesity and diabetes.

Moreover, the Applicant has already described in patent applications WO 02/12210 and WO 03/055867 the use of biaromatic compounds that activate PPARγ type receptors in the preparation of a pharmaceutical composition, the composition being intended for treating skin disorders associated with an anomaly of epidermal cell differentiation.

It nevertheless remains that it is necessary to search for novel compounds that have good activity and advantageous pharmaceutical properties.

The Applicant has now identified a novel family of propanoic derivatives that have the advantage of being 10 to 100 times more active than the compounds identified in the preceding patent applications WO 02/12210 and WO 03/055867, towards the PPAR gamma receptors. Moreover, in addition to their better activity, some of the compounds according to the present invention are obtained in solid form. Their synthesis and their purification are easier.

Finally, the use of compounds in solid form makes it possible to avoid the drawbacks of oils in the context of pharmaceutical development on account of the residual solvents they may contain.

Thus, the present invention relates to compounds corresponding to the general formula (I) below: 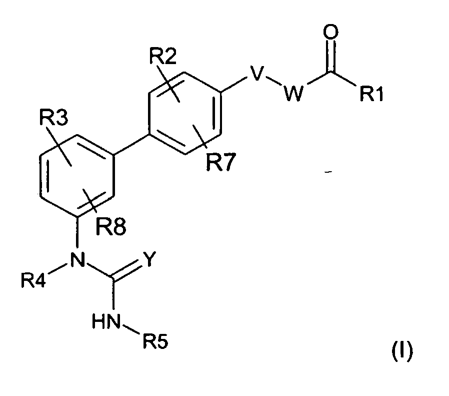 in which:
- **R1** represents a hydroxyl radical, a radical -OR6 or a hydroxylamine radical; R6 being defined below,
- **R2** and **R3,** which may be identical or different, represent a hydrogen atom, a halogen atom, an alkyl radical of 1 to 12 carbon atoms, a hydroxyl radical, an alkoxy radical, a polyether radical, an aralkyl radical, an aryl radical, an amino radical that may be substituted with one or two radicals, which may be identical or different, chosen from an alkyl radical of 1 to 12 carbon atoms and an aralkyl radical;
- **R4** represents a hydrogen atom or a lower alkyl radical containing from 1 to 4 carbon atoms;
- **R5** represents an alkyl radical containing from 1 to 12 carbon atoms, an aryl radical, an aralkyl radical, a heteroaryl radical or a heterocyclic radical;
- **R6** represents an alkyl, aryl or aralkyl radical;
- **R7** and **R8,** which may be identical or different, represent a hydrogen atom, a halogen atom, an alkyl radical of 1 to 12 carbon atoms, a hydroxyl radical, an alkoxy radical, a polyether radical, an aralkyl radical, an aryl radical, an amino radical that may be substituted with one or two radicals, which may be identical or different, chosen from an alkyl radical of 1 to 12 carbon atoms or an aralkyl radical;
- **Y** represents an oxygen or sulfur atom;
- **V-W** represents a sequence CH₂-CH₂ or CH=CH
and also the salts of the compounds of formula (I).

In particular, when the compounds according to the invention are in the form of salts, they are salts of an alkali metal, in particular a sodium or potassium salt, or an alkaline-earth metal salt, or salts of organic amines, more particularly of amino acids such as arginine or lysine.

When the compounds according to the invention contain an amine function and are in the form of salts of this amine, these are salts of a mineral acid, for instance hydrochloric acid, sulfuric acid or hydrobromic acid, or salts of an organic acid, for instance acetic acid, triflic acid, tartaric acid, oxalic acid, citric acid or nitric acid.

According to the present invention, the term "alkyl radical of 1 to 12 carbon atoms" means a linear, branched or cyclic, saturated or unsaturated carbon-based chain that may be interrupted with a hetero atom and that may be substituted with one or more radicals chosen from a halogen atom, a hydroxyl radical, an alkoxy radical and a heterocyclic radical, and preferably the alkyl radicals are methyl, ethyl, propyl, isopropyl, butyl, tert-butyl, isoamyl, amyl, hexyl, heptyl, octyl, decyl, cyclohexyl, methylcyclohexyl, methylcyclobutyl, methylcyclopentyl or methylcyclohexyl radicals.

The term "lower alkyl radical of 1 to 4 carbon atoms" will preferably be a methyl, ethyl, propyl, methylcyclopropyl, isopropyl, tert-butyl or n-butyl radical.

The term "aryl radical" means a phenyl, biphenyl, cinnamyl or naphthyl radical that may be substituted with a halogen atom, a CF₃ radical, an alkyl radical of 1 to 12 carbon atoms, an alkoxy radical, a nitro function, a polyether radical, an aryl radical, a benzoyl radical, an alkyl ester group, a carboxylic acid, a hydroxyl radical optionally protected with an acetyl or benzoyl group or an amino radical optionally protected with an acetyl or benzoyl group or optionally substituted with at least one alkyl containing from 1 to 12 carbon atoms, an aralkoxy radical, a phenoxy radical or an amide radical H2NCO.

The term "aralkyl radical" means an alkyl radical of 1 to 12 carbon atoms substituted with an aryl radical or with a heteroaryl radical.

The term "hydroxylamine radical" means the sequence -NHOH.

The term "halogen atom" means a fluorine, chlorine, bromine or iodine atom.

According to the present invention, the term "hydroxyl radical" means the -OH radical.

The term "alkoxy radical" means an oxygen atom substituted with an alkyl radical of 1 to 12 carbon atoms, and the alkoxy radicals are preferably methoxy, ethoxy, isopropyloxy, n-propyloxy, tert-butoxy, n-butoxy, n-pentyloxy, n-hexyloxy, cyclopropylmethoxy, cyclobutylmethoxy, cyclopentylmethoxy or cyclohexylmethoxy radicals.

The term "aralkoxy radical" means an oxygen atom substituted with an aralkyl radical.

The term "polyether radical" means a radical containing from 1 to 7 carbon atoms interrupted with at least one oxygen atom, and preferably radicals such as methoxyethoxy, ethoxyethoxy or methoxyethoxyethoxy radicals.

The term "heteroaryl radical" means an aryl radical interrupted with one or more hetero atoms, such as a pyridyl, furyl, thienyl, isoxazolyl, oxadiazolyl, oxazolyl, isothiazolyl, quinazolinyl, benzothiadiazolyl, benzimidazole, indolyl or benzofuran radical, optionally substituted with at least one halogen, an alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms.

The term "heterocyclic radical" preferably means a morpholino, pyrrolidino, piperidino, piperazino, 2-oxopiperid-1-yl or 2-oxopyrrolidin-1-yl radical, optionally substituted with at least one alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms.

The term "alkyl ester radical" means a carboxylate function substituted with an alkyl radical containing from 1 to 6 carbon atoms.

Among the compounds of formula (I) above that fall within the context of the present invention, mention may be made especially of the following (alone or as a mixture):
1. (E)-3-[3'-(3-heptyl-1-methylureido)biphenyl-4-yl]acrylic acid
2. 3-[3'-(3-heptyl-1-methylureido)biphenyl-4-yl]propanoic acid
3. 3-[3-fluoro-3'-(3-heptyl-1-methylureido)biphenyl-4-yl]propanoic acid
4. 3-(3'-{3-[2-(4-fluorophenyl)ethyl]-1-methylureido}biphenyl-4-yl)propanoic acid
5. 3-[3'-(1-methyl-3-pentylureido)biphenyl-4-yl]propanoic acid
6. 3-[3'-(3-heptyl-1-methylureido)biphenyl-4-yl]-N-hydroxypropionamide
7. 3-[3'-(1-methyl-3-naphthalen-2-ylureido)biphenyl-4-yl]propanoic acid
8. 3-[3'-(3-heptyl-1-methylthioureido)biphenyl-4-yl]propanoic acid
9. (E)-3-[2-fluoro-3'-(3-heptyl-1-methylureido)biphenyl-4-yl]acrylic acid
10. 3-[2-fluoro-3'-(3-heptyl-1-methylureido)biphenyl-4-yl]propanoic acid
11. 3-[3'-(3-hexyl-1-methylureido)biphenyl-4-yl]propanoic acid
12. 3-[3'-(3-octyl-1-methylureido)biphenyl-4-yl]propanoic acid
13. 3-{3'-[3-(4-benzyloxyphenyl)-1-methylureido]biphenyl-4-yl}propanoic acid
14. 3-{3'-[3-(4-butylphenyl)-1-methylureido]biphenyl-4-yl}propanoic acid
15. 3-[3'-(3-heptyl-1-methylureido)-2-(2-methoxyethoxy)biphenyl-4-yl]propanoic acid
16. 3-[3'-(3-heptyl-1-methylureido)-2-(3-methylbutoxy)biphenyl-4-yl]propanoic acid
17. 3-[2-(3-chloropropoxy)-3'-(3-heptyl-1-methylureido)biphenyl-4-yl]propanoic acid
18. 3-[3'-(3-heptyl-1-methylureido)-2-methoxybiphenyl-4-yl]propanoic acid
19. 3-[3'-(3-heptyl-1-methylureido)-2-methylbiphenyl-4-yl]propanoic acid
20. 3-[3'-[1-methyl-3-(3-phenylpropyl)ureido]biphenyl-4-yl]propanoic acid
21. (E)-3-[5'-(3-heptyl-1-methylureido)-2'-methylbiphenyl-4-yl]acrylic acid
22. 3-[5'-(3-heptyl-1-methylureido)-2'-methylbiphenyl-4-yl]propanoic acid
23. (E)-3-[3-fluoro-3'-(3-heptyl-1-methylureido)biphenyl-4-yl]acrylic acid
24. (E)-3-[2-benzyloxy-3'-(3-heptyl-1-methylureido)biphenyl-4-yl]acrylic acid
25. 3-[3'-(3-heptyl-1-methylureido)-2-propoxybiphenyl-4-yl]propanoic acid
26. 3-[2-butoxy-3'-(3-heptyl-1-methylureido)biphenyl-4-yl]propanoic acid
27. (E)-3-[3'-(1-ethyl-3-heptylureido)biphenyl-4-yl]acrylic acid
28. (E)-3-[3'-(3-heptyl-1-propylureido)biphenyl-4-yl]acrylic acid
29. (E)-3-[3,5-difluoro-3'-(3-heptyl-1-methylureido)biphenyl-4-yl]acrylic acid
30. 3-[3'-(1-ethyl-3-heptylureido)biphenyl-4-yl]propanoic acid
31. 3-[3'-(3-heptyl-1-propylureido)biphenyl-4-yl]propanoic acid
32. 3-[3,5-difluoro-3'-(3-heptyl-1-methylureido)biphenyl-4-yl]propanoic acid
33. 3-{3'-[3-(4-butoxyphenyl)-1-methylureido]biphenyl-4-yl}propanoic acid
34. 3-{3'-[3-(4-butoxyphenyl)-1-ethylureido]biphenyl-4-yl}propanoic acid
35. 3-[2-cyclopropylmethoxy-3'-(3-heptyl-1-methylureido)biphenyl-4-yl]propanoic acid
36. 3-[2-ethoxy-3'-(3-heptyl-1-methylureido)biphenyl-4-yl]propanoic acid
37. 3-[3'-(3-heptyl-1-methylureido)-2-(3,3,3-trifluoropropoxy)biphenyl-4-yl]propanoic acid
38. 3-[3'-(3-heptyl-1-methylureido)-2-(4,4,4-trifluorobutoxy)biphenyl-4-yl]propanoic acid
39. 3-[3'-(3-heptyl-1-methylureido)-2-(3-hydroxypropoxy)biphenyl-4-yl]propanoic acid
40. 3-[3'-(3-heptyl-1-methylureido)-2-(4-hydroxybutoxy)biphenyl-4-yl]propanoic acid
41. 3-[2-butoxy-3'-(1-methyl-3-pentylureido)biphenyl-4-yl]propanoic acid
42. 3-[2-benzyloxy-3'-(3-heptyl-1-methylureido)biphenyl-4-yl]propanoic acid
43. 3-[2-(3-fluorobenzyloxy)-3'-(3-heptyl-1-methylureido)biphenyl-4-yl]propanoic acid
44. 3-[2-(4-fluorobenzyloxy)-3'-(3-heptyl-1-methylureido)biphenyl-4-yl]propanoic acid
45. 3-[3'-(3-heptyl-1-methylureido)-2-pentyloxybiphenyl-4-yl]propanoic acid
46. 3-[3'-(1-methyl-3-pentylureido)-2-pentyloxybiphenyl-4-yl]propanoic acid
47. 3-[2-(2-ethoxyethoxy)-3'-(3-heptyl-1-methylureido)biphenyl-4-yl]propanoic acid
48. 3-[2-butoxy-3'-(1-methyl-3-phenylureido)biphenyl-4-yl]propanoic acid
49. 3-[2-(2-ethoxyethoxy)-3'-(1-methyl-3-pentylureido)biphenyl-4-yl]propanoic acid
50. 3-[2-(2-diethylaminoethoxy)-3'-(3-heptyl-1-methylureido)biphenyl-4-yl]propanoic acid hydrochloride
51. 3-[3'-(3-heptyl-1-methylureido)-2-(2-morpholin-4-ylethoxy)biphenyl-4-yl]propanoic acid
52. 3-[2-amino-3'-(1-methyl-3-pentylureido)biphenyl-4-yl]propanoic acid
53. 3-[2-butylamino-3'-(1-methyl-3-pentylureido)biphenyl-4-yl]propanoic acid hydrochloride
54. 3-[2-benzylamino-3'-(1-methyl-3-pentylureido)biphenyl-4-yl]propanoic acid hydrochloride
55. 3-[2-diethylamino-3'-(1-methyl-3-pentylureido)biphenyl-4-yl]propanoic acid hydrochloride
56. 3-[3'-(1-methyl-3-pentylureido)-2-propylaminobiphenyl-4-yl]propanoic acid hydrochloride
57. 3-[2-(4-fluorobenzylamino)-3'-(1-methyl-3-pentylureido)biphenyl-4-yl]propanoic acid hydrochloride
58. 3-[2-butylamino-3'-(3-heptyl-1-methylureido)biphenyl-4-yl]propanoic acid hydrochloride
59. 3-[2-benzylamino-3'-(3-heptyl-1-methylureido)biphenyl-4-yl]propanoic acid hydrochloride
60. 3-[2-diethylamino-3'-(3-heptyl-1-methylureido)biphenyl-4-yl]propanoic acid hydrochloride
61. 3-[3'-(3-heptyl-1-methylureido)-2-propylaminobiphenyl-4-yl]propanoic acid hydrochloride
62. 3-[2-(4-fluorobenzylamino)-3'-(3-heptyl-1-methylureido)biphenyl-4-yl]propanoic acid hydrochloride
63. 3-[3'-(1-methyl-3-pentylureido)biphenyl-4-yl]acrylic acid
64. 3-[3'-(3-pentyl-1-propylureido)biphenyl-4-yl]acrylic acid
65. 3-[4'-fluoro-3'-(1-methyl-3-pentylureido)biphenyl-4-yl]propanoic acid
66. ethyl 3-[3'-(1-methyl-3-pentylureido)biphenyl-4-yl]acrylate
67. 3-[3'-(1-methyl-3-naphthalen-2-ylureido)biphenyl-4-yl]acrylic acid
68. 3-{3'-[3-(4-fluorophenyl)-1-methylureido]biphenyl-4-yl}acrylic acid
69. 3-(3'-{3-[2-(4-fluorophenyl)ethyl]-1-methylureido}biphenyl-4-yl)acrylic acid
70. 3-[3'-(3-benzyl-1-methylureido)biphenyl-4-yl]propanoic acid
71. 3-[3'-(3-cyclopropylmethyl-1-methylureido)biphenyl-4-yl]propanoic acid
72. 3-[3'-(3-cyclohexyl-1-methylureido)biphenyl-4-yl]propanoic acid
73. 3-[3'-(3-cyclohexyl-1-methylureido)biphenyl-4-yl]acrylic acid
74. 3-[3'-(3-cyclopropylmethyl-1-methylureido)biphenyl-4-yl]acrylic acid
75. ethyl 4-{3-[4'-(2-carboxyethyl)biphenyl-3-yl]-3-methylureido}piperidine-1-carboxylate
76. 3-[3'-(1-methyl-3-pyrid-3-ylureido)biphenyl-4-yl]acrylic acid
77. 3-[3'-(1-methyl-3-pyrid-3-ylureido)biphenyl-4-yl]propanoic acid
78. 3-{3'-[3-(6-methoxypyrid-3-yl)-1-methylureido]biphenyl-4-yl}acrylic acid
79. 3-[3'-(1-methyl-3-propylureido)biphenyl-4-yl]acrylic acid
80. 3-[3'-(3-hexyl-1-methylthioureido)biphenyl-4-yl]propanoic acid
81. 3-[3'-(3-hexyl-1-methylthioureido)biphenyl-4-yl]acrylic acid
82. methyl 3-[3'-(3-Heptyl-1-methylthioureido)biphenyl-4-yl]acrylate
83. 3-[2-methyl-3'-(1-methyl-3-pentylureido)biphenyl-4-yl]propanoic acid
84. 3-[3-hydroxy-3'-(1-methyl-3-pentylureido)biphenyl-4-yl]propanoic acid
85. 3-[3-methoxymethoxy-3'-(1-methyl-3-pentylureido)biphenyl-4-yl]propanoic acid
86. 3-[3'-(1-methyl-3-pentylureido)-2-trifluoromethylbiphenyl-4-yl]propanoic acid
87. 3-[3'-(3-heptyl-1-methylureido)-3-methoxybiphenyl-4-yl]propanoic acid
88. 3-{3'-[1-methyl-3-(4-propylphenyl)ureido]biphenyl-4-yl}propanoic acid
89. 3-{3'-[1-methyl-3-(4-propylphenyl)ureido]biphenyl-4-yl}acrylic acid
90. phenyl 3-{3'-[1-methyl-3-(4-propylphenyl)ureido]biphenyl-4-yl}acrylate
91. benzyl 3-{3'-[1-methyl-3-(4-propylphenyl)ureido]biphenyl-4-yl}acrylate
92. 3-[3'-(3-pentylureido)biphenyl-4-yl]acrylic acid
93. N-hydroxy-3-{3'-[1-methyl-3-(3-phenylpropyl)ureido]biphenyl-4-yl}propionamide
94. 3-[3'-(3-heptyl-1-methylureido)-2-hydroxybiphenyl-4-yl]propanoic acid
95. 3-[2-cyclohexylmethoxy-3'-(3-heptyl-1-methylureido)biphenyl-4-yl]propionic acid
96. 3-[2-cyclopentylmethoxy-3'-(3-heptyl-1-methylureido)biphenyl-4-yl]propionic acid
97. 3-[2-cyclobutylmethoxy-3'-(3-heptyl-1-methylureido)biphenyl-4-yl]-N-hydroxypropionamide
98. 3-[3'-(3-heptyl-1-methylureido)-2-(3-trifluoromethylbenzyloxy)biphenyl-4-yl]propanoic acid
99. 3-[3'-(3-heptyl-1-methylureido)-2-(4-trifluoromethylbenzyloxy)biphenyl-4-yl]propanoic acid
100. 3-[2-(3-carbamoylbenzyloxy)-3'-(3-heptyl-1-methylureido)biphenyl-4-yl]propanoic acid
101. 3-[3'-(3-heptyl-1-methylureido)-2-(2-piperazin-1-ylethoxy)biphenyl-4-yl]propanoic acid
102. 3-[3'-(3-heptyl-1-methylureido)-2-(2-pyrrolidin-1-ylethoxy)biphenyl-4-yl]propanoic acid
103. 3-(2-(3-methoxybenzyloxy)-3'-{3-[2-(3-methoxyphenyl)ethyl]-1-methylureido}biphenyl-4-yl)propanoic acid
104. 3-(2-(4-tert-butylbenzyloxy)-3'-{1-methyl-3-[2-(3-phenoxyphenyl)ethyl]-ureido}biphenyl-4-yl)propanoic acid
105. 3-{2-(3,5-dimethoxybenzyloxy)-3'-[1-methyl-3-(3-phenoxyphenyl)ureido]biphenyl-4-yl}propanoic acid
106. 3-[3'-[1-methyl-3-(4-phenoxyphenyl)ureido]-2-(3-trifluoromethylbenzyloxy)biphenyl-4-yl]propanoic acid
107. 3-(2-(3-isopropoxybenzyloxy)-3'-{3-[2-(4-methoxyphenyl)ethyl]-1-methylureido}biphenyl-4-yl)propanoic acid
108. 3-(2'-(3-methoxybenzyloxy)-5'-{3-[2-(3-methoxyphenyl)ethyl]-1-methylureido}biphenyl-4-yl)propanoic acid
109. 3-[2'-cyclohexylmethoxy-5'-(1-methyl-3-pentylureido)biphenyl-4-yl]propanoic acid
110. 3-[4'-ethoxy-3'-(1-methyl-3-pentylureido)-2-propoxybiphenyl-4-yl]propanoic acid
111. 3-[3,5-dimethoxy-3'-(1-methyl-3-pentylureido)biphenyl-4-yl]propanoic acid
112. 3-(3,5-diethoxy-3'-{3-[2-(3-methoxyphenyl)ethyl]-1-methylureido}biphenyl-4-yl)propanoic acid
113. 3-(3'-{3-[2-(4-methoxyphenyl)ethyl]-1-methylureido}-3-propoxybiphenyl-4-yl)propanoic acid
114. 3-[3-cyclopropylmethoxy-3'-(1-methyl-3-pentylureido)biphenyl-4-yl]propanoic acid
115. 3-[3-ethoxy-4'-fluoro-3'-(1-methyl-3-pentylureido)biphenyl-4-yl]propanoic acid
116. 3-[3'-(1-methyl-3-pentylureido)-3-(4,4,4-trifluorobutoxy)biphenyl-4-yl]propanoic acid
117. 3-[3-benzyloxy-3'-(1-methyl-3-pentylureido)biphenyl-4-yl]propanoic acid
118. 3-[3'-{3-[2-(4-methoxyphenyl)ethyl]-1-methylureido}-3-(3-trifluoromethylbenzyloxy)-biphenyl-4-yl]propanoic acid
119. 3-(3'-{3-[2-(3-methoxyphenyl)ethyl]-1-methylureido}-3,5-dipropylbiphenyl-4-yl)propanoic acid
120. 3-[3-(2,2-dimethylpropyl)-3'-(1-methyl-3-pentylureido)biphenyl-4-yl]propanoic acid
121. 3-[3,5-dimethyl-3'-(1-methyl-3-pentylureido)biphenyl-4-yl]propanoic acid
122. 3-[4"-methoxy-3-(1-methyl-3-pentylureido)[1,1';3',1"]terphenyl-4'-yl]propanoic acid
123. 3-[3"-methoxy-3-(1-methyl-3-pentylureido)[1,1';2',1"]terphenyl-4'-yl]propanoic acid
124. 3-(3-{3-[2-(3-methoxyphenyl)ethyl]-1-methylureido}-3"-trifluoromethyl[1,1';2',1"]terphenyl-4'-yl)propanoic acid
125. 3-{3'-(3-butyl-1-methylureido)-2-[2-(3-isopropoxyphenyl)ethyl]biphenyl-4-yl}propanoic acid
126. 3-{3'-(1-methyl-3-pentylureido)-2-[(pyrid-3-ylmethyl)amino]biphenyl-4-yl}propanoic acid
127. 3-[3-(2-methoxyethylamino)-3'-(1-methyl-3-pentylureido)biphenyl-4-yl]propanoic acid
128. methyl 3-[3,5-diethyl-3'-(1-methyl-3-pentylureido)biphenyl-4-yl]propanoate
129. methyl 3-[3'-[1-methyl-3-(3-phenoxyphenyl)ureido]-2-(3-trifluoromethylbenzyloxy)-biphenyl-4-yl]propanoate
130. methyl 3-[3'-[3-(2-biphenyl-4-ylethyl)-1-methylureido]-2-(3-methoxybenzyloxy)-biphenyl-4-yl]propanoate
131. ethyl 3-[3'-{3-[2-(3-methoxyphenyl)ethyl]-1-methylureido}-2-(4,4,4-trifluorobutoxy)-biphenyl-4-yl]propanoate.

According to the present invention, the compounds of formula (I) that are more particularly preferred are those that have at least one of the following characteristics:
- R1 represents a hydroxyl or hydroxylamine radical;
- R2 and R7 represent an alkoxy or aryloxy radical, an alkylamino radical or a polyether radical;
- R3 and R8 represent a hydrogen atom;
- R4 represents a lower alkyl radical of 1 to 4 carbon atoms;
- R5 represents an alkyl radical of 3 to 8 carbon atoms;
- Y is an oxygen atom;
- the bond V-W is a CH₂-CH₂ or CH=CH.

In particular, the compounds of general formula (I) that have all of the following characteristics will be preferred:
- R1 represents a hydroxyl or hydroxylamine radical;
- R2 and R7 represent an alkoxy or aryloxy radical, an alkylamino radical or a polyether radical;
- R3 and R8 represent a hydrogen atom;
- R4 represents a lower alkyl radical of 1 to 4 carbon atoms;
- R5 represents an alkyl radical of 3 to 8 carbon atoms;
- Y is an oxygen atom;
- the bond V-W is CH₂-CH₂ or CH=CH.

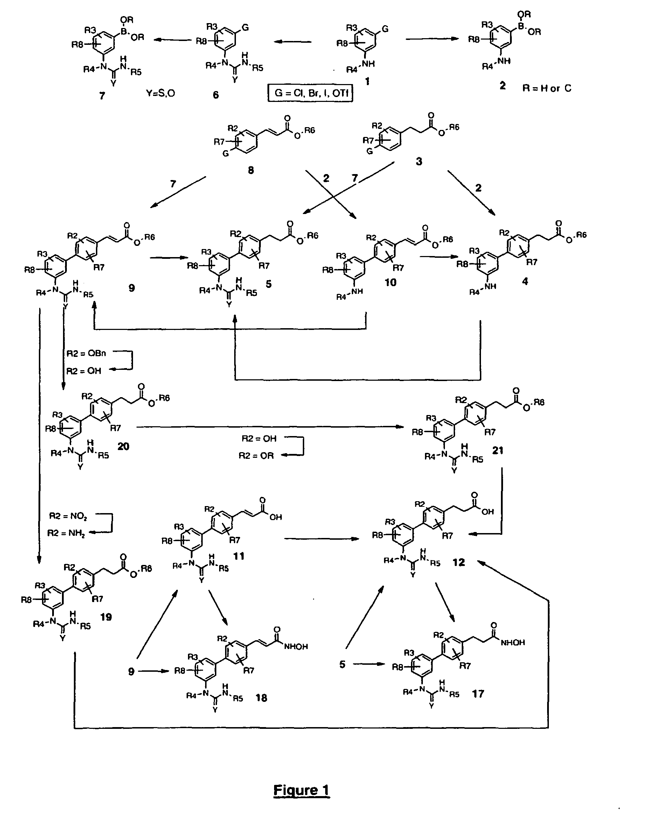

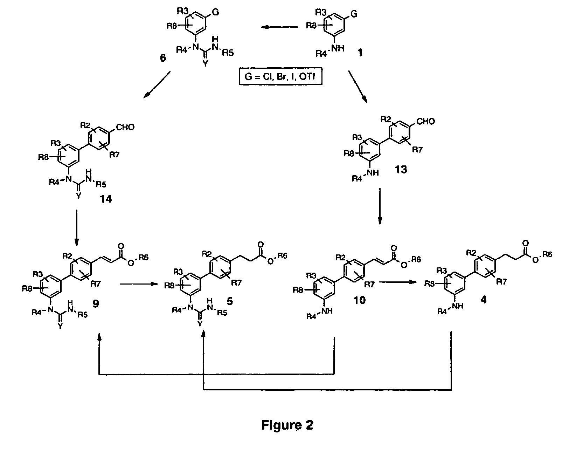

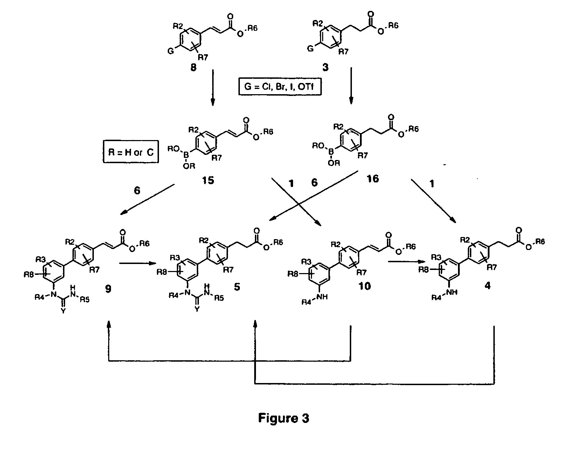

A subject of the present invention is also the processes for preparing the compounds of formula (I), in particular according to the reaction schemes given in **Figures 1****,** **2** **and** **3****.**

### Figure 1 :

The boronic acid **2** may be obtained from compound **1** using standard conditions, for example by reaction with tert-butyllithium, followed by an addition to trimethyl borate.

A Suzuki type palladium coupling between the boronic acid **2** and compound **3** (chosen from an aryl bromide, iodide, chloride or triflate) makes it possible to obtain the compound having the aryl-aryl sequence **4.**

A Suzuki type palladium coupling between the boronic acid **2** and compound **8** (chosen from an aryl bromide, iodide, chloride or triflate) makes it possible to obtain the compound having the aryl-aryl sequence **10.**

Compound **4** may be coupled with isocyanates or isothiocyanates under standard conditions to give compound **5.**

Compound **10** may be coupled with isocyanates or isothiocyanates under standard conditions to give compound **9.**

When it is desired to obtain a compound with **R1** being a hydroxyl radical (compound 12), the acid function may be obtained from compound 5 either:
- by saponification, if R6 is an alkyl chain, with a base such as sodium hydroxide; or
- by hydrogenolysis if R6 is a benzyl; or
- by deprotection with palladium if R6 is an allyl chain.
   Compound **1** may also be coupled directly with an isocyanate or an isothiocyanate under standard conditions to give compound **6.**
   The boronate **7** may be obtained by treating compound **6** with pinacol diborane, for example in the presence of a palladium-based catalyst, for instance diphenylphosphinoferrocenepalladium dichloride.
   A Suzuki type palladium coupling between the boronate **7** and compound **8** (chosen from an aryl bromide, iodide, chloride or triflate) makes it possible to obtain the compound having the aryl-aryl sequence **9.**
   A Suzuki type palladium coupling between the boronate **7** and compound **3** (chosen from an aryl bromide, iodide, chloride or triflate) makes it possible to obtain the compound having the aryl-aryl sequence **5.**
   Compound **5** may be obtained by hydrogenation of compound **9** under standard hydrogenation conditions, for instance: hydrogen catalysed with palladium-on-charcoal.
   When R2 is a benzyloxy, compound **20** may be obtained from **9** under standard hydrogenation conditions, for instance: hydrogen catalysed with palladium-on-charcoal.
   Compound **21** may be obtained from **20** by alkylation of the phenol, for example using a base such as potassium carbonate in the presence of an alkyl halide.
   The acid function of compound **12** may be obtained from **21** by saponification if R6 is an alkyl chain, with a base, for instance sodium hydroxide.
   When R2 is a nitro, compound **9** may be converted into the amine **19** by hydrogenation, for example in the presence of palladium-on-charcoal and hydrogen.
   Compound **19** may then react with aldehydes in the presence of sodium borohydride to form alkylamino or dialkylamino compounds, which may then be saponified into the corresponding acids thereof **12,** for example using sodium hydroxide.
   Compound **4** may be obtained by hydrogenation of compound **10** under standard hydrogenation conditions, for instance: hydrogen catalysed with palladium-on-charcoal.
   The acid function of compound **11** may be obtained from **9:** by saponification if R6 is an alkyl chain, with a base such as sodium hydroxide; or by deprotection with palladium if R6 is an allyl chain.
   Compound **12** may be obtained by hydrogenation of compound **11** under standard hydrogenation conditions, for instance: hydrogen catalysed with palladium-on-charcoal.
   Compound **17** may be obtained from the acid **12** by treatment with oxalyl chloride, for example, followed by treatment with hydroxylamine.
   Compound **18** may be obtained from the acid **11** by treatment with oxalyl chloride, for example, followed by treatment with hydroxylamine.
   Compound **17** may be obtained from compound **5** by treatment with hydroxylamine.
   Compound **18** may be obtained from compound **9** by treatment with hydroxylamine.

### Figure 2 :

A Suzuki type palladium coupling between the 4-formylphenylboronic acids and compound **1** (chosen from an aryl bromide, iodide, chloride or triflate) makes it possible to obtain the compound having the aryl-aryl sequence **13.**

A Suzuki type palladium coupling between the 4-formylphenylboronic acids and compound **6** (chosen from an aryl bromide, iodide, chloride or triflate) makes it possible to obtain the compound having the aryl-aryl sequence **14.**

Compound **9** may be obtained via a Wittig reaction from compound **14** via the action of methyltriphenylphosphoranylidene acetate, for example.

Compound 10 may be obtained via a Wittig reaction from compound **13** via the action of methyltriphenylphosphoranylidene acetate, for example.

The remainder of the synthetic routes is as described for Figure 1.

### Figure 3 :

The boronate **15** may be obtained by treating compound **8** with pinacol diborane, for example in the presence of a palladium-based catalyst, for instance diphenylphosphinoferrocenepalladium dichloride.

The boronate **16** may be obtained by treating compound **3** with pinacol diborane, for example in the presence of a palladium-based catalyst, for instance diphenylphosphinoferrocenepalladium dichloride.

A Suzuki type palladium coupling between compound **15** and compound **6** (chosen from an aryl bromide, iodide, chloride or triflate) makes it possible to obtain the compound having the aryl-aryl sequence **9.**

A Suzuki type palladium coupling between compound **15** and compound **1** (chosen from an aryl bromide, iodide, chloride or triflate) makes it possible to obtain the compound having the aryl-aryl sequence **10.**

A Suzuki type palladium coupling between compound **16** and compound **1** (chosen from an aryl bromide, iodide, chloride or triflate) makes it possible to obtain the compound having the aryl-aryl sequence **4.**

A Suzuki type palladium coupling between compound **16** and compound **6** (chosen from an aryl bromide, iodide, chloride or triflate) makes it possible to obtain the compound having the aryl-aryl sequence **5.**

The remainder of the synthetic routes is as described for Figure 1.

The compounds according to the invention have modulatory properties on PPAR type receptors. This activity on the PPARa, d and γ receptors is measured in a transactivation test and quantified by means of the dissociation constant Kdapp (apparent), as described in Example 63.

The preferred compounds of the present invention have a dissociation constant of less than or equal to 5000 nM and advantageously less than or equal to 1000 nM. -

Preferably, the compounds are specific PPARγ type receptor modulators, i.e. they have a ratio between the Kdapp for the PPARa or PPARd receptors and the Kdapp for the PPARγ receptors, of greater than or equal to 10. Preferably, this PPARa/PPARγ or PPARd/PPARγ ratio is greater than or equal to 50 and more advantageously greater than or equal to 100.

A subject of the present invention is also, as medicaments, the compounds of formula (I) as described above.

Thus, the compounds as described above according to the invention may be used in the manufacture of a composition for regulating and/or restoring skin lipid metabolism.

The compounds according to the invention are particularly suitable in the following fields of treatment:
1) for treating dermatological complaints associated with a keratinization disorder relating to cell differentiation and proliferation, especially for treating common acne, comedones, polymorphs, acne rosacea, nodulocystic acne, acne conglobata, senile acne, and secondary acnes such as solar acne, medication-related acne or occupational acne;
2) for treating other types of keratinization disorders, especially ichthyosis, ichthyosiform conditions, Darier's disease, palmoplantar keratoderma, leukoplakia and leukoplakiform conditions, and cutaneous or mucous (buccal) lichen;
3) for treating other dermatological complaints with an inflammatory immunoallergic component, with or without cell proliferation disorder, and especially all forms of psoriasis, whether cutaneous, mucous or ungual, and even psoriatic rheumatism, or cutaneous atopy, such as eczema, or respiratory atopy, or alternatively gingival hypertrophy;
4) for treating all dermal or epidermal proliferations, whether benign or malignant, and whether of viral origin or otherwise, such as common warts, flat warts and verruciform epidermodysplasia, oral or florid papillomatoses, T lymphoma, and proliferations that may be induced by ultraviolet radiation, especially in the case of basal cell and spinal cell epithelioma, and also any precancerous skin lesion such as keratoacanthomas;
5) for treating other dermatological disorders such as immune dermatoses, such as lupus erythematosus, immune bullous diseases and collagen diseases, such as scleroderma;
6) in the treatment of dermatological or general complaints with an immunological component;
7) in the treatment of skin disorders caused by exposure to UV radiation, and also for repairing or combating ageing of the skin, whether photoinduced or chronological ageing, or for reducing actinic pigmentations and keratosis, or any pathology associated with chronological or actinic ageing, such as xerosis;
8) for combating sebaceous function disorders, such as the hyperseborrhoea of acne or simple seborrhoea;
9) for preventing or treating cicatrization disorders, or for preventing or repairing stretchmarks;
10) in the treatment of pigmentation disorders, such as hyperpigmentation, melasma, hypopigmentation or vitiligo;
11) in the treatment of lipid metabolism complaints, such as obesity, hyperlipidaemia, or non-insulin-dependent diabetes;
12) in the treatment of inflammatory complaints such as arthritis;
13) in the treatment or prevention of cancerous or precancerous conditions;
14) in the prevention or treatment of alopecia of various origins, especially alopecia caused by chemotherapy or radiation;
15) in the treatment of disorders of the immune system, such as asthma, type I sugar diabetes, multiple sclerosis or other selective dysfunctions of the immune system; and
16) in the treatment of complaints of the cardiovascular system, such as arteriosclerosis or hypertension.

A subject of the present invention is also a pharmaceutical or cosmetic composition comprising, in a physiologically acceptable medium, at least one compound of formula (I) as defined above.

A subject of the present invention is also the use of the compounds of formula (I) for manufacturing a composition for treating the abovementioned complaints, in particular for regulating and/or restoring skin lipid metabolism.

The composition according to the invention may be administered orally, enterally, parenterally, topically or ocularly. The pharmaceutical composition is preferably packaged in a form that is suitable for topical application.

Via the oral route, the composition, more particularly the pharmaceutical composition, may be in the form of tablets, gel capsules, dragees, syrups, suspensions, solutions, powders, granules, emulsions, lipid or polymeric microspheres, nanospheres or vesicles allowing a controlled release. Via the parenteral route, the composition may be in the form of solutions or suspensions for infusion or for injection.

The compounds according to the invention are generally administered at a daily dose of about 0.001 mg/kg to 100 mg/kg of body weight, in 1 to 3 dosage intakes.

The compounds are used systemically, at a concentration generally of between 0.001 % and 10% by weight and preferably between 0.01 % and 1% by weight, relative to the weight of the composition.

Via the topical route, the pharmaceutical composition according to the invention is more particularly intended for treating the skin and mucous membranes and may be in the form of ointments, creams, milks, pomades, powders, impregnated pads, solutions, gels, sprays, lotions or suspensions. It may also be in the form of lipid or polymeric microspheres, nanospheres or vesicles or polymer patches and hydrogels allowing a controlled release. This topical composition may be in anhydrous form, in aqueous form or in the form of an emulsion.

The compounds are used topically at a concentration generally of between 0.001% and 10% by weight and preferably between 0.01% and 1% by weight, relative to the total weight of the composition.

The compounds of formula (I) according to the invention also find an application in cosmetics, in particular in body and hair hygiene and more particularly for regulating and/or restoring skin lipid metabolism.

A subject of the invention is thus also the cosmetic use of a composition comprising, in a physiologically acceptable support, at least one of the compounds of formula (I) for body or hair hygiene.

The cosmetic composition according to the invention containing, in a cosmetically acceptable support, at least one compound of formula (I) or an optical or geometrical isomer thereof or a salt thereof, may especially be in the form of a cream, a milk, a lotion, a gel, lipid or polymeric microspheres, nanospheres or vesicles, a soap or a shampoo.

The concentration of compound of formula (I) in the cosmetic composition is between 0.001% and 3% by weight relative to the total weight of the composition.

The compositions as described above may also obviously contain inert or even pharmacodynamically active additives or combinations of these additives, and especially: wetting agents, depigmenting agents such as hydroquinone, azelaic acid, caffeic acid or kojic acid; emollients; moisturizers, for instance glycerol, PEG-400, thiamorpholinone and derivatives thereof, or alternatively urea; anti-seborrhoeic or antiacne agents, such as S-carboxymethylcysteine or S-benzylcysteamine, and salts or derivatives thereof, or benzoyl peroxide; antifungal agents such as ketoconazole or 4,5-polymethylene-3-isothiazolidones; antibacterial agents, carotenoids and especially β-carotene; anti-psoriatic agents such as anthralin and derivatives thereof; eicosa-5,8,11,14-tetraynoic and eicosa-5,8,11-triynoic acids, esters and amides thereof, and, finally, retinoids. The compounds of formula (I) may also be combined with D vitamins or derivatives thereof, with corticosteroids, with free-radical scavengers, a-hydroxy or a-keto acids or derivatives thereof, or alternatively with ion-channel blockers.

These compositions may also contain flavour enhancers, preserving agents such as para-hydroxybenzoic acid esters, stabilizers, moisture regulators, pH regulators, osmotic pressure modifiers, emulsifiers, UV-A and UV-B screening agents, and antioxidants such as a-tocopheryl, butylhydroxyanisole or butylhydroxytoluene.

Needless to say, a person skilled in the art will take care to select the optional compound(s) to be added to these compositions such that the advantageous properties intrinsically associated with the present invention are not, or are not substantially, adversely affected by the envisaged addition.

Several examples of production of active compounds of formula (I) according to the invention, and also results of biological activity of such compounds and various concrete formulations based on these compounds.

### Example 1 : (E)-3-[3'-(3-Heptyl-1-methylureido)biphenyl-4-yl]acrylic acid

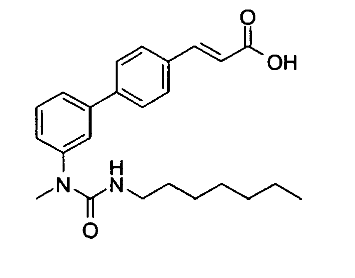

### a. tert-Butyl (3-bromophenyl)carbamate

To a mixture of 94 g (549 mmol) of 3-bromoaniline in 11 of dichloromethane are added portionwise 120 g (549 mmol) of di-tert-butyl dicarbonate, at room temperature. After stirring for 18 hours, the reaction medium is poured into ice-cold water and then extracted with dichloromethane. The organic phase is separated out after settling of the phases, dried over magnesium sulfate and evaporated. 138 g of (3-tert-butyl bromophenyl)carbamate are obtained in a yield of 98%.

### b. tert-Butyl (3-bromophenyl)-N-methylcarbamate

To a solution of 114 g (447 mmol) of tert-butyl (3-bromophenyl)carbamate in 800 ml of DMF are added portionwise 19 g (475 mmol) of sodium hydride (60% in oil) and the reaction medium is stirred until the evolution of gas has ceased. 29.3 ml (470 mmol) of methyl iodide are added dropwise and stirring is continued for 18 hours. The reaction medium is poured into ice-cold water and extracted with ethyl acetate. The organic phase is separated out after settling of the phases, dried over magnesium sulfate and evaporated. 115 g of tert-butyl (3-bromophenyl)-N-methylcarbamate are obtained in a yield of 95%.

### c. (3-Bromophenyl)methylamine

5 mL of trifluoromethanesulfonic acid are added to a solution of 3.6 g (12.7 mmol) of (3- tert-butyl bromophenyl)-N-methylcarbamate in 15 mL of dichloromethane. The reaction medium is stirred for 1 hour at room temperature (r.t.). The reaction is stopped by addition of 50 mL of saturated sodium hydrogen carbonate solution and then extracted with ethyl acetate. The solvents are evaporated off and the residue is then chromatographed on silica gel. Eluent (1/1 heptane/ethyl acetate). 2.14 g of oil are obtained. Yield 90%.

### d. 1-(3-Bromophenyl)-3-heptyl-1-methylurea

3.2 mL (20 mmol, 1.5 eq) of heptyl isocyanate are added to a solution of 2.5 g (13 mmol, 1 eq) of (3-bromophenyl)methylamine in 10 ml of tetrahydrofuran in the presence of 2 ml of triethylamine. The reaction mixture is stirred for 12 hours at r.t. The reaction is stopped by addition of 2 mL of water and then extracted with ethyl acetate. The organic phases are combined and dried over sodium sulfate. The solvents are evaporated off and the residue is then chromatographed on silica gel (70/30 heptane/ethyl acetate). 3.4 g of 1-(3-bromophenyl)-3-heptyl-1-methylurea in solid form are obtained. Yield = 77 %.

### e. 3-Heptyl-1-methyl-1-[3-(4,4,5,5-tetramethy[1,3,2]dioxaborolan-2-yl)phenyl]urea

4.0 g (15.5 mmol, 1.5 eq) of pinacol diborane are added to a mixture of 3.4 g (10 mmol, 1 eq) of 1-(3-bromophenyl)-3-heptyl-1-methylurea and 3.0 g (31 mmol, 3 eq) of potassium acetate, in the presence of 380 mg (0.5 mmol, 5 mol%) of diphenylphosphinoferrocenepalladium dichloride (PdCl₂dppf) in 15 ml of dimethylformamide. The mixture is stirred for 3 hours at 90°C. The reaction is stopped by addition of 50 mL of water and then extracted with ethyl acetate. The organic phases are combined and dried over sodium sulfate. The solvents are evaporated off and the residue is then chromatographed on silica gel (70/30 heptane/ethyl acetate). 2.5 g of 3-heptyl-1-methyl-1-[3-(4,4,5,5-tetramethyl[1,3,2]dioxaborolan-2-yl)phenyl]urea in oil form are obtained. Yield = 64 %.

### f. Ethyl 3-[3'-(3-heptyl-1-methylureido)biphenyl-4-yl]acrylate

308 mg (0.26 mmol, 5 mol%) of tetrakis(triphenylphosphine)palladium are added to a solution of 2 g (5.34mmol, 1.0 eq) of 3-heptyl-1-methyl-1-[3-(4,4,5,5-tetramethyl[1,3,2]dioxaborolan-2-yl)phenyl]urea and 1.6 g (6.4 mmol, 1.2 eq) of ethyl 4-bromocinnamate in 20 mL of an 8/2 mixture of dimethylformamide and of 2M potassium phosphate solution. The reaction mixture is stirred for 3 hours at 90°C. The reaction is stopped by addition of 50 mL of water and then extracted with ethyl acetate. The organic phases are combined and dried over sodium sulfate. The solvents are evaporated off and the residue is then chromatographed on silica gel (70/30 heptane/ethyl acetate). 1.69 g of ethyl 3-[3'-(3-heptyl-1-methylureido)biphenyl-4-yl]acrylate in oil form are obtained. Yield = 75 %.

### g. 3-[3'-(3-Heptyl-1-methylureido)biphenyl-4-yl]acrylic acid

1.6 g (40 mmol, 10 eq) of sodium hydroxide are added to a solution of 1.69 g (4.0 mmol, 1 eq) of ethyl 3-[3'-(3-heptyl-1-methylureido)biphenyl-4-yl]acrylate in 30 ml of 9/1 tetrahydrofuran/methanol. The reaction mixture is stirred for one hour at room temperature. The reaction is stopped by addition of 20 mL of water and 5 mL of acetic acid and then extracted with ethyl acetate. The organic phases are combined and dried over sodium sulfate. The solvents are evaporated off and the residue is then chromatographed on silica gel (70/30 heptane/ethyl acetate). The oil obtained is crystallized from Heptane/ethyl acetate. 1.29 g of 3-[3'-(3-heptyl-1-methylureido)biphenyl-4-yl]acrylic acid in the form of white crystals are obtained. (m.p. = 125-126°C) Yield = 82%.

¹H NMR (CDCl₃, 400 MHz) : 0.86 (t, *J* = 7.2 Hz, 3H) ; 1.24 (m, 8H) ; 1.44 (m, 2H) ; 3.20 (dt, *J* = 6.0, 6.8 Hz, 2H) ; 3.35 (s, 3H) ; 4.42 (t, *J* = 5.6 Hz, 1H) ; 6.54 (d, *J* = 15.9 Hz, 1H) ; 7.29 (d, *J* = 6.4 Hz, 1H) ; 7.55 (m, 3H) ; 7.65 (m, 4H) ; 7.84 (d, *J* = 16 Hz, 1H).

### Example 2 : 3-[3'-(3-Heptyl-1-methylureido)biphenyl-4-yl]propanoic acid

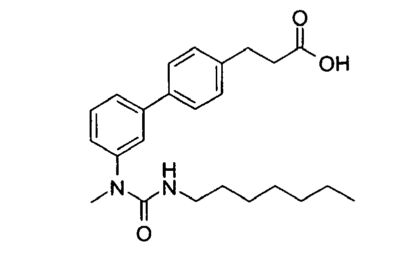

### a. 3-[3'-(3-Heptyl-1-methylureido)biphenyl-4-yl]propanoic acid

A solution of 900 mg (2.28 mmol) of 3-[3'-(3-heptyl-1-methylureido)biphenyl-4-yl]acrylic acid (prepared as in 1g) in 20 ml of methanol is stirred for 4 hours at room temperature in the presence of 100 mg of 10% Pd/C under a hydrogen atmosphere. The palladium is filtered off and the solvents are evaporated off. The residue is crystallized from a dichloromethane/heptane mixture.

620 mg of 3-[3'-(3-heptyl-1-methylureido)biphenyl-4-yl]propanoic acid in the form of a white solid are obtained (m.p. = 101°C). Yield = 68 %.
¹H NMR (CDCl₃, 400 MHz) : 0.86 (t, *J* = 7.2 Hz, 3H) ; 1.24 (m, 8H) ; 1.42 (m, 2H) ; 2.76 (m 2H); 3.04 (m 2H); 3.19 (m 2H); 3.33 (s, 3H); 4.42 (m, 1H); 7.22-7.35 (m, 3H); 7.48-7.54 (m, 5H).

### Example 3 : 3-[3-Fluoro-3'-(3-heptyl-1-methylureido)biphenyl-4-yl]propanoic acid

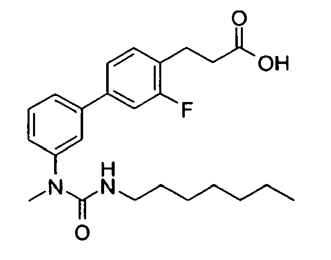

### a. Methyl 3-(4-bromo-2-fluorophenyl)acrylate

2.17 g (6.5 mmol, 1.2 eq) of methyltriphenylphosphoranylidene acetate are added to a solution of 1.1 g (5.4 mmol, 1.0 eq) of 4-bromo-2-fluorobenzaldehyde in 10 ml of toluene. The reaction mixture is stirred for 1 hour at 80°C. The reaction is stopped by addition of 20 mL of water and then extracted with ethyl acetate. The organic phases are combined and dried over sodium sulfate. The solvents are evaporated off and the residue is then chromatographed on silica gel (80/20 heptane/ethyl acetate). 1.1 g of methyl 3-(4-bromo-2-fluorophenyl)acrylate in the form of a white solid are obtained. Yield = 78 %.

### b Methyl 3-[3-fluoro-3'-(3-heptyl-1-methylureido)biphenyl-4-yl]acrylate

44 mg (5 mol%) of tetrakis(triphenylphosphine)palladium are added to a solution of 200 mg (0.77 mmol, 1.0 eq) of methyl 3-(4-bromo-2-fluorophenyl)acrylate and 430 mg (1.15 mmol, 1.5 eq) of 3-heptyl-1-methyl-1-[3-(4,4,5,5-tetramethyl[1,3,2]dioxaborolan-2-yl)phenyl]urea (prepared as in 1e) in a mixture of dimethylformamide and 2M potassium phosphate solution (8/2). The reaction mixture is stirred for 2 hours at 90°C. The reaction is stopped by addition of 10 mL of water and then extracted with ethyl acetate. The organic phases are combined and dried over sodium sulfate. The solvents are evaporated off and the residue is then chromatographed on silica gel (70/30 heptane/ethyl acetate). 138 mg of methyl 3-[3-fluoro-3'-(3-heptyl-1-methylureido)biphenyl-4-yl]acrylate are obtained. Yield = 42 %.

### c Methyl 3-[3-fluoro-3'-(3-heptyl-1-methylureido)bipheny/-4-yl]propanoate

A solution of 138 mg (0.32 mmol) of methyl 3-[3-fluoro-3'-(3-heptyl-1-methylureido)biphenyl-4-yl]acrylate in 3 ml of methanol is stirred for 4 hours at room temperature in the presence of 10 mg of 10% Pd/C under a hydrogen atmosphere. The palladium is filtered off and the solvents are evaporated off. The residue is chromatographed on silica gel (70/30 heptane/ethyl acetate). 130 mg of methyl 3-[3-fluoro-3'-(3-heptyl-1-methylureido)biphenyl-4-yl]propanoate are obtained. Yield = 94 %.

### d. 3-[3-Fluoro-3'-(3-heptyl-1-methy/ureido)bipheny/-4-yl]propanoic acid

100 mg (2.5 mmol, 8 eq) of sodium hydroxide are added to a solution of 130 mg (0.3 mmol, 1 eq) of methyl 3-[3-fluoro-3'-(3-heptyl-1-methylureido)biphenyl-4-yl]propanoate in 3 ml of 9/1 tetrahydrofuran/methanol. The reaction mixture is stirred for 3 hours at room temperature. The reaction is stopped by addition of 10 mL of water and 1 mL of acetic acid and then extracted with ethyl acetate. The organic phases are combined and dried over sodium sulfate. The solvents are evaporated off and the residue is then chromatographed on silica gel (70/30 heptane/ethyl acetate). The oil obtained is crystallized from heptane/dichloromethane. 55 mg of 3-[3-fluoro-3'-(3-heptyl-1-methylureido)biphenyl-4-yl]propanoic acid in the form of a white solid are obtained (m.p. = 92-93°C). Yield = 43 %.
¹H NMR (CDCl₃, 400 MHz) : 0.86 (t, *J* = 7.0 Hz, 3H) ; 1.24 (m, 8H) ; 1.43 (m, 2H) ; 2.75 (t, *J* = 7.6 Hz, 2H) ; 3.06 (t, *J* = 7.6 Hz, 2H) ; 3.20 (dt, *J* = 5.6, 7.2 Hz, 2H) ; 3.32 (s, 3H) ; 4.40 (t, *J* = 5.6 Hz, 1H); 7.24-7.34 (m, 4H) ; 7.46-7.51 (m, 3H).

### Example 4: 3-(3'-{3-[2-(4-Fluorophenyl)ethyl]-1-methylureido}biphenyl-4-yl)propanoic acid

### a. N-Methyl-3-aminophenylboronic acid

37.6 g (202 mmol, 1 eq) of (3-bromophenyl)methylamine (obtained as in 1c) are dissolved in 300 ml of tetrahydrofuran. The reaction mixture is cooled to -70°C, and 166 mL (242 mmol, 1.2 eq) of 1.5 M methyllithium are then added slowly while maintaining the temperature at -70°C. The reaction mixture is stirred for 1 hour at -70°C. 306 mL (444 mmol, 2.2 eq) of 1.46 M tert-butyllithium are added while maintaining the temperature at -70°C. The reaction mixture is stirred for 45 minutes at -70°C. 103.5 mL (808 mmol, 4 eq) of trimethyl borate are added at -65°C, and the reaction mixture is then warmed to room temperature. The reaction is stopped by addition of 1 L of 1 N hydrochloric acid. The pH is adjusted to 5 and the reaction medium is then extracted with n-butanol. The organic phases are combined and dried over sodium sulfate. The solvents are evaporated off and the residue is then chromatographed on silica gel (70/30 heptane/ethyl acetate). 11.3 g of N-methyl-3-aminophenylboronic acid are obtained. Yield = 40 %.

### b Methyl 3-(4-hydroxyphenyl)propanoate

15 g (0.09 mol, 1 eq) of 3-(4-hydroxyphenyl)propanoic acid are dissolved in 50 ml of methanol and 4 drops of sulfuric acid are added. The reaction mixture is stirred for 16 hours at room temperature. The reaction is stopped by addition of 50 mL of saturated sodium hydrogen carbonate solution and then extracted with ethyl acetate. The organic phases are combined and dried over sodium sulfate. The solvents are evaporated off and the residue is then chromatographed on silica gel (70/30 heptane/ethyl acetate). 14.9 g of methyl 3-(4-hydroxyphenyl)propanoate are obtained. Yield = 92 %.

### c. Methyl 3-(3'-methylaminobiphenyl-4-yl)propanoate

1.0 g (5.5 mmol, 1.0 eq) of methyl 3-(4-hydroxyphenyl)propanoate is dissolved in 8 mL of dichloromethane in the presence of 1 ml of triethylamine. 1.12 mL (6.6 mmol, 1.2 eq) of triflic anhydride are added at 0°C. The reaction mixture is stirred for 1 hour at 0°C. The reaction is stopped by addition of 50 mL of saturated sodium hydrogen carbonate solution and then extracted with ethyl acetate. The organic phases are combined and dried over sodium sulfate. The solvents are evaporated off and the residue is then filtered on silica gel (1/1 heptane/ethyl acetate). After evaporating off the solvents, the oil obtained is dissolved in 6 mL of an 8/2 mixture of dimethylformamide and of 2M potassium phosphate solution. 1.0 g (6.6 mmol, 1.2 eq) of N-methyl-3-aminophenylboronic acid are added, along with 317 mg of tetrakis(triphenylphosphine)palladium. The reaction mixture is stirred for 3 hours at 90°C. The reaction is stopped by addition of 50 mL of water and then extracted with ethyl acetate. The organic phases are combined and dried over sodium sulfate. The solvents are evaporated off and the residue is then chromatographed on silica gel (70/30 heptane/ethyl acetate). 0.92 g of methyl 3-(3'-methylaminobiphenyl-4-yl)propanoate is obtained. Yield = 61 %.

### d. Methyl 3-(3'-{3-[2-(4-fluorophenyl)ethyl]-1-methylureido}biphenyl-4-yl)propanoate

340 µL (1.84 mmol, 2 eq) of 4-fluorophenethyl isocyanate are added to a solution of 250 mg (0.92 mmol, 1 eq) of methyl 3-(3'-methylaminobiphenyl-4-yl)propanoate in 8 mL of a 4/1 tetrahydrofuran/triethylamine mixture. The reaction mixture is stirred for 24 hours at room temperature. The reaction is stopped by addition of 10 mL of saturated ammonium chloride solution and then extracted with ethyl acetate. The organic phases are combined and dried over sodium sulfate. The solvents are evaporated off and the residue is then chromatographed on silica gel (70/30 heptane/ethyl acetate). 221 mg of methyl 3-(3'-{3-[2-(4-fluorophenyl)ethyl]-1-methylureido}biphenyl-4-yl)propanoate are obtained. Yield = 55 %.

### e. 3-(3'-{3-[2-(4-Fluorophenyl)ethyl]-1-methylureido}biphenyl-4-yl)propanoic acid

200 mg (5 mmol, 10 eq) of sodium hydroxide are added to a solution of 221 mg (0.51 mmol, 1 eq) of methyl 3-(3'-{3-[2-(4-fluorophenyl)ethyl]-1-methylureido}biphenyl-4-yl)propanoate in 6 ml of an 8/2 tetrahydrofuran/methanol mixture. The reaction mixture is stirred for 3 hours at room temperature. The reaction is stopped by addition of 10 mL of water and 2 mL of acetic acid and then extracted with ethyl acetate. The organic phases are combined and dried over sodium sulfate. The solvents are evaporated off and the residue is then chromatographed on silica gel (70/30 heptane/ethyl acetate). 180 mg of 3-(3'-{3-[2-(4-fluorophenyl)ethyl]-1-methylureido}biphenyl-4-yl)propanoic acid are obtained (m.p. = 146°C). Yield = 84 %.

¹H NMR (CDCl₃, 400 MHz) : 2.73 (t, *J* = 6.7 Hz, 2H) ; 2.76 (t, *J* = 7.8 Hz, 2H) ; 3.05 (t, *J* = 7.6 Hz, 2H) ; 3.31 (s, 3H) ; 3.43 (dt, *J* = 6.6, 6.1 Hz, 2H) ; 4.39 (t, *J* = 5.7 Hz, 1H); 6.83 (m, 2H) ; 7.03 (m, 2H) ; 7.11 (m, 1H) ; 7.35 (m, 3H) ; 7.45 (m, 4H).

### Example 5: 3-[3'-(1-Methyl-3-pentylureido)biphenyl-4-yl]propanoic acid

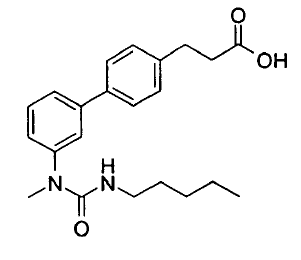

### a methyl 3-[3'-(1-Methyl-3-pentylureido)biphenyl-4-yl]propanoate

236 µL (1.84 mmol, 2 eq) of pentyl isocyanate are added to a solution of 250 mg (0.92 mmol, 1 eq) of methyl 3-(3'-methylaminobiphenyl-4-yl)propanoate (obtained as in 4c) in 5 mL of a 9/1 tetrahydrofuran/triethylamine mixture. The reaction mixture is stirred for 12 h at room temperature. The reaction is stopped by addition of 10 mL of water and then extracted with ethyl acetate. The organic phases are combined and dried over sodium sulfate. The solvents are evaporated off and the residue is then chromatographed on silica gel (70/30 heptane/ethyl acetate). 245 mg of methyl 3-[3'-(1-methyl-3-pentylureido)biphenyl-4-yl]propanoate are obtained. Yield = 70%.

### b. 3-[3'-(1-Methyl-3-pentylureido)biphenyl-4-yl]propanoic acid

130 mg (3.2 mmol, 5 eq) of sodium hydroxide are added to a solution of 245 mg (0.64 mmol, 1 eq) of methyl 3-[3'-(1-methyl-3-pentylureido)biphenyl-4-yl]propanoate in 6 ml of a 9/1 tetrahydrofuran/methanol mixture. The reaction mixture is stirred for 2 hours at room temperature. The reaction is stopped by addition of 10 mL of water and 2 mL of acetic acid and then extracted with ethyl acetate. The organic phases are combined and dried over sodium sulfate. The solvents are evaporated off and the residue is then chromatographed on silica gel (70/30 heptane/ethyl acetate). The oil obtained is crystallized from pentane. 63 mg of solid are obtained (m.p. = 100-102°C). Yield = 27%.

¹H NMR (CDCl₃, 400 MHz) : 0.86 (t, *J* = 7.2 Hz, 3H) ; 1.25 (m, 4H) ; 1.42 (m, 2H) ; 2.75 (t, *J* = 7.7 Hz, 2H); 3.04 (t, *J* = 7.7 Hz, 2H) ; 3.18 (dt, *J* = 4.3, 5.9 Hz, 2H) ; 3.32 (s, 3H) ;4.42 (t, *J* = 5.5 Hz, 1H) ; 7.22 (m, 1H) ;7.33 (d, *J* = 8.1 Hz, 2H) ; 7.47-7.54 (m, 5H).

### Example 6 : 3-[3'-(3-Heptyl-1-methylureido)biphenyl-4-yl]-N-hydroxypropionamide

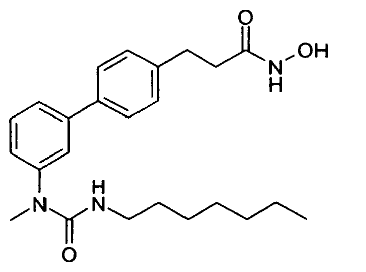

### a. Methyl 3-[3'-(3-heptyl-1-methylureido)biphenyl-4-yl]propanoate

1.2 mL (7.43 mmol, 2 eq) of heptyl isocyanate are added to a solution of 1.0 g (3.7 mmol, 1eq) of methyl 3-(3'-methylaminobiphenyl-4-yl)propanoate (obtained as in 4c) in 15 mL of a 4/1 tetrahydrofuran/triethylamine mixture. The reaction mixture is stirred for 12 hours at 40°C. The reaction is stopped by addition of 10 mL of water and then extracted with ethyl acetate. The organic phases are combined and dried over sodium sulfate. The solvents are evaporated off and the residue is then chromatographed on silica gel (70/30 heptane/ethyl acetate). 1.15 g of methyl 3-[3'-(1-methyl-3-heptyl-ureido)biphenyl-4-yl]propanoate are obtained. Yield = 76%.

### b. 3-[3'-(3-Heptyl-1-methylureido)biphenyl-4-yl]-N-hydroxypropionamide

320 mg (5.7 mmol, 11.4 eq) of potassium hydroxide dissolved in 1.7 ml of methanol are added to a solution of 251 mg (3.6 mmol, 7.2 eq) of hydroxylamine hydrochloride in 2.5 ml of methanol. The reaction mixture is stirred for one hour at room temperature (formation of a white precipitate).
The supernatant solution is taken up and added dropwise to a solution of 206 mg of methyl 3-[3'-(3-heptyl-1-methylureido)biphenyl-4-yl]propanoate in 2 ml of methanol. The reaction mixture is stirred for 24 hours at room temperature. The reaction medium is evaporated to dryness, taken up in water, neutralized to pH 6-7 with 1 N acetic acid solution and extracted with ethyl acetate. The organic phases are combined, washed with water and dried over sodium sulfate. The solvent is evaporated off and the residue (242 mg) is chromatographed on silica gel (10 g CombiFlash column) eluted with 95/5 dichloromethane/methanol. 155 mg of the pinkish powder obtained are crystallized from ethyl acetate with the product obtained during a preceding synthesis performed under the same conditions.
142 mg of 3-[3'-(3-heptyl-1-methylureido)biphenyl-4-yl]-N-hydroxypropionamide in the form of white crystals are obtained (m.p. = 100-101°C). Yield = 69%

¹H NMR (DMSO-d6, 400 MHz) : 0.85 (t, 3H); 1.24 (m, 8H); 1.39 (m, 2H); 2.29 (t, 2H); 2.85 (t, 2H); 3.02 (q, 2H) ; 3.20 (s, 3H); 6.06 (t, 1H); 7.02 (d, 1H); 7.29 (d, 2H); 7.46 (m, 3H); 7.57 (d, 2H); 8.72 (s, 1H); 10.4 (s, 1H).

### Example 7 : 3-[3'-(1-Methyl-3-naphthalen-2-ylureido)biphenyl-4-yl]propanoic acid

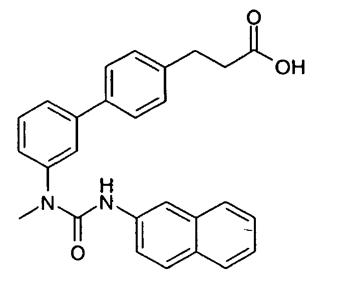

### a. Methyl[3-(4,4,5,5-tetramethyl[1,3,2]dioxaborolan-2-yl)phenyl]amine

35 g (4 mol%) of diphenylphosphinoferrocene-palladium dichloride are added to a solution of 200 g (1.075 mol, 1 eq) of (3-bromophenyl)methylamine and 286.7 g (1.129 mol, 1.05 eq) of pinacol diborane in 2 L of dimethylformamide in the presence of 316.4 g (3.225 mol, 3 eq) of potassium acetate. The reaction medium is heated at 100°C for 3 hours and then stirred at room temperature for 15 hours. The reaction medium is filtered through Celite and 2 L of ethyl acetate are then added to the filtrate. The organic medium is washed with water and then separated out by settling. The solvents are evaporated off and the black oil obtained is chromatographed on silica gel eluted with heptane/ethyl acetate (90/10). 183 g of methyl-[3-(4,4,5,5-tetramethyl[1,3,2]dioxaborolan-2-yl)phenyl]amine in the form of an orange-yellow oil are obtained. Yield = 73%

### b. Ethyl (E)-3-(3'-methylaminobiphenyl-4-yl)acrylate

In a manner similar to that of Example (1f), by reaction of 2.2 g (8.6 mmol, 1 eq) of ethyl 4-bromocinnamate, 2.0 g (8.6 mmol, 1 eq) of methyl[3-(4,4,5,5-tetramethyl[1,3,2]dioxaborolan-2-yl)phenyl]amine and 500 mg of tetrakis(triphenylphosphine)palladium in 20 mL of a mixture of dimethylformamide and of 2M potassium phosphate solution (5/1), 1.7 g of ethyl (E)-3-(3'-methylaminobiphenyl-4-yl)acrylate are obtained in oil form. Yield = 71%

### c. Ethyl 3-(3'-methylaminobiphenyl-4-yl)propanoate

A solution of 1.7 g (6.0 mmol) of ethyl (E)-3-(3'-methylaminobiphenyl-4-yl)acrylate in 25 ml of methanol is stirred for 12 hours at room temperature in the presence of 300 mg (18% by mass) of 10% palladium-on-charcoal under a hydrogen atmosphere. The palladium is filtered off and the solvent is evaporated off. 1.52 g of ethyl 3-(3'-methylaminobiphenyl-4-yl)propanoate are obtained. Yield = 90%

### d. Ethyl 3-[3'(1-methyl-3-naphthalen-2-ylureido)biphenyl-4-yl]propanoate

250 mg (1.43 mmol, 1.3 eq) of naphthyl isocyanate are added to a solution of 310 mg (1.1 mmol, 1 eq) of ethyl 3-(3'-methylaminobiphenyl-4-yl)propanoate in 3 mL of dichloromethane. The reaction mixture is heated at 45-50°C for 20 hours. The reaction medium is evaporated to dryness and the residue is then crystallized from ethyl ether, filtered and dried. 452 mg of ethyl 3-[3'-(1-methyl-3-naphthalen-2-ylureido)biphenyl-4-yl]propanoate are obtained in the form of a white powder. Yield = 91 %

### e. 3-[3'-(1-Methyl-3-naphthalen-2-ylureido)biphenyl-4-yl]propanoic acid

392 mg (9.8 mmol, 10 eq) of sodium hydroxide are added to a solution of 444 mg (0.98 mmol, 1 eq) of ethyl 3-[3'-(1-methyl-3-naphthalen-2-ylureido)biphenyl-4-yl]propanoate in 10 mL of ethanol. The reaction mixture is heated at 55°C overnight. The reaction medium is evaporated to dryness, taken up in water, acidified with 1N hydrochloric acid solution and extracted with ethyl acetate. The organic phases are combined, washed with water and dried over sodium sulfate. The solvent is evaporated off and the residue is recrystallized from ethyl acetate, filtered and dried.
308 mg of 3-[3'-(1-methyl-3-naphthalen-2-ylureido)biphenyl-4-yl]propanoic acid in the form of an off-white powder are obtained (m.p. = 170-171°C). Yield = 87%

¹H NMR (CDCl₃, 400 MHz): 2.61 (t, 2H); 2.97 (t, 2H); 3.39 (s, 3H); 6.55 (s, 1H); 7.29 (m, 5H); 7.31 (s, 1H); 7.51 (m, 5H); 7.67 (t, 3H); 7.91 (s, 1H).

### Example 8 : 3-[3'-(3-Heptyl-1-methylthioureido)biphenyl-4-yl]propanoic acid

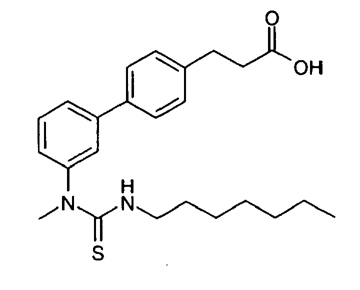

### a. Ethyl 3-[3'-(3-heptyl-1-methylthioureido)biphenyl-4-yl]propanoate

525 µl (3.04 mmol, 3 eq) of heptyl isothiocyanate are added to 288 mg (1.02 mmol, 1 eq) of ethyl 3-(3'-methylaminobiphenyl-4-yl)propanoate (prepared in Example 7c). The reaction mixture is heated at 100°C by microwave for 3 hours 30 minutes. The residue is chromatographed on silica gel (15 g FlashSmart Pack column) eluted with 90/10 heptane/ethyl acetate.
361 mg of ethyl 3-[3'-(3-heptyl-1-methylthioureido)biphenyl-4-yl]propanoate are obtained in the form of a yellowish oil. Yield = 80%

### b. 3-[3'-(3-Heptyl-1-methylthioureido)biphenyl-4-yl]propanoic acid

422 mg (10.5 mmol, 10 eq) of sodium hydroxide are added to a solution of 465 mg (1.05 mmol, 1 eq) of ethyl 3-[3'-(3-heptyl-1-methylthioureido)biphenyl-4-yl]propanoate in 10 mL of ethanol. The reaction mixture is heated at 50°C for 2 hours. The reaction medium is evaporated to dryness, taken up in water, acidified with 2N hydrochloric acid solution and extracted with ethyl acetate. The organic phases are combined, washed with water and dried over sodium sulfate. The solvent is evaporated off and the paste obtained is crystallized from ethyl ether, filtered and dried.
398 mg of 3-[3'-(3-heptyl-1-methylthioureido)biphenyl-4-yl]propanoic acid in the form of off-white crystals are obtained (m.p. = 95-96°C). Yield = 92%

¹H NMR (CDCl₃, 400 MHz) : 0.85 (t, 3H); 1.24 (m, 8H); 1.48 (m, 2H); 2.75 (t, 2H); 3.04 (t, 2H); 3.57 (q, 2H) ; 3.72 (s, 3H); 5.42 (t, 1H); 7.20 (d, 1H); 7.34 (d, 2H); 7.46 (s, 1H); 7.56 (m, 3H); 7.62 (d, 1H).

### Example 9 : (E)-3-[2-Fluoro-3'-(3-heptyl-1-methylureido)biphenyl-4-yl]acrylic acid

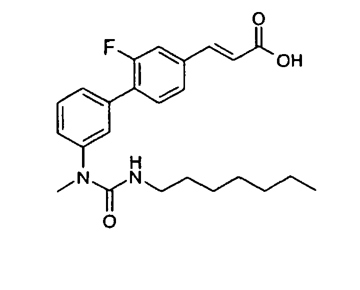

### a. Methyl (E)-3-(3-fluoro-4-hydroxyphenyl)acrylate

13.1 g (0.039 mol, 2.2 eq) of methyl (triphenylphosphoranylidene)acetate are added to a solution of 2.5 g (0.0178 mol, 1 eq) of 3-fluoro-4-hydroxybenzaldehyde in 30 ml of toluene. The reaction mixture is heated at 80°C for 2 hours. The reaction medium is hydrolysed in water and extracted with ethyl acetate. The organic phases are combined, washed with water and dried over sodium sulfate. The solvent is evaporated off and the oil obtained is chromatographed on silica gel (70/30 heptane/ethyl acetate). 2.7 g of methyl (E)-3-(3-fluoro-4-hydroxyphenyl)acrylate are obtained in solid form. Yield = 78%

### b. Methyl (E)-3-(3-fluoro-4-trifluoromethanesulfonyloxyphenyl)acrylate

1.40 mL (8.3 mmol, 1.1 eq) of triflic anhydride are added to a solution of 1.47 g (7.5 mmol, 1 eq) of methyl (E)-3-(3-fluoro-4-hydroxyphenyl)acrylate and 1.57 mL (9.0 mmol, 1.2 eq) of diisopropylethylamine in 10 mL of dichloromethane. The reaction mixture is stirred at room temperature overnight. The reaction medium is hydrolysed in water, acidified with 1 N hydrochloric acid solution and extracted with ethyl acetate. The organic phases are combined, washed with water and dried over sodium sulfate. The solvent is evaporated off and 2.51 g of methyl (E)-3-(3-fluoro-4-trifluoromethane-sulfonyloxyphenyl)acrylate are obtained in the form of a brown oil. Yield = 100%

### c. Methyl (E)-3-[2-fluoro-3'-(3-heptyl-1-methylureido)biphenyl-4-yl]acrylate

85 mg (0.38 mmol, 0.05 eq) of palladium acetate are added to a mixture of 2.50 g (7.6 mmol, 1 eq) of methyl (E)-3-(3-fluoro-4-trifluoromethanesulfonyloxyphenyl)acrylate, 3.71 g (9.9 mmol, 1.3 eq) of 3-heptyl-1-methyl-1-[3-(4,4,5,5-tetramethyl[1,3,2]dioxaborolan-2-yl)phenyl]urea (prepared as in Example 1e), 272 mg (0.78 mmol, 0.1 eq) of 2-(dicyclohexylphosphino)biphenyl and 5.5 mL of 2M potassium phosphate solution in 28 ml of dimethylformamide. The reaction mixture is heated at 90-95°C overnight. The reaction medium is hydrolysed in saturated ammonium chloride solution and extracted with ethyl acetate. The organic phases are combined, washed with water and dried over sodium sulfate. The solvent is evaporated off and the brown paste obtained (4.16 g) is chromatographed on silica gel (120 g CombiFlash column) eluted with 80/20 heptane/ethyl acetate. 706 mg (22%) of a "cis/trans" mixture are obtained. This solid is washed in an ethyl ether/heptane mixture, filtered and dried. 238 mg of methyl (E)-3-[2-fluoro-3'-(3-heptyl-1-methylureido)biphenyl-4-yl]acrylate in the form of a white solid are obtained. Yield = 7%

### d. (E)-3-[2-Fluoro-3'-(3-heptyl-1-methylureido)biphenyl-4-yl]acrylic acid

220 mg (5.5 mmol, 10 eq) of sodium hydroxide are added to a solution of 234 mg (0.55 mmol, 1 eq) of methyl 3-[2-fluoro-3'-(3-heptyl-1-methylureido)biphenyl-4-yl]acrylate in 6 mL of ethanol + 2 ml of tetrahydrofuran. The reaction mixture is heated at 50°C overnight. The reaction medium is evaporated to dryness, taken up in water, acidified with 1 N hydrochloric acid solution and extracted with ethyl acetate. The organic phases are combined, washed with water and dried over sodium sulfate. The solvent is evaporated off and the yellowish paste obtained (240 mg) is chromatographed on silica gel (15 g FlashSmart Pack column) eluted with 96/4 dichloromethane/methanol, and the solid obtained is then taken up in heptane and then in ethyl ether, filtered and dried. 105 mg of (E)-3-[2-fluoro-3'-(3-heptyl-1-methylureido)biphenyl-4-yl]acrylic acid are obtained in the form of white crystals (m.p. = 130-131 °C). Yield = 79%

¹H NMR CDCl₃, 400 MHz) : 0.85 (t, 3H); 1.21 (m, 8H); 1.42 (m, 2H); 3.19 (q, 2H) ; 3.32 (s, 3H); 4.45 (t, 1H); 6.52 (d, *J* = 16 Hz, 1H); 7.29 (d, 1H); 7.38 (d, 1H); 7.50 (m, 4H); 7.76 (d, *J* = 16 Hz, 1H).

### Example 10 : 3-[2-Fluoro-3'-(3-heptyl-1-methylureido)biphenyl-4-yl]propanoic acid

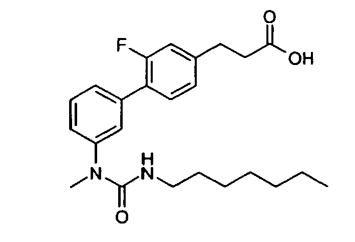

### a. Ethyl 3-[2-fluoro-3'-(3-heptyl-1-methylureido)biphenyl-4-yl]propanoate

37 mg (10% by mass) of 10% palladium-on-charcoal are added to a solution of 365 mg (0.85 mmol, 1 eq) of methyl (E)-3-[2-fluoro-3'-(3-heptyl-1-methylureido)biphenyl-4-yl]acrylate (prepared in Example 9c) in 7 ml of methanol. The reaction mixture, under a pressure of 7 bar of hydrogen, is heated at 40°C overnight. The reaction medium is filtered through Celite and then evaporated to dryness. The oil obtained (328 mg) is chromatographed on silica gel (15 g FlashSmart Pack column) eluted with 70/30 heptane/ethyl acetate.
246 mg of ethyl 3-[2-fluoro-3'-(3-heptyl-1-methylureido)biphenyl-4-yl]propanoate are obtained in the form of a yellow oil. Yield = 68%

### b. 3-[2-Fluoro-3'-(3-heptyl-1-methylureido)biphenyl-4-yl]propanoic acid

227 mg (5.7 mmol, 10 eq) of sodium hydroxide are added to a solution of 243 mg (0.56 mmol, 1 eq) of ethyl 3-[2-fluoro-3'-(3-heptyl-1-methylureido)biphenyl-4-yl]propanoate in 6 ml of methanol. The reaction mixture is heated at 50°C for 4 hours 30 minutes. The reaction medium is evaporated to dryness, taken up in water, acidified with 2N hydrochloric acid solution and extracted with ethyl acetate. The organic phases are combined, washed with water and dried over sodium sulfate. The solvent is evaporated off and the paste obtained (258 mg) is crystallized from ethyl ether, filtered and dried. 150 mg of 3-[2-fluoro-3'-(3-heptyl-1-methylureido)biphenyl-4-yl]propanoic acid are obtained in the form of a white powder (m.p. = 80-82°C). Yield = 64%

¹H NMR (CDCl₃, 400 MHz) : 0.87 (t, 3H); 1.27 (m, 8H); 1.43 (m, 2H); 2.75 (t, 2H); 3.03 (t, 2H); 3.19 (q, 2H) ; 3.32 (s, 3H); 4.47 (t, 1H); 7.06 (d, 1H); 7.11 (d, 2H); 7.25 (d, 1H); 7.37 (t, 1H); 7.47 (m, 3H).

### Example 11 : 3-[3'-(3-Hexyl-1-methylureido)biphenyl-4-yl]propanoic acid

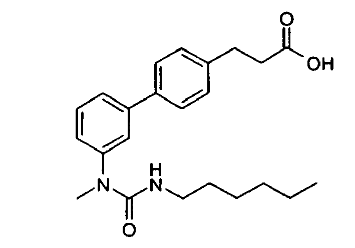

### a. Ethyl 3-[3'-(3-hexyl-1-methylureido)biphenyl-4-yl]propanoate

380 µl (2.55 mmol, 1.5 eq) of hexyl isocyanate are added to 482 mg (1.7 mmol, 1 eq) of ethyl 3-(3'-methylaminobiphenyl-4-yl)propanoate (Example 7c). The reaction mixture is heated at 100°C by microwave for 30 minutes. The residue is chromatographed on silica gel (50 g FlashSmart Pack column) eluted with 70/30 heptane/ethyl acetate.
470 mg of ethyl 3-[3'-(3-hexyl-1-methylureido)biphenyl-4-yl]propanoate are obtained in the form of a yellow oil. Yield = 67%

### b. 3-[3'-(3-Hexyl-1-methylureido)biohenyl-4-yl]propanoic acid

452 mg (11.3 mmol, 10 eq) of sodium hydroxide are added to a solution of 464 mg (1.13 mmol, 1 eq) of ethyl 3-[3'-(3-hexyl-1-methylureido)biphenyl-4-yl]propanoate in 10 mL of ethanol. The reaction mixture is heated at 50°C for 6 hours. The reaction medium is evaporated to dryness, taken up in water, acidified with 1N hydrochloric acid solution and extracted with ethyl acetate. The organic phases are combined, washed with water and dried over sodium sulfate. The solvent is evaporated off and the orange oil that crystallizes is taken up in ethyl ether, filtered and dried.
357 mg of 3-[3'-(3-hexyl-1-methylureido)biphenyl-4-yl]propanoic acid are obtained in the form of a cream-coloured powder (m.p. = 119-120°C). Yield = 83%

¹H NMR (CDCl₃, 400 MHz) : 0.85 (t, 3H); 1.24 (m, 6H); 1.42 (m, 2H); 2.75 (t, 2H); 3.04 (t, 2H); 3.18 (q, 2H) ; 3.32 (s, 3H); 4.43 (t, 1H); 7.22 (d, 1H); 7.33 (d, 2H); 7.50 (m, 5H).

### Example 12 : 3-[3'-(3-Octyl-1-methylureido)biphenyl-4-yl]propanoic acid

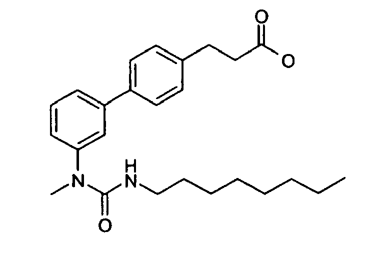

### a. Ethyl 3-[3'-(3-octyl-1-methylureido)biphenyl-4-yl]propanoate

464 µl (2.55 mmol, 1.5 eq) of octyl isocyanate are added to 482 mg (1.7 mmol, 1 eq) of ethyl 3-(3'-methylaminobiphenyl-4-yl)propanoate (prepared in Example 7c). The reaction mixture is heated at 100°C by microwave for 30 minutes. The residue is chromatographed on silica gel (50 g FlashSmart Pack column) eluted with 70/30 heptane/ethyl acetate. 755 mg of ethyl 3-[3'-(3-octyl-1-methylureido)biphenyl-4-yl]propanoate are obtained in the form of a yellowish oil. Yield = 100%

### b. 3-[3'-(3-Octyl-1-methylureido)biphenyl-4-yl]propanoic acid

684 mg (17.1 mmol, 10 eq) of sodium hydroxide are added to a solution of 750 mg (1.71 mmol, 1 eq) of ethyl 3-[3'-(3-octyl-1-methylureido)biphenyl-4-yl]propanoate in 15 mL of ethanol. The reaction mixture is heated at 50°C for 6 hours. The reaction medium is evaporated to dryness, taken up in water, acidified with 1 N hydrochloric acid solution and extracted with ethyl acetate. The organic phases are combined, washed with water and dried over sodium sulfate. The solvent is evaporated off and the orange oil obtained is chromatographed on silica gel (30 g FlashSmart Pack column) eluted with 30/70 heptane/ethyl acetate and then crystallized from ethyl ether, filtered and dried. 500 mg of 3-[3'-(3-octyl-1-methylureido)biphenyl-4-yl]propanoic acid are obtained in the form of a white powder (m.p. = 86-88°C). Yield = 71 %

¹H NMR (CDCl₃, 400 MHz) : 0.87 (t, 3H); 1.24 (m, 10H); 1.42 (m, 2H); 2.75 (t, 2H); 3.04 (t, 2H); 3.18 (q, 2H) ; 3.32 (s, 3H); 4.43 (t, 1H); 7.22 (d, 1H); 7.33 (d, 2H); 7.47-7.54 (m, 5H).

### Example 13: 3-{3'-[3-(4-Benzyloxyphenyl)-1-methylureido]biphenyl-4-yl}propanoic acid

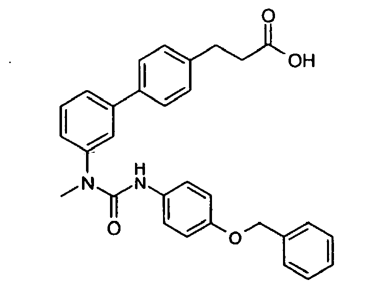

### a. Ethyl 3-{3'-[3-(4-benzyloxyphenyl)-1-methylureido]biphenyl-4-yl}propanoate

345 mg (1.5 mmol, 1.5 eq) of 1-benzyloxy-4-isocyanatobenzene are added to 283 mg (1.0 mmol, 1 eq) of ethyl 3-(3'-methylaminobiphenyl-4-yl)propanoate (prepared in Example 7c). The reaction mixture is heated at 100°C by microwave for 30 minutes. The residue is taken up in a heptane/ethyl ether mixture, filtered and dried.
522 mg of ethyl 3-{3'-[3-(4-benzyloxyphenyl)-1-methylureido]biphenyl-4-yl}propanoate are obtained in the form of a cream-coloured powder. Yield = 100%

### b. 3-{3'-[3-(4-Benzyloxyphenyl)-1-methylureido]biphenyl-4-yl}propanoic acid

400 mg (10 mmol, 10 eq) of sodium hydroxide are added to a solution of 515 mg (1.0 mmol, 1 eq) of ethyl 3-{3'-[3-(4-benzyloxyphenyl)-1-methylureido]biphenyl-4-yl}propanoate in 20 mL of ethanol. The reaction mixture is heated at 50°C for 5 hours. The reaction medium is evaporated to dryness, taken up in water, acidified with 1 N hydrochloric acid solution and extracted with ethyl acetate. The organic phases are combined, washed with water and dried over sodium sulfate. The solvent is evaporated off and the solid obtained is taken up in ethyl ether and then in ethyl acetate, filtered and dried.
441 mg of 3-{3'-[3-(4-benzyloxyphenyl)-1-methylureido]biphenyl-4-yl}propanoic acid are obtained in the form of a light-beige powder (m.p. = 173.5-175°C). Yield = 92%

¹H NMR (CDCl₃, 400 MHz) : 2.64 (t, 2H); 3.00 (t, 2H); 3.37 (s, 3H); 5.00 (s, 2H); 6.29 (s, 1H); 6.85 (d, 2H); 7.20 (d, 2H); 7.30-7.40 (m, 8H); 7.50-7.56 (m, 5H).

### Example 14 : 3-{3'-[3-(4-Butylphenyl)-1-methylureido]biphenyl-4-yl}propanoic acid

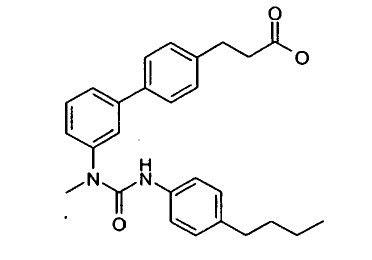

### a. Ethyl 3-{3'-[3-(4-Butylphenyl)-1-methylureido]biphenyl-4-yl}propanoate

345 mg (1.5 mmol, 1.5 eq) of 1-butyl-4-isocyanatobenzene are added to 283 mg (1.0 mmol, 1 eq) of ethyl 3-(3'-methylaminobiphenyl-4-yl)propanoate (prepared in Example 7c). The reaction mixture is heated at 100°C by microwave for 30 minutes. The mixture is chromatographed on silica gel (15 g FlashSmart column) eluted with 80/20 heptane/ethyl acetate. 442 mg of ethyl 3-{3'-[3-(4-butylphenyl)-1-methylureido]biphenyl-4-yl}propanoate are obtained in the form of a yellow oil that crystallizes. Yield = 96%

### b. 3-{3'-[3-(4-Butylphenyl)-1-methylureido]biphenyl-4-yl}propanoic acid

380 mg (10 mmol, 10 eq) of sodium hydroxide are added to a solution of 436 mg (1.0 mmol, 1 eq) of ethyl 3-{3'-[3-(4-butylphenyl)-1-methylureido]biphenyl-4-yl}propanoate in 20 mL of ethanol. The reaction mixture is heated at 50°C for 3 hours. The reaction medium is evaporated to dryness, taken up in water, acidified with 1 N hydrochloric acid solution and extracted with ethyl acetate. The organic phases are combined, washed with water and dried over sodium sulfate. The solvent is evaporated off and 352 mg of 3-{3'-[3-(4-butylphenyl)-1-methylureido]biphenyl-4-yl}propanoic acid are obtained in the form of white crystals (m.p. = 157-158°C). Yield = 86%

¹H NMR (CDCl₃, 400 MHz) : 0.88 (t, 3H); 1.30 (m, 2H); 1.52 (m, 2H); 2.52 (t, 2H); 2.72 (t, 2H); 3.01 (t, 2H) ; 3.38 (s, 3H); 6.26 (s, 1H); 7.04 (d, 2H); 7.18 (d, 2H); 7.30 (m, 3H); 7.54 (m, 5H).

### Example 15 : 3-[3'-(3-Heptyl-1-methylureido)-2-(2-methoxyethoxy)biphenyl-4-yl]propanoic acid

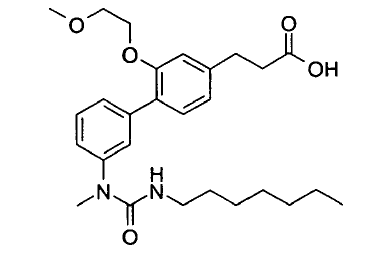

### a. Methyl 3-benzyloxy-4-iodobenzoate

2.35 mL (19.78 mmol, 1.1 eq) of benzyl bromide are added to a solution of 5.0 g (17.98 mmol, 1 eq) of methyl 3-hydroxy-4-iodobenzoate in 30 ml of methyl ethyl ketone in the presence of 5.0 g (36.18 mmol, 2 eq) of potassium carbonate. The reaction mixture is heated at 60°C for 5 hours and then hydrolysed in water and extracted with ethyl acetate. 6.9 g of methyl 3-benzyloxy-4-iodobenzoate are obtained in oil form and are used in the following reaction without further purification.

### b. (3-Benzyloxy-4-iodophenyl)methanol

1.18 g (54 mmol, 3 eq) of lithium borohydride are added to a solution of 6.9 g crude (17.98 mmol, 1 eq) of methyl 3-benzyloxy-4-iodobenzoate in 30 ml of THF. The reaction mixture is heated at 60°C for 12 hours and then hydrolysed in saturated ammonium chloride solution NH4CI and extracted with ethyl acetate. 6.5 g of (3-benzyloxy-4-iodophenyl)methanol are obtained in oil form and are used in the following reaction without further purification.

### c. 3-Benzyloxy-4-iodobenzaldehyde

7.8 g (90 mmol, 5 eq) of manganese oxide are added to a solution of 6.5 g (17.98 mmol, 1 eq) of (3-benzyloxy-4-iodophenyl)methanol in 50 mL of dichloromethane. The reaction medium is stirred for 12 hours at room temperature, and the solid is then filtered off and the solvent is evaporated off. The residual oil is chromatographed on silica gel (8/2 heptane/ethyl acetate). The oil obtained is crystallized from pentane. 3.25 g of 3-benzyloxy-4-iodobenzaldehyde are obtained. Yield = 54% for the three steps c, d and e.

### d. Methyl (E)-3-(3-benzyloxy-4-iodophenyl)acrylate

5.0 g (14.95 mmol, 1.5 eq) of methyl (triphenylphosphoranylidene)acetate are added to a solution of 3.25 g (9.6 mmol, 1 eq) of 3-benzyloxy-4-iodobenzaldehyde in 15 ml of toluene. The reaction mixture is heated at 90°C for 1 hour. The solvent is evaporated off and the oil obtained is chromatographed on silica gel (8/2 heptane/ethyl acetate). After recrystallization from dichloromethane/heptane, 2.59 g of methyl (E)-3-(3-benzyloxy-4-iodophenyl)acrylate are obtained. Yield = 68%

### e. Methyl (E)-3-[3'-(3-heptyl-1-methylureido)-hydroxybiphenyl-4-yl]acrylate

A solution of 4.6 g (11.67 mmol, 1 eq) of methyl (E)-3-(3-benzyloxy-4-iodophenyl)acrylate, 4.8 g (12.83 mmol, 1.1 eq) of 3-heptyl-1-methyl-1-[3-(4,4,5,5-tetramethyl[1,3,2]dioxaborolan-2-yl)phenyl]urea (prepared as in Example 1e) in 18 ml of dimethylformamide + 3 mL of 2M potassium phosphate solution, in the presence of 128 mg (5 mol%) of palladium acetate and 408 mg (10 mol%) of 2-(dicyclohexylphosphino)biphenyl is stirred at 80°C for 5 hours. The reaction medium is hydrolysed in water and then extracted with ethyl acetate. The organic phases are washed with saturated sodium chloride solution and dried over sodium sulfate. The solvents are evaporated off and the oil obtained is chromatographed on silica (7/3 heptane/ethyl acetate). 5.89 g of methyl (E)-3-[3'-(3-heptyl-1-methylureido)-2-hydroxybiphenyl-4-yl]acrylate are obtained in oil form. Yield = 98%

### f. Methyl 3-[3'-(3-heptyl-1-methylureido)-2-hydroxybiphenyl-4-yl]propanoate

A solution of 5.89 g (13.87 mmol, 1 eq) of methyl (E)-3-[3'-(3-heptyl-1-methylureido)-2-hydroxybiphenyl-4-yl]acrylate in 10 ml of methanol and 10 mL of ethyl acetate in the presence of 3 mL of acetic acid and 1.0 g (17% by mass) of 10% palladium-on-charcoal is stirred for 12 hours at room temperature under a hydrogen atmosphere. The palladium is filtered off through Celite and the solvents are evaporated off. The residue is chromatographed on silica gel (7/3 heptane/ethyl acetate) and crystallized from pentane. 3.0 g of methyl 3-[3'-(3-heptyl-1--methylureido)-2-hydroxybiphenyl-4-yl]propanoate are obtained in solid form. Yield = 60%

### g. Methyl 3-[3'-(3-heptyl-1-methylureido)-2-(2-methoxyethoxy)biphenyl-4-yl]propanoate

A solution of 200 mg (0.47 mmol, 1 eq) of methyl 3-[3'-(3-heptyl-1-methylureido)-2-hydroxybiphenyl-4-yl]propanoate, 200 mg (1.36 mmol, 3 eq) of 2-bromoethylmethyl ether and 390 mg (2.82 mmol, 6 eq) of potassium carbonate in the presence of 28 mg (0.188 mmol, 0.4 eq) of sodium iodide in 8 ml of methyl ethyl ketone is refluxed for 15 hours. The insoluble matter is filtered off and the solvent is evaporated off. The oil obtained is chromatographed on silica gel (10 g FlashSmart column) eluted with 40/60 heptane/ethyl acetate. 223 mg of methyl 3-[3'-(3-heptyl-1-methylureido)-2-(2-methoxyethoxy)biphenyl-4-yl]propanoate are obtained in the form of a yellowish oil. Yield = 98%

### h. 3-[3'-(3-Heptyl-1-methylureido)-2-(2-methoxvethoxy)biphenyl-4-yl]propanoic acid

278 mg (6.95 mmol, 10 eq) of sodium hydroxide are added to a solution of 337 mg (0.695 mmol, 1 eq) of methyl 3-[3'-(3-heptyl-1-methylureido)-2-(2-methoxyethoxy)biphenyl-4-yl]propanoate in 10 ml of methanol. The reaction mixture is heated at 50°C for 2 hours. The reaction medium is evaporated to dryness, taken up in water, acidified with 2N hydrochloric acid solution and extracted with ethyl acetate. The organic phases are combined, washed with water and dried over magnesium sulfate. The solvent is evaporated off and the oil obtained is chromatographed on silica gel (10 g FlashSmart column) eluted with ethyl acetate. 274 mg of 3-[3'-(3-heptyl-1-methylureido)-2-(2-methoxyethoxy)biphenyl-4-yl]propanoic acid are obtained in the form of a whitish oil. Yield = 84%

¹H NMR CDCl₃, 400 MHz) : 0.84 (t, 3H); 1.22-1.26 (m, 8H); 1.40 (m, 2H); 2.72 (t, 2H); 2.99 (t, 2H); 3.16 (q, 2H) ; 3.29 (s, 3H); 3.33 (s, 3H); 3.66 (m, 2H); 4.13 (m, 2H); 4.5 (t, 1H); 6.86 (s, 1H); 6.90 (d, 1H); 7.17 (d, 1H); 7.26 (d, 1H); 7.43 (t, 3H); 7.50 (d, 1H); 7.52 (s, 1H).

### Example 16 : 3-[3'-(3-Heptyl-1-methylureido)-2-(3-methylbutoxy)biphenyl-4-yl]propanoic acid

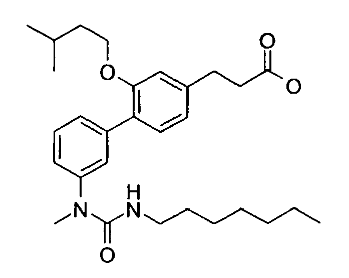

### a. Methyl 3-[3'-(3-heptyl-1-methylureido)-2-(3-methylbutoxy)biphenyl-4-yl]propanoate

A solution of 300 mg (0.703 mmol, 1 eq) of methyl 3-[3'-(3-heptyl-1-methylureido)-2-hydroxybiphenyl-4-yl]propanoate (prepared in Example 15f), 319 mg (2.11 mmol, 3 eq) of 1-bromo-3-methylbutane and 583 mg (4.22 mmol, 6 eq) of potassium carbonate in the presence of 42 mg (0.281 mmol, 0.4 eq) of sodium iodide in 10 ml of methyl ethyl ketone is refluxed for 21 hours. The insoluble matter is filtered off and the solvent is evaporated off. 400 mg of methyl 3-[3'-(3-heptyl-1-methylureido)-2-(3-methylbutoxy)biphenyl-4-yl]propanoate are obtained in the form of a yellowish oil. Quantitative yield.

### b. 3-[3'-(3-Heptyl-1-methylureido)-2-(3-methylbutoxy)biphenyl-4-yl]propanoic acid

281 mg (7.03 mmol, 10 eq) of sodium hydroxide are added to a solution of 400 mg (0.703 mmol, 1 eq) of methyl 3-[3'-(3-heptyl-1-methylureido)-2-(3-methylbutoxy)biphenyl-4-yl]propanoate in 10 ml of methanol. The reaction mixture is heated at 55°C for 1 hour. The reaction medium is evaporated to dryness, taken up in water, acidified with 2N hydrochloric acid solution and extracted with ethyl acetate. The organic phases are combined, washed with water and dried over magnesium sulfate. The solvent is evaporated off and the oil obtained is chromatographed on silica gel (10 g FlashSmart column) eluted with 30/70 heptane/ethyl acetate. The product is crystallized from heptane and 284 mg of 3-[3'-(3-heptyl-1-methylureido)-2-(3-methylbutoxy)biphenyl-4-yl]propanoic acid are obtained in the form of white crystals (m.p. = 60-62°C). Yield = 84%

¹H NMR (CDCl₃, 400 MHz) : 0.84 (t, 3H); 0.86 (s, 3H); 0.89 (s, 3H); 1.21-1.27 (m, 8H); 1.39 (m, 2H); 1.60 (q, 2H); 1.70-1.75 (m, 1H); 2.73 (t, 2H); 3.00 (t, 2H); 3.16 (q, 2H) ; 3.29 (s, 3H); 3.99 (t, 2H); 4.41 (t, 1H); 6.85 (s, 1H); 6.89 (d, 1 H); 7.17 (d, 1H); 7.24 (d, 1H); 7.40-7.45 (m, 3H).

### Example 17: 3-[2-(3-Chloropropoxy)-3'-(3-heptyl-1-methylureido)biphenyl-4-yl]propanoic acid

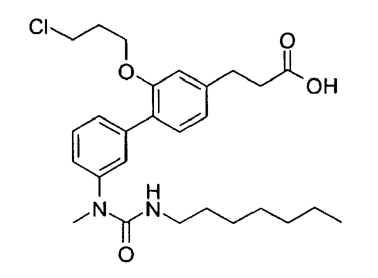

### a. Methyl 3-[2-(3-chloropropoxy)-3'-(3-heptyl-1-methylureido)biphenyl-4-yl]propanoate

A solution of 300 mg (0.703 mmol, 1 eq) of methyl 3-[3'-(3-heptyl-1-methylureido)-2-hydroxybiphenyl-4-yl]propanoate (prepared in Example 15f), 319 mg (2.11 mmol, 3 eq) of 1-chloro-3-iodopropane and 583 mg (4.22 mmol, 6 eq) of potassium carbonate in 10 ml of methyl ethyl ketone is refluxed for 7 hours. The insoluble matter is filtered off, the solvent is evaporated off and the oil obtained is chromatographed on silica gel (35 g FlashSmart column) eluted with 60/40 heptane/ethyl acetate. 394 mg of methyl 3-[2-(3-chloropropoxy)-3'-(3-heptyl-1-methylureido)biphenyl-4-yl]propanoate are obtained in the form of a yellowish oil. Quantitative yield.

### b. 3-[2-(3-Chloropropoxy)-3'-(3-heptyl-1-methylureido)biphenyl-4-yl]propanoic acid

31 mg (0.773 mmol, 1 eq) of sodium hydroxide are added to a solution of 389 mg (0.773 mmol, 1 eq) of methyl 3-[2-(3-chloropropoxy)-3'-(3-heptyl-1-methylureido)-biphenyl-4-yl]propanoate in 10 ml of methanol. The reaction mixture is stirred at room temperature for 18 hours. The reaction medium is evaporated to dryness, taken up in water, acidified with 2N hydrochloric acid solution and extracted with ethyl acetate. The organic phases are combined, washed with water and dried over magnesium sulfate. The solvent is evaporated off and the oil obtained is chromatographed on silica gel (10 g FlashSmart column) eluted with 98/2 dichloromethane/methanol. The product is crystallized from heptane, the filtrate is evaporated and 21 mg of 3-[2-(3-chloropropoxy)-3'-(3-heptyl-1-methylureido)biphenyl-4-yl]propanoic acid are obtained in the form of a whitish oil. Yield = 6%

¹H NMR (CDCl₃, 400 MHz) : 0.84 (t, 3H); 1.22-1.27 (m, 8H); 1.40 (m, 2H); 1.98 (m, 2H); 2.73 (t, 2H); 3.00 (t, 2H); 3.15 (q, 2H) ; 3.29 (s, 3H); 3.45 (t, 2H); 4.07 (t, 2H); 4.43 (t, 1H); 6.87 (s, 1H); 6.89 (d, 1H); 7.17 (d, 1H); 7.24 (d, 1H); 7.40-7.46 (m, 3H).

### Example 18 : 3-[3'-(3-Heptyl-1-methylureido)-2-methoxybiphenyl-4-yl]propanoic acid

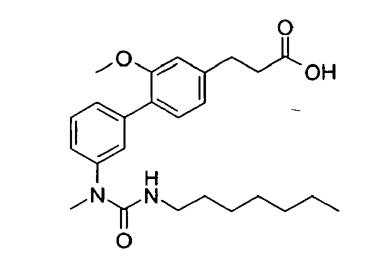

### a. 3-(Methylamino)phenylboronic acid

166 mL (0.242 mol, 1.2 eq) of 1.6 M methyllithium in diethyl ether are added to a solution cooled to -70°C of 37.6 g (0.202 mol, 1 eq) of 3-bromo-N-methylaniline (prepared in Example 7c) in 300 ml of tetrahydrofuran. The reaction medium is stirred at -70°C for one hour and 306 mL (0.444 mol, 2.2 eq) of 1.5 M tert-butyllithium in diethyl ether are then added. The reaction medium is stirred at -70°C for 45 minutes and 103.5 mL (0.808 mol, 4 eq) of trimethyl borate are then added. The reaction medium is stirred at room temperature for one hour and then hydrolysed by addition of ice, acidified with 1 L of 2N hydrochloric acid and extracted with ethyl acetate. The organic phases are combined, washed with water and dried over magnesium sulfate. The solvent is evaporated off and 4.2 g (14%) of 3-(methylamino)phenylboronic acid are obtained. The aqueous phase is basified and extracted with ethyl acetate. The organic phases are combined, washed with water and dried over magnesium sulfate. The solvent is evaporated off and the residue is chromatographed on silica gel (8/2 heptane/ethyl acetate). 3.4 g (11%) of 3-(methylamino)phenylboronic acid are obtained.

### b. Methyl (E)-3-(4-hydroxy-3-methoxyphenyl)acrylate

6.5 g (19.5 mmol, 3 eq) of methyl triphenylphosphoranylideneacetate are added to a solution of 1.0 g (6.5 mmol, 1 eq) of vanillin in 15 ml of toluene. The reaction mixture is stirred for 1 hour at 90°C. The solvent is evaporated off and the residual oil is then chromatographed on silica gel (8/2 heptane/ethyl acetate). 1.12 g of methyl (E)-3-(4-hydroxy-3-methoxyphenyl)acrylate are obtained in oil form. Yield = 83%

### c. Methyl (E)-3-(3-methoxy-4-trifluoromethoxyohenyl)acrylate

0.472 mL (2.88 mmol, 1.2 eq) of triflic anhydride are added to a solution at 0°C of 500 mg (2.40 mmol, 1 eq) of methyl (E)-3-(4-hydroxy-3-methoxyphenyl)acrylate in 10 mL of dichloromethane in the presence of 1 ml of triethylamine. The reaction mixture is stirred for 1 hour at 0°C. The reaction medium is hydrolysed in sodium hydrogen carbonate solution and then extracted with ethyl acetate. The organic phases are washed with saturated sodium chloride solution and dried over sodium sulfate. The solvent is evaporated off and the methyl (E)-3-(3-methoxy-4-trifluoromethoxyphenyl)acrylate obtained in oil form is used in the next step without further purification.

### d. Methyl (E)-3-(2-methoxy-3'-methylaminobiphenyl-4-yl)acrylate

27 mg (5 mol%) of palladium acetate and 84 mg (10 mol%) of dicyclohexylbiphenylphosphine are added to a solution of methyl (E)-3-(3-methoxy-4-trifluoromethoxyphenyl)acrylate (2.40 mmol, 1 eq) obtained in the preceding step, 435 mg (2.88 mmol, 1.2 eq) of 3-(methylamino)phenylboronic acid (prepared in step 18a) in 4 ml of dimethylformamide + 1 ml of 2M potassium phosphate solution. The mixture is heated at 90°C for 3 hours. The reaction medium is hydrolysed in water and then extracted with ethyl acetate. The organic phases are washed with saturated sodium chloride solution and dried over sodium sulfate. The solvent is evaporated off and the residual oil is then chromatographed on silica gel (7/3 heptane/ethyl acetate). 560 mg of methyl (E)-3-(2-methoxy-3'-methylaminobiphenyl-4-yl)acrylate are obtained in oil form. Yield = 78%

### e. Methyl 3-(2-methoxy-3'-methylaminobiphenyl-4-yl)propanoate

100 mg (18% by mass) of 10% palladium-on-charcoal are added to a solution of 560 mg (1.88 mmol, 1 eq) of methyl (E)-3-(2-methoxy-3'-methylaminobiphenyl-4-yl)acrylate in 10 ml of methanol. The reaction mixture is stirred at room temperature under a hydrogen atmosphere for 4 hours. The reaction medium is filtered through Celite and then evaporated to dryness. 495 mg of methyl 3-(2-methoxy-3'-methylaminobiphenyl-4-yl)propanoate are obtained in oil form. Yield = 88%

### f. Methyl 3-[3'-(3-heptyl-1-methylureido)-2-methoxybiphenyl-4-yl]propanoate

540 µL (3.3 mmol, 2 eq) of heptyl isocyanate are added to a solution of 495 mg (1.65 mmol, 1 eq) of methyl 3-(2-methoxy-3'-methylaminobiphenyl-4-yl)propanoate in 10 mL of an 8/2 tetrahydrofuran/triethylamine mixture. The reaction mixture is stirred for 12 hours at room temperature. The reaction medium is hydrolysed in water and then extracted with ethyl acetate. The organic phases are combined and dried over sodium sulfate. The solvents are evaporated off and the residue is then chromatographed on silica gel (70/30 heptane/ethyl acetate). 525 mg of methyl 3-[3'-(3-heptyl-1-methylureido)-2-methoxybiphenyl-4-yl]propanoate are obtained in oil form. Yield = 72%

### g. 3-[3'-(3-Heptyl-1-methylureido)-2-methoxybiphenyl-4-yl]propanoic acid

200 mg (5.0 mmol, 4.2 eq) of sodium hydroxide are added to a solution of 525 mg (1.19 mmol, 1 eq) of methyl 3-[3'-(3-heptyl-1-methylureido)-2-methoxybiphenyl-4-yl]propanoate in 6 ml of a tetrahydrofuran/methanol mixture (8/2). The reaction mixture is stirred at room temperature for 3 hours. The reaction medium is hydrolysed with water, acidified with acetic acid solution and extracted with ethyl acetate. The organic phases are combined, washed with sodium chloride solution and dried over sodium sulfate. The solvent is evaporated off and the residual oil is chromatographed on silica (7/3 to 1/1 heptane/ethyl acetate). The product is crystallized from pentane and 216 mg of 3-[3'-(3-heptyl-1-methylureido)-2-methoxybiphenyl-4-yl]propanoic acid are obtained in the form of a white powder (m:p. = 100-101 °C). Yield = 42%

¹H NMR (CDCl₃, 400 MHz) : 0.87 (t, 3H); 1.25 (m, 8H); 1.44 (m, 2H); 2.76 (t, 2H); 3.04 (t, 2H); 3.18 (q, 2H) ; 3.32 (s, 3H); 3.84 (s, 3H); 4.53 (t, 1H); 6.88 (s, 1H); 6.92 (d, 1H); 7.19 (m, 1H); 7.25 (d, 1H); 7.43-7.46 (m, 3H).

### Example 19 : 3-[3'-(3-Heptyl-1-methylureido)-2-methylbiphenyl-4-yl]propanoic acid

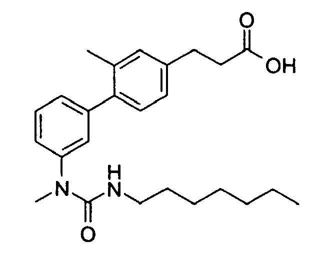

### a. (4-Bromo-3-methylphenyl)methanol

284 mg (12.9 mmol, 3 eq) of lithium borohydride are added to a solution of 1.0 g (4.3 mmol, 1 eq) of methyl 3-methyl-4-bromobenzoate in 10 ml of tetrahydrofuran. The reaction mixture is stirred at 60°C for 12 hours. The reaction medium is hydrolysed with ammonium chloride solution and extracted with ethyl acetate. The organic phases are combined, washed with sodium chloride solution and dried over sodium sulfate. 900 mg of (4-bromo-3-methylphenyl)methanol are obtained in oil form and used in the following reaction without further purification.

### b. 4-Bromo-3-methylbenzaldehyde

3.7 g (43 mmol, 10 eq) of manganese dioxide are added to a solution of 900 mg (4.3 mmol, 1 eq) of (4-bromo-3-methylphenyl)methanol in 8 mL of dichloromethane. The reaction mixture is stirred for 12 hours at room temperature. The solid is filtered off and the solvent is evaporated off. 900 mg of 4-bromo-3-methylbenzaldehyde are obtained in oil form and used in the following reaction without further purification.

### c. Methyl (E)-3-(4-bromo-3-methylphenyl)acrylate

2.1 g (6.45 mmol, 1.5 eq) of methyl triphenylphosphoranylideneacetate are added to a solution of 900 mg of 4-bromo-3-methylbenzaldehyde in 5 ml of toluene. The reaction mixture is heated at 90°C for 1 hour. The solvent is evaporated off and the residual oil is chromatographed on silica gel (8/2 heptane/ethyl acetate). 610 mg of methyl (E)-3-(4-bromo-3-methylphenyl)acrylate are obtained in oil form.
Yield = 55% over steps a, b and c.

### d. Methyl (E)-3-(2-methyl-3'-methylaminobiphenyl-4-yl)acrylate

In a manner similar to that of Example (18d), by reaction of 44 mg (10 mol%) of palladium acetate, 136 mg (20 mol%) of dicyclohexylbiphenylphosphine, 500 mg (1.96 mmol, 1 eq) of methyl (E)-3-(4-bromo-3-methylphenyl)acrylate and 355 mg (2.35 mmol, 1.2 eq) of 3-(methylamino)phenylboronic acid (prepared in Example 18a) in 4 ml of dimethylformamide + 1 ml of 2M potassium phosphate solution, 390 mg of methyl (E)-3-(2-methyl-3'-methylaminobiphenyl-4-yl)acrylate are obtained in oil form. Yield = 70%

### e. Methyl 3-(2-methyl-3'-methylaminobiphenyl-4-yl)propanoate

In a manner similar to that of Example (18e), by reaction of 100 mg (25% by mass) of 10% palladium-on-charcoal and 390 mg (1.39 mmol, 1 eq) of methyl (E)-3-(2-methyl-3'-methylaminobiphenyl-4-yl)acrylate in 10 ml of methanol, 360 mg of methyl 3-(2-methyl-3'-methylaminobiphenyl-4-yl)propanoate are obtained in oil form. Yield = 92%

### f. Methyl 3-[3'-(3-heptyl-1-methylureido)-2-methylbiphenyl-4-yl]propanoate

In a manner similar to that of Example (18f), by reaction of 410 µL (2.54 mmol, 2 eq) of heptyl isocyanate and 360 mg (1.27 mmol, 1 eq) of methyl 3-(2-methyl-3'-methylaminobiphenyl-4-yl)propanoate in 8 mL of an 8/2 tetrahydrofuran/triethylamine mixture, 360 mg of methyl 3-[3'-(3-heptyl-1-methylureido)-2-methylbiphenyl-4-yl]propanoate are obtained in oil form. Yield = 66%

### g. 3-[3'-(3-Heptyl-1-methylureido)-2-methylbiphenyl-4-yl]propanoic acid

200 mg (5.0 mmol, 4.2 eq) of sodium hydroxide are added to a solution of 360 mg (0.85 mmol, 1 eq) of methyl 3-[3'-(3-heptyl-1-methylureido)-2-methylbiphenyl-4-yl]propanoate in 6 ml of a tetrahydrofuran/methanol mixture (8/2). The reaction mixture is stirred at room temperature for 3 hours. The reaction medium is hydrolysed with water, acidified with acetic acid solution and extracted with ethyl acetate. The organic phases are combined, washed with sodium chloride solution and dried over sodium sulfate. The solvent is evaporated off and the residual oil is chromatographed on silica (7/3 to 1/1 heptane/ethyl acetate). 185 mg of 3-[3'-(3-heptyl-1-methylureido)-2-methylbiphenyl-4-yl]propanoic acid are obtained in oil form. Yield = 53%

¹H NMR (CDCl₃, 400 MHz): 0.87 (t, 3H); 1.25 (m, 8H); 1.42 (m, 2H); 2.28 (s, 3 H); 2.75 (t, 2H); 3.00 (t, 2H); 3.19 (q, 2H); 3.31 (s, 3H); 4.42 (t, 1H); 7.12-7.18 (m, 3H); 7.24 (m, 3H); 7.47 (t, 1H).

### Example 20 : 3-[3'-[1-Methyl-3-(3-phenylpropyl)ureido]biphenyl-4-yl]propanoic acid

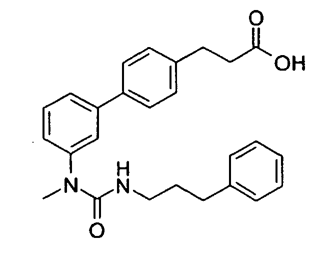

### a. Ethyl 3-{3'-[1-methyl-3-(3-phenyloropyl)ureido]biphenyl-4-yl}prooanoate

188 µL (1.2 mmol, 1.5 eq) 3-phenylpropyl isocyanate are added to a solution of 230 mg (0.81 mmol, 1 eq) of ethyl 3-(3'-methylaminobiphenyl-4-yl)propanoate (prepared in Example 7c) in 10 mL of an 8/2 dichloromethane/triethylamine mixture. The reaction medium is stirred at 40°C for 12 hours and then hydrolysed with water and extracted with ethyl acetate. The organic phases are combined, washed with sodium chloride solution and dried over sodium sulfate. The solvents are evaporated off and the residual oil is chromatographed on silica (7/3 heptane/ethyl acetate). 348 mg of ethyl 3-{3'-[1-methyl-3-(3-phenylpropyl)ureido]biphenyl-4-yl}propanoate are obtained in solid form. Yield = 96%

### b. 3-[3'-(3-Heptyl-1-methylureido)-2-methylbiphenyl-4-yl]propanoic acid

100 mg (2.5 mmol, 3.2 eq) of sodium hydroxide are added to a solution of 348 mg (0.78 mmol, 1 eq) of ethyl 3-{3'-[1-methyl-3-(3-phenylpropyl)ureido]biphenyl-4-yl}propanoate in 6 ml of a tetrahydrofuran/methanol mixture (8/2). The reaction mixture is stirred at room temperature for 12 hours. The reaction medium is hydrolysed with water, acidified with acetic acid solution and extracted with ethyl acetate. The organic phases are combined, washed with sodium chloride solution and dried over sodium sulfate. The solvent is evaporated off and the residual oil is chromatographed on silica (7/3 to 1/1 heptane/ethyl acetate). 180 mg of 3-[3'-(3-heptyl-1-methylureido)-2-methylbiphenyl-4-yl]propanoic acid are obtained in the form of a tacky solid. Yield = 55%

¹H NMR (CDCl₃, 400 MHz) : 1.76-1.82 (m, 2H); 2.59 (t, 2H); 2.75 (t, 2H); 3.04 (t, 2H); 3.24 (q, 2H); 3.32 (s, 3H); 4.44 (t, 1H); 7.10- 7.24 (m, 6H); 7.33 (d, 2H); 7.47-7.53 (m, 5H).

### Example 21 : (E)-3-[5'-(3-Heptyl-1-methylureido)-2'-methylbiphenyl-4-yl]acrylic acid

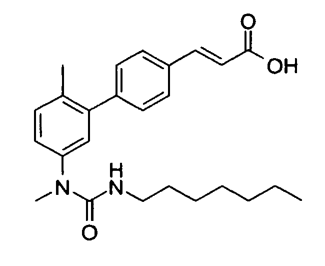

### a. tert-Butyl (3-bromo-4-methylphenyl)carbamate

57.62 g (0.3 mol, 1 eq) of 3-bromo-4-methylaniline are added to a solution of 75.8 g (0.344 mol, 1.15 eq) of di-tert-butyl dicarbonate in 580 mL of 2N sodium hydroxide solution. The reaction medium is refluxed for one hour and stirred at room temperature for 4 hours. The reaction medium is filtered and the solid is washed with water until the filtrate is neutral, and then dried. 90.33 g of tert-butyl (3-bromo-4-methylphenyl)carbamate are obtained in solid form.
Crude yield > 100%

### b. tert-Butyl (3-bromo-4-methylphenyl)methy carbamate

1.6 g (0.042 mol, 1.2 eq) of 60% sodium hydride are added to a solution of 10.0 g (0.035 mol, 1 eq) of tert-butyl (3-bromo-4-methylphenyl)carbamate in 150 ml of dimethylformamide. The reaction medium is stirred at room temperature for 2 hours and 6.2 mL (0.099 mol, 3 eq) of methyl iodide are then added. The reaction medium is stirred at room temperature for 20 hours and then hydrolysed in water and extracted with ethyl acetate. The organic phases are combined, washed with water and dried over sodium sulfate. The solvent is evaporated off and 10.4 g of tert-butyl (3-bromo-4-methylphenyl)methyl carbamate are obtained. Yield = 100%

### c. (3-Bromo-4-methylphenyl)methylamine

A solution of 10.4 g (0.035 mol, 1 eq) of tert-butyl (3-bromo-4-methylphenyl)methyl carbamate in 25 mL of dichloromethane in the presence of 13.5 mL (0.173 mol, 5 eq) of trifluoroacetic acid is stirred at room temperature for 30 hours. The reaction medium is hydrolysed in water, basified to pH 8-9 with 1N sodium hydroxide solution and extracted with dichloromethane. The organic phases are combined and dried over sodium sulfate. The solvent is evaporated off and 6.39 g of (3-bromo-4-methylphenyl)methylamine are obtained. Yield = 91%

### d. Metyl[4-methyl-3-(4,4,5,5-tetramethyl[1,3,2]dioxaborolan-2-yl)phenyl]amine

In a manner similar to that of Example (7a), by reaction of 3.2 g (0.016 mol, 1 eq) of (3-bromo-4-methylphenyl)methylamine, 4.06 g (0.016 mol, 1 eq) of pinacol diborane and 4.7 g (0.048 mol, 3 eq) of potassium acetate in the presence of 650 mg (5 mol%) of diphenylphosphinoferrocenepalladium dichloride in 20 ml of dimethylformamide, at 90°C for 8 hours, 3.28 g of methyl[4-methyl-3-(4,4,5,5-tetramethyl[1,3,2]dioxaborolan-2-yl)phenyl]amine are obtained. Yield = 83%

### e. Ethyl (E)-3-(2'-methyl-5'-methylaminobiphenyl-4-yl)acrylate

In a manner similar to that of Example (18d), by reaction of 45 mg (5 mol%) of palladium acetate, 140 mg (10 mol%) of dicyclohexylbiphenylphosphine, 1.1 g (4.4 mmol, 1.1 eq) of of ethyl 4-bromocinnamate and 1.0 g (4.0 mmol, 1 eq) of methyl[4-methyl-3-(4,4,5,5-tetramethyl[1,3,2]dioxaborolan-2-yl)phenyl]amine in 18 ml of dimethylformamide + 3 ml of 2M potassium phosphate solution, 805 mg of ethyl (E)-3-(2'-methyl-5'-methylaminobiphenyl-4-yl)acrylate are obtained in oil form. Yield = 68%

### f. Ethyl (E)-3-[5'-(3-heptyl-1-methylureido)-2'-methylbiphenyl-4-yl]acrylate

660 µL (4.0 mmol, 1.5 eq) of heptyl isocyanate are added to a solution of 805 mg (2.72 mmol, 1 eq) of ethyl (E)-3-(2'-methyl-5'-methylaminobiphenyl-4-yl)acrylate in 8 mL of a 7/1 dichloromethane/triethylamine mixture. The reaction medium is stirred at 50°C for 12 hours and then hydrolysed with water and extracted with ethyl acetate. The organic phases are combined, washed with sodium chloride solution and dried over sodium sulfate. The solvents are evaporated off and the residual oil is chromatographed on silica (7/3 heptane/ethyl acetate). 920 mg of ethyl (E)-3-[5'-(3-heptyl-1-methylureido)-2'-methylbiphenyl-4-yl]acrylate are obtained in oil form. Yield = 77%

### g. (E)-3-[5'-(3-Heptyl-1-methylureido)-2'-methylbiphenyl-4-yl]acrylic acid

In a manner similar to that of Example (19g), by reaction of 200 mg (5.0 mmol, 4.7 eq) of sodium hydroxide and 460 mg (1.05 mmol, 1 eq) of ethyl (E)-3-[5'-(3-heptyl-1-methylureido)-2'-methylbiphenyl-4-yl]acrylate in 6 ml of a tetrahydrofuran/methanol mixture (8/2), and after crystallization from a dichloromethane/pentane mixture, 160 mg of (E)-3-[5'-(3-heptyl-1-methylureido)-2'-methylbiphenyl-4-yl]acrylic acid are obtained in the form of a white powder. (m.p. = 159°C). Yield = 37%

¹H NMR (CDCl₃, 400 MHz) : 0.87 (t, 3H); 1.25 (m, 8H); 1.43 (m, 2H); 2.32 (s, 3 H); 3.19 (q, 2H) ; 3.30 (s, 3H); 4.43 (t, 1H); 6.52-6.56 (d, 1H); 7.15-7.20 (m, 2H); 7.33-7.39 (m, 3H); 7.64 (d, 2H); 7.83-7.87 (d, 1H).

### Example 22 : 3-[5'-(3-Heptyl-1-methylureido)-2'-methylbiphenyl-4-yl]propanoic acid

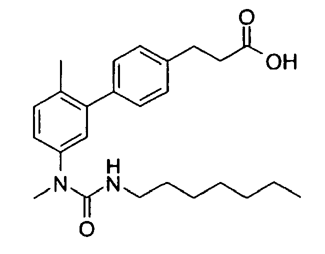

### a. Ethyl 3-[5'-(3-heptyl-1-methylureido)-2'-methylbiphenyl-4-yl]propanoate

A solution of 460 mg of ethyl (E)-3-[5'-(3-heptyl-1-methylureido)-2'-methylbiphenyl-4-yl]acrylate (prepared in step 21f) in 10 ml of methanol is stirred for 12 hours at room temperature in the presence of 100 mg (22% by mass) of 10% palladium-on-charcoal under a hydrogen atmosphere. The palladium is filtered off and the solvent is evaporated off. The oil is used directly in the following step.

### b. 3-[5'-(3-Heptyl-1-methylureido)-2'-methylbiphenyl-4-yl]propanoic acid

In a manner similar to that of Example (19g), by reaction of 200 mg (5.0 mmol, 4.7 eq) of sodium hydroxide, the oil obtained above (ethyl 3-[5'-(3-heptyl-1-methylureido)-2'-methylbiphenyl-4-yl]propanoate) in 6 ml of a tetrahydrofuran/methanol mixture (8/2), and after crystallization from pentane, 170 mg of 3-[5'-(3-heptyl-1-methylureido)-2'-methylbiphenyl-4-yl]propanoic acid are obtained in the form of a white powder. (m.p. = 104°C). Yield = 39% over the two steps a and b.

¹H NMR (CDCl₃, 400 MHz) : 0.87 (t, 3H); 1.28 (m, 8H); 1.42 (m, 2H); 2.30 (s, 3 H); 2.76 (t, 2H); 3.05 (t, 2H); 3.18 (q, 2H) ; 3.28 (s, 3H); 4.44 (t, 1H); 7.12-7.15 (m, 2H); 7.24-7.32 (m, 5H).

### Example 23 : (E)-3-[3-Fluoro-3'-(3-heptyl-1-methylureido)biphenyl-4-yl]acrylic acid

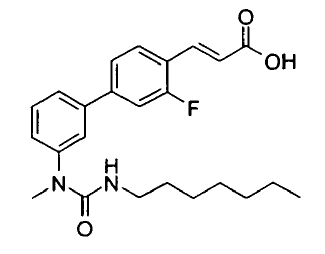

In a manner similar to that of Example (19g), by reaction of 200 mg (5.0 mmol, 4.7 eq) of sodium hydroxide, 210 mg (0.51 mmol, 1 eq) of methyl (E)-3-[3-fluoro-3'-(3-heptyl-1-methylureido)biphenyl-4-yl]acrylate (prepared in Example 3b) in 6 ml of a tetrahydrofuran/methanol mixture (8/2), and after crystallization from pentane, 165 mg of (E)-3-[3-fluoro-3'-(3-heptyl-1-methylureido)biphenyl-4-yl]acrylic acid are obtained in the form of a white powder. (m.p. = 148-149°C). Yield = 81%
¹H NMR (CDCl₃, 400 MHz) : 0.87 (t, 3H); 1.25 (m, 8H); 1.44 (m, 2H); 3.21 (q, 2H) ; 3.35 (s, 3H); 4.40 (t, 1H); 6.61-6.66 (d, 1H); 7.30-7.33 (m, 3H); 7.36-7.38 (m, 3H); 7.52-7.55 (t, 1H); 7.94-7.98 (d, 1H).

### Example 24 : (E)-3-[2-Benzyloxy-3'-(3-heptyl-1-methylureido)biphenyl-4-yl]acrylic acid

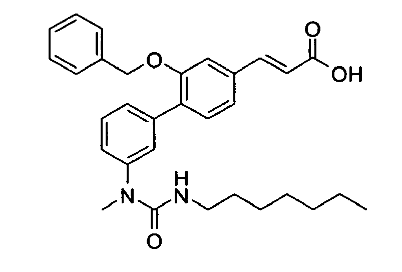

### a. Methyl 3-benzyloxy-4-iodobenzoate

2.35 mL (19.78 mmol, 1.1 eq) of benzyl bromide are added to a solution of 5.0 g (17.98 mmol, 1 eq) of methyl 3-hydroxy-4-iodobenzoate in 30 ml of methyl ethyl ketone in the presence of 5.0 g of potassium carbonate. The reaction medium is heated at 60°C for 5 hours and then hydrolysed with water and extracted with ethyl acetate. The organic phases are combined and the solvents are evaporated off. 6.9 g of methyl 3-benzyloxy-4-iodobenzoate in oil form are used in the following reaction without further purification.

### b. (3-Benzyloxy-4-iodophenyl)methanol

1.18 g (54 mmol, 3 eq) of lithium borohydride are added to a solution of 6.9 g of methyl 3-benzyloxy-4-iodobenzoate in 30 ml of tetrahydrofuran. The reaction medium is heated at 60°C for 12 hours and then hydrolysed with ammonium chloride solution and extracted with ethyl acetate. The organic phases are combined and the solvents are evaporated off. 6.5 g of (3-benzyloxy-4-iodophenyl)methanol in oil form are used in the following reaction without further purification.

### c. 3-Benzyloxv-4-iodobenzaldehyde

7.8 g (90 mmol, 5 eq) of manganese dioxide are added to a solution of 6.5 g of (3-benzyloxy-4-iodophenyl)methanol in 50 mL of dichloromethane. The reaction medium is stirred at room temperature for 12 hours.

The solid is filtered off and the solvent is evaporated off. The residual oil is chromatographed on silica gel (8/2 heptane/ethyl acetate) and then crystallized from pentane. 3.25 g of 3-benzyloxy-4-iodobenzaldehyde are obtained in solid form. Yield = 54% over the three steps a, b and c.

### d. Methyl (E)-3-(3-benzvloxy-4-iodophenyl)acrylate

5.0 g (14.95 mmol, 1.8 eq) of methyl triphenylphosphoranylideneacetate are added to a solution of 3.25 g (8.2 mmol, 1 eq) of 3-benzyloxy-4-iodobenzaldehyde in 15 ml of toluene. The reaction mixture is stirred for 1 hour at 90°C. The solvent is evaporated off and the residual oil is then chromatographed on silica gel (8/2 heptane/ethyl acetate) and recrystallized from dichloromethane/heptane. 2.59 g of methyl (E)-3-(3-benzyloxy-4-iodophenyl)acrylate are obtained in solid form. Yield = 68%

### e. Methyl (E)-3-[2-benzyloxy-3'-(3-heptyl-1-methylureido)biphenyl-4-yl]acrylate

In a manner similar to that of Example (18d), by reaction of 50 mg (10 mol%) of palladium acetate, 180 mg (20 mol%) of dicyclohexylbiphenylphosphine, 1.0 g (2.53 mmol, 1 eq) of methyl (E)-3-(3-benzyloxy-4-iodophenyl)acrylate and 1.0 g (2.79 mmol, 1.1 eq) of 3-heptyl-1-methyl-1-[3-(4,4,5,5-tetramethyl[1,3,2]dioxaborolan-2-yl)phenyl]urea (prepared in Example 1e) in 8 ml of dimethylformamide + 2 ml of 2M potassium phosphate solution, 980 mg of methyl (E)-3-[2-benzyloxy-3'-(3-heptyl-1-methylureido)biphenyl-4-yl]acrylate are obtained in oil form.
Yield = 75%

### f. (E)-3-[2-Benzyloxy-3'-(3-heptyl-1-methylureido)biphenyl-4-yl]acrylic acid

In a manner similar to that of Example (19g), by reaction of 200 mg (5.0 mmol, 12.8 eq) of sodium hydroxide and 200 mg (0.39 mmol, 1 eq) of methyl (E)-3-[2-benzyloxy-3'-(3-heptyl-1-methylureido)biphenyl-4-yl]acrylate in 6 ml of a tetrahydrofuran/methanol mixture (8/2), and after crystallization from pentane, 125 mg of (E)-3-[2-benzyloxy-3'-(3-heptyl-1-methylureido)biphenyl-4-yl]acrylic acid are obtained in the form of a white powder (m.p. = 115-116°C). Yield = 64%

¹H NMR (CDCl₃, 400 MHz) : 0.86 (t, 3H); 1.26 (m, 8H); 1.38 (m, 2H); 3.12 (q, 2H) ; 3.24 (s, 3H); 4.41 (t, 1H); 5.16 (s, 2H); 6.48-6.52 (d, 1H); 7.23-7.53 (m, 12H); 7.79-7.83 (d, 1H).

### Example 25 : 3-[3'-(3-Heptyl-1-methylureido)-2-propoxybiphenyl-4-yl]propanoic acid

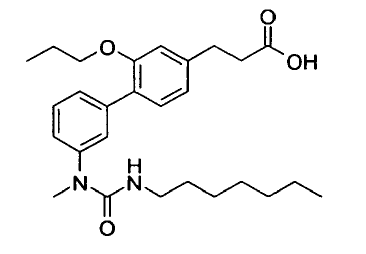

### a. Methyl 3-[3'-(3-heptyl-1-methylureido)-2-propoxybiphenyl-4-yl]propanoate

100 µL (1.0 mmol, 1.5 eq) of 1-iodopropane are added to a solution of 285 mg (0.67 mmol, 1 eq) of methyl 3-[3'-(3-heptyl-1-methylureido)-2-hydroxybiphenyl-4-yl]propanoate (prepared in Example 15f) in 6 ml of methyl ethyl ketone in the presence of 200 mg (1.45 mmol, 2.2 eq) of potassium carbonate. The reaction medium is heated at 60°C for 16 hours. The solid is filtered off and the solvent is evaporated off. The methyl 3-[3'-(3-heptyl-1-methylureido)-2-propoxybiphenyl-4-yl]propanoate obtained in oil form is used in the following reaction without further purification.

### b. 3-[3'-(3-Heptyl-1-methylureido)-2-propoxybiphenyl-4-yl]propanoic acid

In a manner similar to that of Example (19g), by reaction of 200 mg (5.0 mmol, -8 eq) of sodium hydroxide and methyl 3-[3'-(3-heptyl-1-methylureido)-2-propoxybiphenyl-4-yl]propanoate, prepared in the preceding step, in 6 ml of a tetrahydrofuran/methanol mixture (8/2), and after crystallization from pentane, 180 mg of 3-[3'-(3-heptyl-1-methylureido)-2-propoxybiphenyl-4-yl]propanoic acid are obtained in the form of a white powder (m.p. = 79-80°C). Yield = 59% over the two steps b and c.

¹H NMR (CDCl₃, 400 MHz) : 0.87 (t, 3H); 0.98 (t, 3H); 1.23-1.29 (m, 8H); 1.41 (m, 2H); 1.72-1.79 (m, 2H); 2.75 (t, 2H); 3.02 (t, 2H); 3.17 (q, 2H); 3.30 (s, 3H); 3.96 (t, 2H); 4.43 (t, 1 H); 6.86 (s, 1 H); 6.90 (d, 1H); 7.19 (d, 1 H); 7.26 (d, 1 H); 7.42-7.49 (m, 3H).

### Example 26 : 3-[2-Butoxy-3'-(3-heptyl-1-methylureido)biphenyl-4-yl]propanoic acid

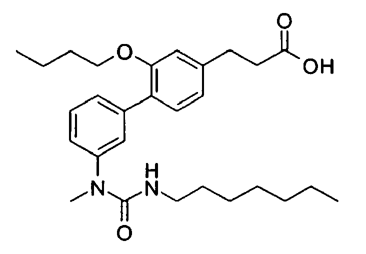

### a. Methyl 3-[2-butoxy-3'-(3-heptyl-1-methylureido)biphenyl-4-yl]propanoate

In a manner similar to that of Example (25a), by reaction of 114 µL (1.0 mmol, 1.5 eq) of 1-iodobutane and 285 mg (0.67 mmol, 1 eq) of methyl 3-[3'-(3-heptyl-1-methylureido)-2-hydroxybiphenyl-4-yl]propanoate (prepared in Example 15f) in 6 ml of methyl ethyl ketone in the presence of 200 mg of potassium carbonate, methyl 3-[2-butoxy-3'-(3-heptyl-1-methylureido)biphenyl-4-yl]propanoate is obtained in oil form and is used in the following reaction without further purification.

### b. 3-[2-Butoxy-3'-(3-heptyl-1-methylureido)biphenyl-4-yl]propanoic acid

In a manner similar to that of Example (19g), by reaction of 200 mg (5.0 mmol, ∼8 eq) of sodium hydroxide and methyl 3-[2-butoxy-3'-(3-heptyl-1-methylureido)biphenyl-4-yl]propanoate, prepared in the preceding step, in 6 ml of a tetrahydrofuran/methanol mixture (8/2), and after crystallization from pentane, 200 mg of 3-[2-butoxy-3'-(3-heptyl-1-methylureido)biphenyl-4-yl]propanoic acid are obtained in the form of a white powder (m.p. = 77-78°C). Yield = 64% over the two steps a and b.

¹H NMR (CDCl₃, 400 MHz) : 0.87 (t, 3H); 0.93 (t, 3H); 1.24 (m, 8H); 1.38-1.46 (m, 4H); 1.69-1.76 (m, 2H); 2.75 (t, 2H); 3.02 (t, 2H); 3.17 (q, 2H) ; 3.31 (s, 3H); 3.99 (t, 2H); 4.43 (t, 1 H); 6.87 (s, 1 H); 6.90 (d, 1H); 7.19 (d, 1 H); 7.26 (d, 1H); 7.44-7.49 (m, 3H).

### Example 27 : (E)-3-[3'-(1-Ethyl-3-heptylureido)biphenyl-4-yl]acrylic acid

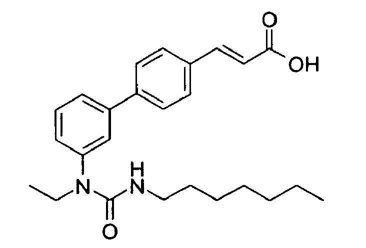

### a. N-(3-Bromophenyl)acetamide

691 mL of acetic anhydride are added to a solution cooled to 10°C of 1200 g (6.976 mol, 1 eq) of 3-bromoaniline in 6 L of dichloromethane in the presence of 1.07 L (7.67 mol, 1.1 eq) of triethylamine and 25.6 g (0.209 mol, 0.03 eq) of 4-(dimethylamino)pyridine. The reaction medium is stirred at room temperature for 4 hours and then hydrolysed with 850 mL of 1 N hydrochloric acid solution, and the phases are separated by settling. The organic phase is washed with 0.5N hydrochloric acid solution and then with water, and the solvent is evaporated off. After recrystallization from diisopropyl ether/heptane, 1506.1 g of N-(3-bromophenyl)acetamide are obtained in solid form. Yield = 100%

### b. N-(3-Bromophenyl)-N-ethylacetamide

820 mg (20.5 mmol, 1.1 eq) of 60% sodium hydride are added to a solution of 4.0 g (18.7 mmol, 1eq) of N-(3-bromophenyl)acetamide and 1.80 mL (22.4 mmol, 1.2 eq) of ethyl iodide in 15 ml of tetrahydrofuran in the presence of 1.5 ml of dimethylformamide. The reaction medium is stirred at room temperature for 12 hours and then hydrolysed with water and extracted with ethyl acetate. The organic phases are combined, washed with sodium chloride solution and dried over sodium sulfate. The solvents are evaporated off and the N-(3-bromophenyl)-N-ethylacetamide obtained in oil form is used in the following reaction without further purification.

### c. (3-Bromophenyl)ethylamine

10 ml of 2.5 M potassium hydroxide solution are added to a solution of N-(3-bromophenyl)-N-ethylacetamide (obtained in the preceding step) in 10 ml of ethanol. The reaction medium is refluxed for 12 hours and then hydrolysed with water and extracted with ethyl acetate. The organic phases are combined, washed with sodium chloride solution and dried over sodium sulfate. The solvents are evaporated off and the residual oil is chromatographed on silica (7/3 heptane/ethyl acetate). 2.3 g of (3-bromophenyl)ethylamine are obtained in oil form. Yield = 62% over the two steps.

### d. 1-(3-Bromophenyl)-1-ethyl-3-heptylurea

In a manner similar to that of Example (11ac), by reaction of 1.6 mL (10 mmol, 2 eq) of heptyl isocyanate and 1.0 g (5.0 mmol, 1 eq) of (3-bromophenyl)ethylamine, 1.6 g of 1-(3-bromophenyl)-1-ethyl-3-heptylurea are obtained. Yield = 94%

### e. 1-Ethyl-1-(4'-formylbiphenyl-3-yl)-3-heptylurea

135 mg (5 mol%) of tetrakis(triphenylphosphine)palladium are added to a solution of 800 mg (2.34 mmol, 1 eq) of 1-(3-bromophenyl)-1-ethyl-3-heptylurea and 460 mg (3.0 mmol, 1.3 eq) of 4-formylphenylboronic acid in 10 mL of a mixture of dimethylformamide and of 2M potassium phosphate solution (8/2). The reaction mixture is stirred for 3 hours at 90°C. The reaction is stopped by addition of 50 mL of water and then extracted with ethyl acetate. The organic phases are combined and dried over sodium sulfate. The solvents are evaporated off and the residue is then chromatographed on silica gel (70/30 heptane/ethyl acetate). 740 mg of 1-ethyl-1-(4'-formylbiphenyl-3-yl)-3-heptylurea are obtained in oil form. Yield = 86%

### f. Methyl (E)-3-[3'-(1-ethyl-3-heptylureido)biphenyl-4-yl]acrylate

1.0 g (3 mmol, 1.5 eq) of methyl triphenylphosphoranylideneacetate are added to a solution of 740 mg (2.02 mmol, 1 eq) of 1-ethyl-1-(4'-formylbiphenyl-3-yl)-3-heptylurea in 8 ml of toluene. The reaction mixture is heated at 90°C for 1 hour. The solvent is evaporated off and the residual oil is then chromatographed on silica gel (8/2 heptane/ethyl acetate). 775 mg of methyl (E)-3-[3'-(1-ethyl-3-heptylureido)biphenyl-4-yl]acrylate are obtained in the form of an off-white solid. Yield = 91 %

### g. (E)-3-[3'-(1-Ethyl-3-heptylureido)biphenyl-4-yl]acrylic acid

In a manner similar to that of Example (19g), by reaction of 400 mg (10 mmol, 5.5 eq) of sodium hydroxide and 775 mg (1.83 mmol, 1 eq) of methyl (E)-3-[3'-(1-ethyl-3-heptylureido)biphenyl-4-yl]acrylate in 15 ml of a tetrahydrofuran/methanol mixture (8/2), and after crystallization from dichloromethane/heptane, 580 mg of (E)-3-[3'-(1-ethyl-3-heptylureido)biphenyl-4-yl]acrylic acid are obtained in the form of a white powder (m.p. = 116°C). Yield = 77%

¹H NMR (CDCl₃, 400 MHz) : 0.87 (t, 3H); 1.16 (t, 3H); 1.24 (m, 8H); 1.42 (m, 2H); 3.19 (m, 2H) ; 3.82 (m, 2H); 4.21 (m, 1 H); 6.52-6.56 (d, 1 H); 7.26 (m, 2H); 7.50-7.66 (m, 6H); 7.83-7.87 (d, 1H).

### Example 28 : (E)-3-[3'-(3-Heptyl-1-propylureido)biphenyl-4-yl]acrylic acid

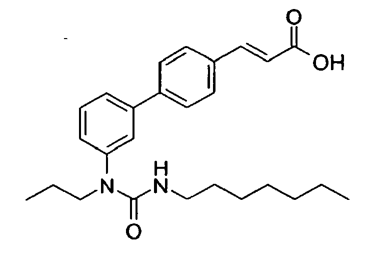

### a. N-(3-Bromophenyl)-N-propylacetamide

In a manner similar to that of Example (27b), by reaction of 820 mg (20.5 mmol, 1.1 eq) of 60% sodium hydride, 4.0 g (18.7 mmol, 1 eq) of N-(3-bromophenyl)acetamide (prepared in Example 27a) and 2.20 mL (22.4 mmol, 1.2 eq) of 1-iodopropane in 15 ml of tetrahydrofuran in the presence of 1.5 ml of dimethylformamide, N-(3-bromophenyl)-N-propylacetamide is obtained in oil form and is used in the following reaction without further purification.

### b. (3-Bromophenyl)propylamine

In a manner similar to that of Example (27c), by reaction of 10 mL of 2.5 M potassium hydroxide solution and N-(3-bromophenyl)-N-propylacetamide (obtained in the preceding step) in 10 mL of ethanol, 2.0 g of (3-bromophenyl)propylamine are obtained in oil form. Yield = 50% over the two steps a and b.

### c. 1-(3-Bromophenyl)-1-propyl-3-heptylures

In a manner similar to that of Example (11a), by reaction of 1.6 mL (10 mmol, 2.1 eq) of heptyl isocyanate and 1.0 g (4.7 mmol, 1 eq) of (3-bromophenyl)propylamine, 1.45 g of 1-(3-bromophenyl)-1-propyl-3-heptylurea are obtained. Yield = 90%

### d. 1-Propyl-1-(4'-formylbiphenyl-3-yl)-3-heptylurea

135 mg (5 mol%) of tetrakis(triphenyl-phosphine)palladium are added to a solution of 830 mg (2.34 mmol, 1 eq) of 1-(3-bromophenyl)-1-propyl-3-heptylurea and 460 mg (3.0 mmol, 1.3 eq) of 4-formylphenylboronic acid in 10 mL of a mixture of dimethylformamide and of 2M potassium phosphate solution (8/2). The reaction mixture is stirred for 3 hours at 90°C. The reaction is stopped by addition of 50 mL of water and then extracted with ethyl acetate. The organic phases are combined and dried over sodium sulfate. The solvents are evaporated off and the residue is then chromatographed on silica gel (70/30 heptane/ethyl acetate). 735 mg of 1-propyl-1-(4'-formylbiphenyl-3-yl)-3-heptylurea are obtained in oil form. Yield = 82%

### e. Methyl (E)-3-[3'-(1-propyl-3-heptylureido)biphenyl-4-yl]acrylate

In a manner similar to that of Example (27f), by reaction of 1.0 g (3 mmol, 1.5 eq) of methyl triphenylphosphoranylideneacetate and 735 mg (1.93 mmol, 1 eq) of 1-propyl-1-(4'-formylbiphenyl-3-yl)-3-heptylurea in 8 ml of toluene, 680 mg of methyl (E)-3-[3'-(1-propyl-3-heptylureido)biphenyl-4-yl]acrylate are obtained in the form of an off-white solid. Yield = 81%

### f. (E)-3-[3'-(1-Propyl-3-heptylureido)biphenyl-4-yl]acrylic acid

In a manner similar to that of Example (19g), by reaction of 400 mg (10 mmol, 6.4 eq) of sodium hydroxide and 680 mg (1.55 mmol, 1 eq) of methyl (E)-3-[3'-(1-propyl-3-heptylureido)biphenyl-4-yl]acrylate in 15 ml of a tetrahydrofuran/methanol mixture (8/2), and after crystallization from dichloromethane/heptane, 560 mg of (E)-3-[3'-(1-propyl-3-heptylureido)biphenyl-4-yl]acrylic acid are obtained in the form of a white powder (m.p. = 126°C). Yield = 85%

¹H NMR (CDCl₃, 400 MHz) : 0.87 (t, 3H); 0.92 (t, 3H); 1.24 (m, 8H); 1.40-1.45 (m, 2H); 1.53-1.63 (m, 2H); 3.19 (m, 2H) ; 3.71 (m, 2H); 4.22 (m, 1 H); 6.52-6.56 (d, 1 H); 7.26 (m, 2H); 7.50-7.69 (m, 6H); 7.83-7.87 (d, 1 H).

### Example 29 : (E)-3-[3.5-Difluoro-3'-(3-heptyl-1-methylureido)biphenyl-4-yl]acrylic acid

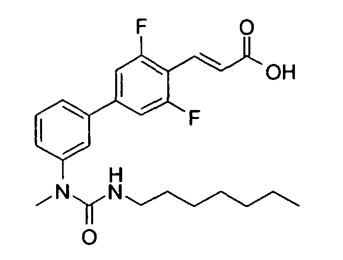

### a. 4-Bromo-2,6-difluorobenzaldehyde

0.7 g (7.7 mmol 10 eq) of manganese dioxide is added to a solution of 1.0 g (0.77 mmol, 1 eq) of 2,6-difluoro-4-bromobenzylalcool in 15 mL of dichloromethane. The reaction medium is stirred at room temperature for 48 hours.
The solid is filtered off and the solvent is evaporated off. The residual oil is chromatographed on silica gel (8/2 heptane/ethyl acetate) and 760 mg of 4-bromo-2,6-difluorobenzaldehyde are obtained. Yield = 76%

### b. Methyl 3-(4-bromo-2,6-difluorophenyl)acrylate

In a manner similar to that of Example (3a), by reaction of 1.7 g (5.15 mmol, 1.5 eq) of methyl triphenylphosphoranylideneacetate and 760 mg (3.44 mmol, 1 eq) of 4-bromo-2,6-difluorobenzaldehyde in 15 ml of toluene, 550 mg of methyl 3-(4-bromo-2,6-difluorophenyl)acrylate are obtained in solid form. Yield = 57%

### c. Methyl (E)-3-[3,5-difluoro-3'-(3-heptyl-1-methylureido)biphenyl4-yl]acrylate

In a manner similar to that of Example (1f), by reaction of 550 mg (1.98 mmol, 1 eq) of methyl 3-(4-bromo-2,6-difluorophenyl)acrylate, 965 mg (2.58 mmol, 1.3 eq) 3-heptyl-1-methyl-1-[3-(4,4,5,5-tetramethyl[1,3,2]dioxaborolan-2-yl)phenyl]urea (prepared in Example 1e) and 100 mg (5 mol%) of tetrakis(triphenylphosphine)palladium in 10 mL of a 6/1 mixture of dimethylformamide and of 2M potassium phosphate solution, 630 mg of methyl (E)-3-[3,5-difluoro-3'-(3-heptyl-1-methylureido)biphenyl-4-yl]acrylate are obtained in oil form. Yield = 72%

### d. (E)-3-[3.5-Difluoro-3'-(3-heptyl-1-methylureido)biphenyl-4-yl]acrylic acid

In a manner similar to that of Example (19g), by reaction of 400 mg (10 mmol, 7.0 eq) of sodium hydroxide and 630 mg (1.41 mmol, 1 eq) of methyl (E)-3-[3,5-difluoro-3'-(3-heptyl-1-methylureido)biphenyl-4-yl]acrylate in 15 ml of a tetrahydrofuran/methanol mixture (8/2), and after crystallization from dichloromethane/heptane, 450 mg of (E)-3-[3,5-difluoro-3'-(3-heptyl-1-methylureido)biphenyl-4-yl]acrylic acid are obtained in the form of a white powder (m.p. = 136°C). Yield = 74%
¹H NMR (CDCl₃, 400 MHz) : 0.86 (m, 3H); 1.25 (m, 8H); 1.44 (m, 2H); 3.21 (m, 2H) ; 3.34 (s, 3H); 4.37 (m, 1 H); 6.80-6.86 (d, 1H); 7.20-7.23 (d, 2H); 7.34 (d, 1H); 7.50-7.57 (m, 3H); 7.88-7.94 (d, 1 H).

### Example 30 : 3-[3'-(1-Ethyl-3-heptylureido)biphenyl-4-yl]propanoic acid

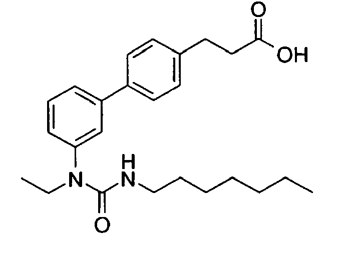

A solution of 400 mg (0.98 mmol, 1 eq) of (E)-3-[3'-(1-ethyl-3-heptylureido)biphenyl-4-yl]acrylic acid (prepared in Example 27g) in 10 mL of ethyl acetate in the presence of 0.2 ml of methanol is stirred for 4 hours at room temperature in the presence of 200 mg (50% by mass) of 10% palladium-on-charcoal under a hydrogen atmosphere. The reaction medium is filtered through Celite and then evaporated to dryness. After crystallization from ethyl ether/pentane, 265 mg of 3-[3'-(1-ethyl-3-heptylureido)biphenyl-4-yl]propanoic acid are obtained in the form of a white powder (m.p. = 70°C). Yield = 66%

¹H NMR (CDCl₃, 400 MHz) : 0.86 (t, 3H); 1.15 (t, 3H); 1.23 (m, 8H); 1.42 (m, 2H); 2.75 (t, 2H); 3.05 (t, 2H); 3.17 (m, 2H) ; 3.79 (q, 2H); 4.22 (t, 1H); 7.19 (d, 1H); 7.34 (d, 2H); 7.45-7.57 (m, 5H).

### Example 31 : 3-[3'-(3-Heptyl-1-propylureido)biphenyl-4-yl]propanoic acid

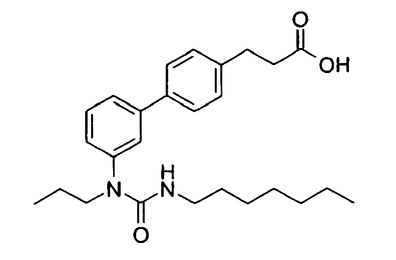

In a manner similar to that of Example (18e), by reaction of 400 mg (0.95 mmol, 1 eq) of (E)-3-[3'-(1-propyl-3-heptylureido)biphenyl-4-yl]acrylic acid (prepared in Example 28f) and 200 mg (50% by mass) of 10% palladium-on-charcoal in 10 mL of ethyl acetate in the presence of 0.2 ml of methanol, 250 mg of 3-[3'-(1-propyl-3-heptylureido)biphenyl-4-yl]propanoic acid are obtained in the form of a white powder (m.p. = 79°C). Yield = 63%
¹H NMR (CDCl₃, 400 MHz) : 0.86 (t, 3H); 0.90 (t, 3H); 1.23 (m, 8H); 1.41 (m, 2H); 1.51-1.61 (m, 2H); 2.75 (t, 2H); 3.05 (t, 2H); 3.17 (m, 2H); 3.99 (m, 2H); 4.23 (m, 1H); 7.19 (d, 1H); 7.34 (d, 2H); 7.44-7.55 (m, 5H).

### Example 32: 3-[3,5-Difluoro-3'-(3-heptyl-1-methylureido)biphenyl-4-yl]propanoic acid

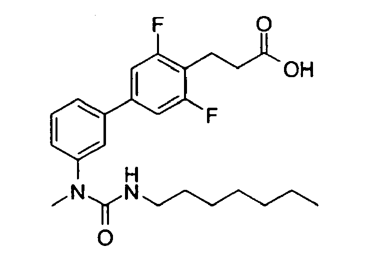

In a manner similar to that of Example (18e), by reaction of 300 mg (0.69 mmol, 1 eq) of (E)-3-[3,5-difluoro-3'-(3-heptyl-1-methylureido)biphenyl-4-yl]acrylic acid (prepared in Example 29d) and 200 mg (67% by mass) of 10% palladium-on-charcoal in 10 mL of ethyl acetate in the presence of 0.2 ml of methanol, 130 mg of 3-[3,5-difluoro-3'-(3-heptyl-1-methylureido)biphenyl-4-yl]propanoic acid are obtained in the form of a white powder (m.p. = 86°C). Yield = 44%
¹H NMR (CDCl₃, 400 MHz) : 0.86 (t, 3H); 1.24 (m, 8H); 1.43 (m, 2H); 2.72 (t, 2H); 3.08 (t, 2H); 3.19 (q, 2H) ; 3.33 (s, 3H); 4.38 (t, 1 H); 7.11 (d, 2H); 7.30 (s, 1 H); 7.45 (s, 1 H); 7.47-7.54 (m, 2H).

### Example 33: 3-{3'-[3-(4-Butoxyphenyl)-1-methylureido]biphenyl-4-yl}propanoic acid

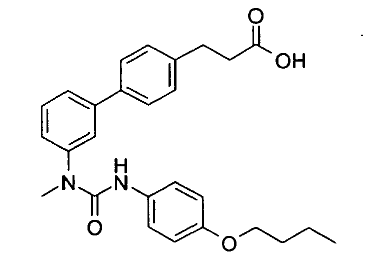

### a. Ethyl 3-{3'-[3-(4-butoxyphenyl)-1-methylureido]biphenyl-4-yl}propanoate

In a manner similar to that of Example (5a), by reaction of 574 µl (3.17 mmol, 3 eq) of 4-butoxyphenyl isocyanate and 300 mg (1.06 mmol, 1 eq) of ethyl 3-(3'-methylaminobiphenyl-4-yl)propanoate at 100°C by microwave for 15 minutes, 490 mg of ethyl 3-{3'-[3-(4-butoxyphenyl)-1-methylureido]biphenyl-4-yl}propanoate are obtained in the form of a white solid. Yield = 97%

### b. 3-{3'-[3-(4-Butoxyphenyl)-1-methylureido]biphenyl-4-y/}propanoic acid

In a manner similar to that of Example (19g), by reaction of 200 mg (5.0 mmol, 4.8 eq) of sodium hydroxide and 490 mg (1.03 mmol, 1 eq) of ethyl 3-{3'-[3-(4-butoxyphenyl)-1-methylureido]biphenyl-4-yl}propanoate in 6 ml of a tetrahydrofuran/methanol mixture (8/2), and after recrystallization from ethyl acetate/heptane, 330 mg of 3-{3'-[3-(4-butoxyphenyl)-1-methylureido]biphenyl-4-yl}propanoic acid are obtained in the form of white crystals. (m.p. = 165°C). Yield = 71 %

¹H NMR (CDCl₃, 400 MHz) : 0.95 (t, 3H); 1.43-1.48 (m, 2H); 1.68-1.75 (m, 2H); 2.73 (t, 2H); 3.01 (t, 2H); 3.37 (s, 3H); 3.89 (t, 2H); 6.18 (s, 1 H); 6.77 (d, 2H); 7.16 (d, 2H); 7.29-7.32 (m, 3H); 7.51-7.56 (m, 5H).

### Example 34 : 3-{3'-[3-(4-Butoxyphenyl)-1-ethylureido]biphenyl-4-yl}propanoic acid

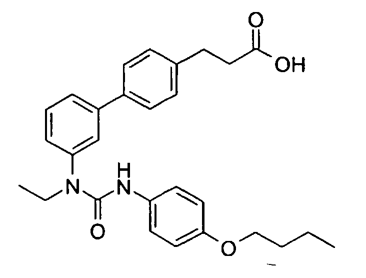

### a. Ethyl (E)-3-[4-(4,4,5,5-tetramethyl[1,3,2]dioxaborolan-2-yl)phenyl]acrylate

In a manner similar to that of Example (1e), by reaction of 7.0 g (27 mmol, 1 eq) of of ethyl 4-bromocinnamate, 8.0 g (81 mmol, 3 eq) of potassium acetate and 8.3 g (33 mmol, 1.2 eq) of pinacol diborane in the presence of 980 mg (5 mol%) of diphenylphosphinoferrocenepalladium dichloride in 50 ml of dimethylformamide, 8.0 g of ethyl (E)-3-[4-(4,4,5,5-tetramethyl[1,3,2]dioxaborolan-2-yl)phenyl]acrylate are obtained in oil form. Yield = 98%

### b. Ethyl 3-[4-(4,4,5,5-tetramethyl[1,3,2]dioxaborolan-2-yl)phenyl]propanoate

In a manner similar to that of Example (18e), by reaction of 400 mg (5% by mass) of 10% palladium-on-charcoal and 8.0 g (1.39 mmol, 1 eq) of ethyl (E)-3-[4-(4,4,5,5-tetramethyl[1,3,2]dioxaborolan-2-yl)phenyl]acrylate in 30 ml of methanol, 7.8 g of ethyl 3-[4-(4,4,5,5-tetramethyl[1,3,2]dioxaborolan-2-yl)phenyl]propanoate are obtained in the form of a colourless oil. Yield = 96%

### c. Ethyl 3-(3'-ethylaminobiphenyl-4-yl)propanoate

In a manner similar to that of Example (1f), by reaction of 1.5 g (4.93 mmol, 1 eq) of ethyl 3-[4-(4,4,5,5-tetramethyl[1,3,2]dioxaborolan-2-yl)phenyl]propanoate, 1.28 g (6.41 mmol, 1.3 eq) of (3-bromophenyl)ethylamine (prepared in Example 27c) and 280 mg (5 mol%) of tetrakis(triphenylphosphine)-palladium in 15 mL of a 6/1 mixture of dimethylformamide and of 2M potassium phosphate solution, and after crystallization from pentane, 700 mg of ethyl (3-(3'-ethylaminobiphenyl-4-yl)propanoate are obtained in the form of a white solid. Yield = 48%

### d. Ethyl 3-{3'-[3-(4-butoxyphenyl)-1-ethylureido]biphenyl-4-yl}propanoate

In a manner similar to that of Example (11a), by reaction of 543 µl (3.0 mmol, 3 eq) of 4-butoxyphenyl isocyanate and 300 mg (1.0 mmol, 1 eq) of ethyl 3-(3'-methylaminobiphenyl-4-yl)propanoate, 460 mg of ethyl 3-{3'-[3-(4-butoxyphenyl)-1-ethylureido]biphenyl-4-yl}propanoate are obtained in the form of a colourless oil. Yield = 94%

### e. 3-{3'-[3-(4-Butoxyphenyl)-1-ethylureido]biphenyl-4-yl}propanoic acid

In a manner similar to that of Example (19g), by reaction of 200 mg (5.0 mmol, 4.8 eq) of sodium hydroxide and 460 mg (0.94 mmol, 1 eq) of ethyl 3-{3'-[3-(4-butoxyphenyl)-1-ethylureido]biphenyl-4-yl}propanoate in 6 ml of a tetrahydrofuran/methanol mixture (8/2), and after crystallization from pentane, 118 mg of 3-{3'-[3-(4-butoxyphenyl)-1-ethylureido]biphenyl-4-yl}propanoic acid are obtained in the form of a white powder (m.p. = 102-103°C). Yield = 27%

¹H NMR (CDCl₃, 400 MHz) : 0.94 (t, 3H); 1.19 (t, 3H); 1.40-1.50 (m, 2H); 1.68-1.75 (m, 2H); 2.72 (t, 2H); 3.02 (t, 2H); 3.82 (m, 2H); 3.89 (t, 2H); 6.02 (s, 1 H); 6.77 (d, 2H); 7.16 (d, 2H); 7.27-7.32 (m, 3H); 7.51-7.58 (m, 5H).

### Example 35: 3-[2-Cyclopropylmethoxy-3'-(3-heptyl-1-methylureido)biphenyl-4-yl]propanoic acid

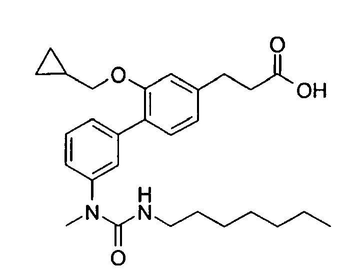

### a. Methyl 3-[2-cyclopropylmethyl-3'-(3-heptyl-1-methylureido)biphenyl-4-yl]propanoate

In a manner similar to that of Example (25a), by reaction of 170 µL (1.75 mmol, 1.5 eq) of methylcyclopropyl bromide and 500 mg (1.17 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 3 eq) of potassium carbonate, methyl 3-[2-cyclopropylmethoxy-3'-(3-heptyl-1-methylureido)biphenyl-4-yl]propanoate is obtained in oil form and is used in the following reaction without further purification.

### b. 3-[2-Cyclopropylmethoxy-3'-(3-heatyl-1-methylureido)biphenyl-4-yl]propanoic acid

In a manner similar to that of Example (19g), by reaction of 400 mg (10.0 mmol, 4.8 eq) of sodium hydroxide and methyl 3-[2-cyclopropylmethoxy-3'-(3-heptyl-1-methylureido)biphenyl-4-yl]propanoate, obtained in the preceding step, in 10 ml of a tetrahydrofuran/methanol mixture (8/2), and after crystallization from pentane, 426 mg of 3-[2-cyclopropylmethoxy-3'-(3-heptyl-1-methylureido)biphenyl-4-yl]propanoic acid are obtained in the form of a white powder (m.p. = 64°C). Yield = 78% over the two steps

¹H NMR (DMSO-d6, 400 MHz) : 0.29 (m, 2H); 0.51 (m, 2H); 0.85 (t, 3H); 1.17-1.21 (m, 8H); 1.37 (m, 2H); 2.57 (t, 2H); 2.84 (t, 2H); 2.99 (m, 2H) ; 3.18 (s, 3H); 3.86 (d, 2H); 5.92 (t, 1 H); 6.88 (d, 1 H); 6.97 (s, 1 H); 7.17 (d, 1 H); 7.24 (d, 1H); 7.38-7.43 (m, 3H).

### Example 36 : 3-[2-Ethoxy-3'-(3-heptyl-1-methylureido)biphenyl-4-yl]propanoic acid

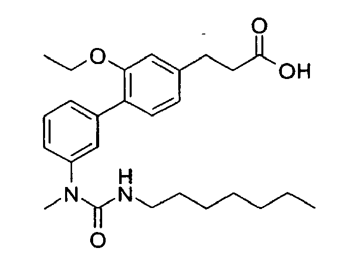

### a. Methyl 3-[2-ethoxy-3'-(3-heptyl-1-methylureido)biphenyl-4-yl]propanoate

In a manner similar to that of Example (25a), by reaction of 200 µL (2.46 mmol, 3 eq) of 1-iodoethane and 350 mg (0.82 mmol, 1 eq) of methyl 3-[3'-(3-heptyl-1-methylureido)-2-hydroxybiphenyl-4-yl]propanoate (prepared in Example 15f) in 6 ml of methyl ethyl ketone in the presence of 500 mg (3.61 mmol, 4.4 eq) of potassium carbonate at 80°C for 5 hours, methyl 3-[2-ethoxy-3'-(3-heptyl-1-methylureido)biphenyl-4-yl]propanoate is obtained in oil form and is used in the following reaction without further purification.

### b. 3-[2-Ethoxy-3'-(3-heptyl-1-methylureido)biphenyl-4-yl]propanoic acid

In a manner similar to that of Example (19g), by reaction of 400 mg (10.0 mmol, 4.8 eq) of sodium hydroxide and methyl 3-[2-ethoxy-3'-(3-heptyl-1-methylureido)biphenyl-4-yl]propanoate, obtained in the preceding step, in 10 ml of a tetrahydrofuran/methanol mixture (8/2), and after crystallization from pentane/isopropyl ether, 246 mg of 3-[2-ethoxy-3'-(3-heptyl-1-methylureido)biphenyl-4-yl]propanoic acid are obtained in the form of a white powder (m.p. = 64°C). Yield = 78% over the two steps

¹H NMR (CDCl₃, 400 MHz) : 0.84 (t, 3H); 1.22-1.27 (m, 8H); 1.35 (t, 3H); 1.40 (m, 2H); 2.73 (t, 2H); 3.00 (t, 2H); 3.16 (q, 2H) ; 3.29 (s, 3H); 4.05 (q, 2H); 4.44 (t, 1H); 6.84 (s, 1 H); 6.88 (d, 1 H); 7.17 (d, 1H); 7.24 (d, 1 H); 7.40-7.47 (m, 3H).

### Example 37: 3-[3'-(3-Heptyl-1-methylureidol-2-(3,3,3-trifluoropropoxy)biphenyl-4-yl]propanoic acid

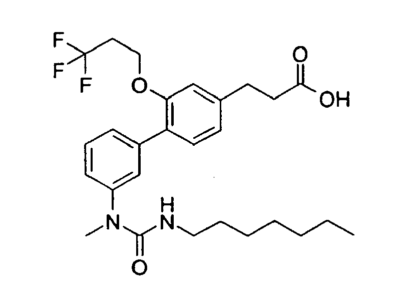

### a. Methyl 3-[3'-(3-heptyl-1-methylureido)-2-(3,3,3-trifluoropropoxy)biphenyl-4-yl]propanoate

In a manner similar to that of Example (25a), by reaction of 1.0 mL (8.52 mmol, 7.3 eq) 1-iodo-3,3,3-trifluoropropane and 500 mg (1.17 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 3 eq) of potassium carbonate at 80°C for 7 days, 50 mg of methyl 3-[3'-(3-heptyl-1-methylureido)-2-(3,3,3-trifluoropropoxy)biphenyl-4-yl]propanoate are obtained in oil form. Yield = 8%

### b. 3-[3'-(3-Heptyl-1-methylureido)-2-(3,3,3-trifluoropropoxy)biphenyl-4-yl]propanoic acid

In a manner similar to that of Example (19g), by reaction of 50 mg (1.25 mmol, 13 eq) of sodium hydroxide and 50 mg (0.096 mmol, 1 eq) of methyl 3-[3'-(3-heptyl-1-methylureido)-2-(3,3,3-trifluoropropoxy)biphenyl-4-yl]propanoate in 3 ml of a tetrahydrofuran/methanol mixture (8/2) at room temperature for 2 hours, and after crystallization from pentane/isopropyl ether, 14 mg of 3-[3'-(3-heptyl-1-methylureido)-2-(3,3,3-trifluoropropoxy)biphenyl-4-yl]propanoic acid are obtained in the form of a white powder (m.p. = 90°C). Yield = 29%

¹H NMR (CDCl₃, 400 MHz) : 0.84 (t, 3H); 1.21-1.32 (m, 8H); 1.40 (m, 2H); 2.53 (m, 2H); 2.68 (t, 2H); 2.99 (t, 2H); 3.15 (q, 2H); 3.28 (s, 3H); 4.19 (t, 2H); 4.36 (t, 1H); 6.82 (s, 1 H); 6.92 (d, 1 H); 7.17 (m, 1 H); 7.25 (m, 1 H); 7.26-7.42 (m, 3H).

### Example 38: 3-[3'-(3-Heptyl-1-methylureido)-2-(4,4,4-trifluorobutoxy)biphenyl-4-yl]propanoic acid

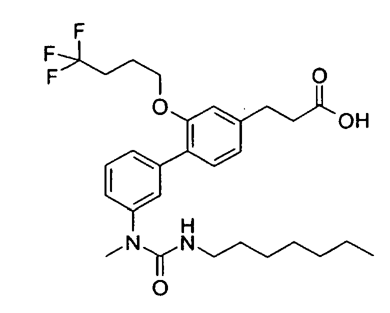

### a. Methyl 3-[3'-(3-heptyl-1-methylureido)-2-(4,4,4-trifluorobutoxy)biphenyl-4-yl]propanoate

In a manner similar to that of Example (25a), by reaction of 500 µL (3.78 mmol, 5.8 eq) of 1-iodo-4,4,4-trifluorobutane and 280 mg (0.65 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 5.5 eq) of potassium carbonate at 80°C for 12 hours, methyl 3-(3'-(3-heptyl-1-methylureido)-2-(4,4,4-trifluorobutoxy)biphenyl-4-yl]propanoate is obtained in oil form and is used in the following reaction without further purification.

### b. 3-[3'-(3-Heptyl-1-methylureido)-2-(4,4,4-trifluorobutoxy)biphenyl-4-yl]propanoic acid

In a manner similar to that of Example (19g), by reaction of 400 mg (10.0 mmol, 4.8 eq) of sodium hydroxide and methyl 3-[3'-(3-heptyl-1-methylureido)-2-(4,4,4-trifluorobutoxy)biphenyl-4-yl]propanoate, obtained in the preceding step, in 10 ml of a tetrahydrofuran/methanol mixture (8/2), and after crystallization from pentane, 252 mg of 3-[3'-(3-heptyl-1-methylureido)-2-(4,4,4-trifluorobutoxy)biphenyl-4-yl]propanoic acid are obtained in the form of a white powder (m.p. = 78°C). Yield = 74% over the two steps

¹H NMR (CDCl₃, 400 MHz) : 0.84 (t, 3H); 1.21-1.27 (m, 8H); 1.39 (m, 2H); 1.95-2.0 (m, 2H); 2.12-2.17 (m, 2H); 2.73 (t, 2H); 3.00 (t, 2H); 3.15 (q, 2H) ; 3.29 (s, 3H); 4.02 (t, 2H); 4.39 (t, 1 H); 6.83 (s, 1 H); 6.93 (d, 1 H); 7.18 (m, 1 H); 7.24 (d, 1 H); 7.38 (s, 1 H); 7.43 (d, 2H).

### Example 39: 3-[3'-(3-Heptyl-1-methylureido)-2-(3-hydroxypropoxy)biphenyl-4-yl]propanoic acid

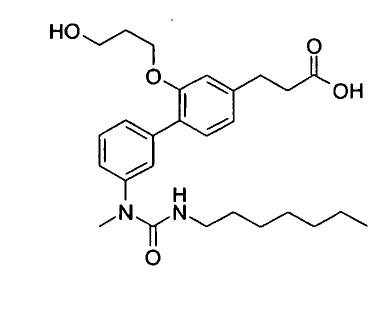

### a. Methyl 3-(3'-(3-heptyl-1-methylureido)-2-(3-hydroxypropoxy)biphenyl-4-yl)propanoate

In a manner similar to that of Example (25a), by reaction of 500 µL (5.2 mmol, 7.4 eq) of 3-iodo-1-propanol and 300 mg (0.7 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 5.1 eq) of potassium carbonate at 80°C for 15 hours, 270 mg of methyl 3-[3'-(3-heptyl-1-methylureido)-2-(3-hydroxypropoxy)biphenyl-4-yl]propanoate are obtained in oil form. Yield = 79%

### b. 3-[3'-(3-Heptyl-1-methylureido)-2-(3-hydroxypropoxy)biphenyl-4-yl]propanoic acid

In a manner similar to that of Example (19g), by reaction of 400 mg (10.0 mmol, 18 eq) of sodium hydroxide and 270 mg (0.55 mmol, 1 eq) of methyl 3-[3'-(3-heptyl-1-methylureido)-2-(3-hydroxypropoxy)biphenyl-4-yl]propanoate in 3 ml of a tetrahydrofuran/methanol mixture (8/2), and after crystallization from ethyl acetate/heptane, 135 mg of 3-[3'-(3-heptyl-1-methylureido)-2-(3-hydroxypropoxy)biphenyl-4-yl]propanoic acid are obtained in the form of a white powder (m.p. = 107°C). Yield = 51%

¹H NMR (CDCl₃, 400 MHz) : 0.84 (t, 3H); 1.22-1.27 (m, 8H); 1.40 (m, 2H); 1.92-1.98 (m, 2H); 2.73 (t, 2H); 3.00 (t, 2H); 3.15 (q, 2H); 3.26 (s, 3H); 3.67 (t, 2H); 4.15 (t, 2H); 4.76 (t, 1H); 6.87 (s, 1 H); 6.92 (d, 1 H); 7.18-7.22 (m, 2H); 7.35 (d, 2H); 7.43 (t, 1H).

### Example 40: 3-[3'-(3-Heptyl-1-methylureido)-2-(4-hydroxybutoxy)biphenyl-4-yl]propanoic acid

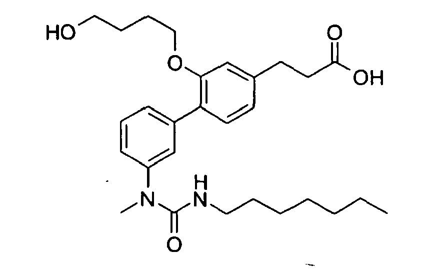

### a. Methyl 3-[3'-(3-Heptyl-1-methylureido)-2-(4-hydroxybutoxy)biphenyl-4-yl]propanoate

In a manner similar to that of Example (25a), by reaction of 500 µL (3.45 mmol, 4.9 eq) of 4-bromobutyl acetate and 300 mg (0.7 mmol, 1 eq) of methyl 3-[3'-(3-heptyl-1-methylureido)-2-hydroxybiphenyl-4-yl]propanoate (prepared in Example 15f) in 6 ml of methyl ethyl ketone in the presence of 500 mg (3.61 mmol, 5.1 eq) of potassium carbonate at 80°C for 15 hours, 305 mg of methyl 3-[3'-(3-heptyl-1-methylureido)-2-(4-hydroxybutoxy)biphenyl-4-yl]propanoate are obtained in oil form. Yield = 80%

### b. 3-[3'-(3-Heptyl-1-methylureido)-2-(4-hydroxybutoxy)biphenyl-4-yl]propanoic acid

In a manner similar to that of Example (19g), by reaction of 300 mg (7.5 mmol, 13 eq) of sodium hydroxide and 305 mg (0.56 mmol, 1 eq) of methyl 3-[3'-(3-heptyl-1-methylureido)-2-(4-hydroxybutoxy)biphenyl-4-yl]propanoate in 3 ml of a tetrahydrofuran/methanol mixture (8/2), 156 mg of 3-[3'-(3-heptyl-1-methylureido)-2-(4-hydroxybutoxy)biphenyl-4-yl]propanoic acid are obtained in the form of a whitish oil. Yield = 57%

¹H NMR (CDCl₃, 400 MHz) : 0.84 (t, 3H); 1.21-1.27 (m, 8H); 1.38-1.41 (m, 2H); 1.63-1.68 (m, 2H); 1.78-1.83 (m, 2H); 2.71 (t, 2H); 2.99 (t, 2H); 3.14 (q, 2H) ; 3.28 (s, 3H); 3.57 (t, 2H); 4.03 (t, 2H); 4.63 (t, 1 H); 6.86 (s, 1 H); 6.88 (d, 1 H); 7.17 (m, 1H); 7.24 (d, 1 H); 7.42-7.46 (m, 3H).

### Example 41 : 3-[2-Butoxy-3'-(1-methyl-3-pentylureido)biphenyl-4-yl]propanoic acid

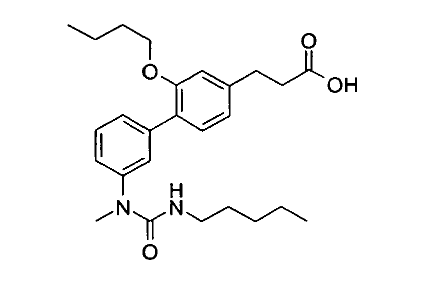

### a. Methyl (E)-3-(2-benzyloxy-3'-methylaminobiphenyl-4-yl)acrylate

In a manner similar to that of Example (15e), by reaction of 130 mg (5 mol%) of palladium acetate, 406 mg (10 mol%) of dicyclohexylbiphenyl phosphine, 4.6 g (11.7 mmol, 1.0 eq) of methyl (E)-3-(3-benzyloxy-4-iodophenyl)acrylate (prepared in Example 15d) and 3.26 g (14.0 mmol, 1.2 eq) of methyl[3-(4,4,5,5-tetramethyl[1 ,3,2]dioxaborolan-2-yl)phenyl]amine (prepared in Example 7a) in 20 mL of a mixture of dimethylformamide/2M potassium phosphate solution (6/1), 4.3 g of methyl (E)-3-(2-benzyloxy-3'-methylaminobiphenyl-4-yl)acrylate are obtained in oil form. Yield = 98%

### b. Methyl (E)-3-[2-benzyloxy-3'-(1-methyl-3-pentylureido)biphenyl-4-yl]acrylate

In a manner similar to that of Example (1d), by reaction of 600 µL (4.6 mmol, 3.5 eq) of pentyl isocyanate and 500 mg (1.33 mmol, 1 eq) of methyl (E)-3-(2-benzyloxy-3'-methylaminobiphenyl-4-yl)acrylate, 544 mg of methyl (E)-3-[2-benzyloxy-3'-(1-methyl-3-pentylureido)biphenyl-4-yl]acrylate are obtained in oil form. Yield = 84%

### c. Methyl 3-[2-hydroxy-3'-(1-methyl-3-pentylureido)biphenyl-4-yl]propanoate

In a manner similar to that of Example (18e), by reaction of 100 mg (18% by mass) of 10% palladium-on-charcoal and 544 mg (1.12 mmol, 1 eq) of methyl (E)-3-[2-benzyloxy-3'-(1-methyl-3-pentylureido)biphenyl-4-yl]acrylate in 10 ml of methanol, 530 mg of methyl 3-[2-hydroxy-3'-(1-methyl-3-pentylureido)biphenyl-4-yl]propanoate are obtained in oil form. Yield = 97%

### d. Methyl 3-[2-butoxy-3'-(1-methyl-3-pentylureido)biphenyl-4-yl]propanoate

In a manner similar to that of Example (25a), by reaction of 500 µL (4.4 mmol, 3.3 eq) of 1-iodobutane and 530 mg (1.33 mmol, 1 eq) of methyl 3-[2-hydroxy-3'-(1-methyl-3-pentylureido)biphenyl-4-yl]propanoate in 10 ml of methyl ethyl ketone in the presence of 500 mg (3.61 mmol, 2.7 eq) of potassium carbonate at 80°C for 16 hours, methyl 3-[2-butoxy-3'-(1-methyl-3-pentylureido)biphenyl-4-yl]propanoate is obtained in oil form and is used in the following reaction without further purification.

### e. 3-[2-Butoxy-3'-(1-methyl-3-pentylureido)biphenyl-4-yl]propanoic acid

In a manner similar to that of Example (25a), by reaction of 400 mg (10 mmol, 7.5 eq) of sodium hydroxide and methyl 3-[2-butoxy-3'-(1-methyl-3-pentylureido)biphenyl-4-yl]propanoate, obtained in the preceding step, in 6 ml of a tetrahydrofuran/methanol mixture (8/2), and after crystallization from pentane, 220 mg of 3-[2-butoxy-3'-(1-methyl-3-pentylureido)biphenyl-4-yl]propanoic acid are obtained in the form of a white powder (m.p. = 78°C). Yield = 46% over the two steps.

¹H NMR (CDCl₃, 400 MHz) : 0.84 (t, 3H); 0.91 (t, 3H); 1.18-1.27 (m, 4H); 1.36-1.44 (m, 4H); 1.68-1.72 (m, 2H); 2.73 (t, 2H); 3.00 (t, 2H); 3.15 (q, 2H) ; 3.29 (s, 3H); 3.97 (t, 2H); 4.41 (t, 1 H); 6.84 (s, 1 H); 6.87 (d, 1 H); 7.17 (d, 1 H); 7.25 (d, 1 H); 7.42-7.46 (m, 3H).

### Example 42: 3-[2-Benzyloxy-3'-(3-heptyl-1-methylureido)biphenyl-4-yl]propanoic acid

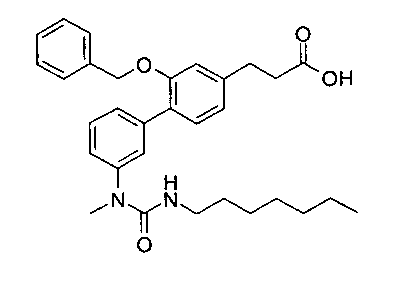

### a. Methyl 3-[2-benzyloxy-3'-(3-heptyl-1-methylureido)biphenyl-4-yl]propanoate

In a manner similar to that of Example (25a), by reaction of 300 µL (2.5 mmol, 2.7 eq) of benzyl bromide and 400 mg (0.93 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 3.9 eq) of potassium carbonate at 90°C for 3 hours, methyl 3-[2-benzyloxy-3'-(3-heptyl-1-methylureido)biphenyl-4-yl]propanoate is obtained in oil form and is used in the following reaction without further purification.

### b. 3-[2-Benzyloxy-3'-(3-heptyl-1-methylureido)biphenyl-4-yl]propanoic acid

In a manner similar to that of Example (19g), by reaction of 400 mg (10 mmol, 10 eq) of sodium hydroxide and methyl 3-[2-benzyloxy-3'-(3-heptyl-1-methylureido)biphenyl-4-yl]propanoate, obtained in the preceding step, in 10 ml of a tetrahydrofuran/methanol mixture (8/2), and after crystallization from pentane, 305 mg of 3-[2-benzyloxy-3'-(3-heptyl-1-methylureido)biphenyl-4-yl]propanoic acid are obtained in the form of a white powder (m.p. = 76°C). Yield = 65% over the two steps.

¹H NMR (CDCl₃, 400 MHz) : 0.83 (t, 3H); 1.19-1.26 (m, 8H); 1.30-1.35 (m, 2H); 2.72 (t, 2H); 3.00 (t, 2H); 3.05-3.10 (q, 2H) ; 3.20 (s, 3H); 4.39 (t, 1 H); 5.07 (s, 2H); 6.92 (d, 2H); 7.17 (d, 1 H); 7.28-7.33 (m, 6H); 7.42-7.47 (m, 3H).

### Example 43: 3-[2-(3-Fluorobenzyloxy)-3'-(3-heptyl-1-methylureido)biphenyl-4-yl]-propanoic acid

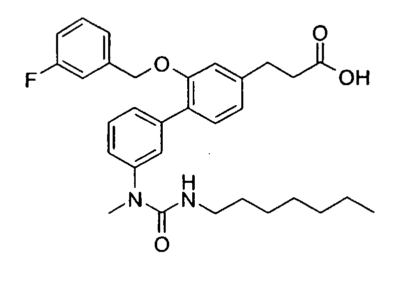

### a. Methyl 3-[2-(3-fluorobenzyloxy)-3'-(3-heptyl-1-methylureido)biphenyl-4-yl]propanoate

In a manner similar to that of Example (25a), by reaction of 300 µL (2.4 mmol, 2.6 eq) of 3-fluorobenzyl bromide and 400 mg (0.93 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 3.9 eq) of potassium carbonate at 90°C for 3 hours, methyl 3-[2-(3-fluorobenzyloxy)-3'-(3-heptyl-1-methylureido)biphenyl-4-yl]propanoate is obtained in oil form and is used in the following reaction without further purification.

### b. 3-[2-(3-Fluorobenzyloxy)-3'-(3-heptyl-1-methylureido)biphenyl-4-yl]propanoic acid

In a manner similar to that of Example 19g, by reaction of 400 mg (10 mmol, 10 eq) of sodium hydroxide and methyl 3-[2-(3-fluorobenzyloxy)-3'-(3-heptyl-1-methylureido)biphenyl-4-yl]propanoate, obtained in the preceding step, in 10 ml of a tetrahydrofuran/methanol mixture (8/2), and after crystallization from pentane, 305 mg of 3-[2-(3-fluorobenzyloxy)-3'-(3-heptyl-1-methylureido)biphenyl-4-yl]propanoic acid are obtained in the form of a white powder (m.p. = 73°C). Yield = 68% over the two steps.

¹H NMR (CDCl₃, 400 MHz) : 0.83 (t, 3H); 1.19-1.26 (m, 8H); 1.31-1.36 (m, 2H); 2.72 (t, 2H); 3.00 (t, 2H); 3.07-3.12 (q, 2H) ; 3.23 (s, 3H); 4.40 (t, 1 H); 5.06 (s, 2H); 6.90 (s, 1 H); 6.93-7.00 (m, 3H); 7.08 (d, 1 H); 7.18 (d, 1 H); 7.28 (m, 2H); 7.44-7.46 (m, 3H).

### Example 44: 3-[2-(4-Fluorobenzyloxy)-3'-(3-heptyl-1-methylureido)biphenyl-4-yl]propanoic acid

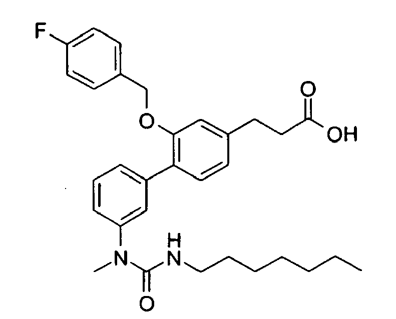

### a. Methyl 3-[2-(4-fluorobenzyloxy)-3'-(3-heptyl-1-methylureido)biphenyl-4-yl]propanoate

In a manner similar to that of Example (25a), by reaction of 300 µL (2.4 mmol, 2.6 eq) of 3-fluorobenzyl bromide and 400 mg (0.93 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 3.9 eq) of potassium carbonate at 90°C for 3 hours, methyl 3-[2-(4-fluorobenzyloxy)-3'-(3-heptyl-1-methylureido)biphenyl-4-yl]propanoate is obtained in oil form and is used in the following reaction without further purification.

### b. 3-[2-(4-Fluorobenzyloxy)-3'-(3-heptyl-1-methylureido)biphenyl-4-yl]propanoic acid

In a manner similar to that of Example (19g), by reaction of 400 mg (10 mmol, 10 eq) of sodium hydroxide and methyl 3-[2-(4-fluorobenzyloxy)-3'-(3-heptyl-1-methylureido)biphenyl-4-yl]propanoate, obtained in the preceding step, in 10 ml of a tetrahydrofuran/methanol mixture (8/2), and after crystallization from pentane, 310 mg of 3-[2-(4-fluorobenzyloxy)-3'-(3-heptyl-1-methylureido)biphenyl-4-yl]propanoic acid are obtained in the form of a white powder (m.p. = 79°C). Yield = 64% over the two steps.

¹H NMR (CDCl₃, 400 MHz) : 0.83 (t, 3H); 1.19-1.26 (m, 8H); 1.32-1.36 (m, 2H); 2.73 (t, 2H); 3.00 (t, 2H); 3.07-3.12 (q, 2H); 3.21 (s, 3H); 4.38 (t, 1H); 5.03 (s, 2H); 6.92 (d, 2H); 6.99-7.03 (m, 2H); 7.17 (d, 1 H); 7.27-7.29 (m, 3H); 7.42-7.45 (m, 3H).

### Example 45: 3-[3'-(3-Heptyl-1-methylureido)-2-pentyloxvbiphenyl-4-yl]propanoic acid

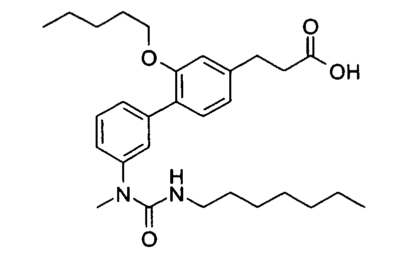

### a. Methyl 3-[3'-(3-heptyl-1-methylureido)-2-pentyloxybiphenyl-4yl]propanoate

In a manner similar to that of Example (25a), by reaction of 500 µL (3.8 mmol, 5.4 eq) of 1-iodopentane and 300 mg (0.7 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 5.1 eq) of potassium carbonate at 80°C for 12 hours, methyl 3-[3'-(3-heptyl-1-methylureido)-2-pentyloxybiphenyl-4-yl]propanoate is obtained in oil form and is used in the following reaction without further purification.

### b. 3-[3'-(3-Heptyl-1-methylureido)-2-pentyloxybiphenyl-4-yl]propanoic acid

In a manner similar to that of Example (19g), by reaction of 400 mg (10 mmol, 14 eq) of sodium hydroxide and methyl 3-[3'-(3-heptyl-1-methylureido)-2-pentyloxybiphenyl-4-yl]propanoate, obtained in the preceding step, in 10 ml of a tetrahydrofuran/methanol mixture (8/2), and after crystallization from pentane, 260 mg of 3-[3'-(3-heptyl-1-methylureido)-2-pentyloxybiphenyl-4-yl]propanoic acid are obtained in the form of a white powder (m.p. = 69°C). Yield = 77% over the two steps.

¹H NMR (CDCl₃, 400 MHz) : 0.84 (t, 3H); 0.88 (t, 3H); 1.21-1.25 (m, 8H); 1.32-1.40 (m, 6H); 1.72 (m, 2H); 2.73 (t, 2H); 3.00 (t, 2H); 3.15 (q, 2H); 3.29 (s, 3H); 3.96 (t, 2H); 4.42 (t, 1H); 6.84 (s, 1 H); 6.87 (d, 1 H); 7.17 (d, 1H); 7.24 (d, 1H); 7.40-7.46 (m, 3H).

### Example 46: 3-[3'-(1-Methyl-3-pentylureido)-2-pentyloxybiphenyl-4-yl]propanoic acid

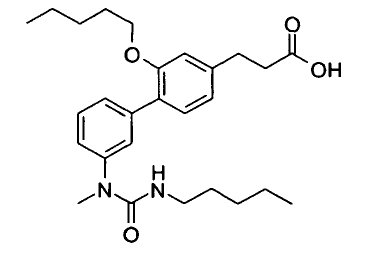

### a. 1-(3-Bromophenyl)-1-methyl-3-pentylurea

A solution of 50.0 g (0.442 mol, 1 eq) of pentyl isocyanate in 50 mL of dichloromethane is added to a mixture of 82.2 g (0.442 mol, 1 eq) of (3-bromophenyl)methylamine in 250 mL of dichloromethane in the presence of 20 mL (0.143 mol, 0.3 eq) of triethylamine. The reaction medium is refluxed for 3 days and then hydrolysed in 200 mL of 1 N hydrochloric acid solution and extracted with ethyl acetate. The organic phases are combined, washed with water and sodium chloride, and evaporated. 123 g of 1-(3-bromophenyl)-1-methyl-3-pentylurea are obtained.
Yield = 93%

### b. 3-(1-Methyl-3-pentylureido)phenylboronic acid

In a manner similar to that of Example (18a), by reaction of 123 g (0.411 mol, 1 eq) of 1-(3-bromophenyl)-1-methyl-3-pentylurea in 1.23 L of tetrahydrofuran, 150 mL (0.452 mol, 1.1 eq) of methyllithium, 530 mL (0.904 mol, 2.2 eq) of a 1.7 M solution of tert-butyllithium in pentane and 115 mL (0.904 mol, 2.2 eq) of trimethyl borate, and after purification by chromatography on silica gel (50/50 heptane/ethyl acetate) and crystallization from ethyl acetate/heptane, 42.0 g of 3-(1-methyl-3-pentylureido)phenylboronic acid are obtained in the form of a pink-coloured powder.
Yield = 39%

### c. Methyl (E)-3-[2-benzyloxy-3'-(1-methyl-3-pentylureido)phenyl-4-yl]acrylate

In a manner similar to that of Example (18d), by reaction of 17 mg (2 mol%) of palladium acetate, 53 mg (4 mol%) of dicyclohexylbiphenyl phosphine, 1.5 g (3.8 mmol, 1.0 eq) of methyl (E)-3-(3-benzyloxy-4-iodophenyl)acrylate (prepared in Example 24d) and 1.3 g (4.94 mmol, 1.3 eq) of 3-(1-methyl-3-pentylureido)phenylboronic acid in 20 mL of a mixture of dimethylformamide/2M potassium phosphate solution (6/1), 1.54 g of methyl (E)-3-[2-benzyloxy-3'-(1-methyl-3-pentylureido)biphenyl-4-yl]acrylate are obtained in oil form. Yield = 83%

### d. Methyl 3-[2-hydroxy-3'-(1-methyl-3-pentylureido)biphenyl-4-yl]propanoate

In a manner similar to that of Example (18e), by reaction of 400 mg (26% by mass) of 10% palladium-on-charcoal, 1.54 g (3.16 mmol, 1 eq) of methyl (E)-3-[2-benzyloxy-3'-(1-methyl-3-pentylureido)biphenyl-4-yl]acrylate in 20 ml of methanol and 5 mL of ethyl acetate, and after crystallization from pentane, 1.1 g of methyl 3-[2-hydroxy-3'-(1-methyl-3-pentylureido)biphenyl-4-yl]propanoate are obtained in solid form. Yield = 87%

### e. Methyl 3-[3'-(1-methyl-3-pentylureido)-2-pentyloxybiphenyl-4-yl]propanoate

In a manner similar to that of Example (25a), by reaction of 500 µL (3.8 mmol, 3.8 eq) of 1-iodopentane and 400 mg (1.0 mmol, 1 eq) of methyl 3-[2-hydroxy-3'-(1-methyl-3-pentylureido)biphenyl-4-yl]propanoate in 10 ml of methyl ethyl ketone in the presence of 500 mg (3.61 mmol, 3.6 eq) of potassium carbonate at 80°C for 12 hours, methyl 3-[3'-(1-methyl-3-pentylureido)-2-pentyloxybiphenyl-4-yl]propanoate is obtained in oil form and is used in the following reaction without further purification.

### f. 3-[3'-(1-Methyl-3-pentylureido)-2-pentyloxvbiphenyl-4-yl]propanoic acid

In a manner similar to that of Example (19g), by reaction of 400 mg (10 mmol, 10 eq) of sodium hydroxide and methyl 3-[3'-(1-methyl-3-pentylureido)-2-pentyloxybiphenyl-4-yl]propanoate, obtained in the preceding step, in 10 ml of a tetrahydrofuran/methanol mixture (8/2), and after crystallization from pentane, 312 mg of 3-[3'-(1-methyl-3-pentylureido)-2-pentyloxybiphenyl-4-yl]propanoic acid are obtained in the form of a white powder (m.p. = 83°C). Yield = 74% over the two steps.

¹H NMR (CDCl₃, 400 MHz) : 0.83 (t, 3H); 0.88 (t, 3H); 1.19-1.40 (m, 10H); 1.72 (m, 2H); 2.73 (t, 2H); 3.00 (t, 2H); 3.15 (q, 2H) ; 3.29 (s, 3H); 3.96 (t, 2H); 4.43 (t, 1 H); 6.84 (s, 1 H); 6.88 (d, 1 H); 7.16 (d, 1 H); 7.24 (d, 1 H); 7.42-7.47 (m, 3H).

### Example 47: 3-[2-(2-Ethoxyethoxy)-3'-(3-heptyl-1-methylureido)biphenyl-4-yl]propanoic acid

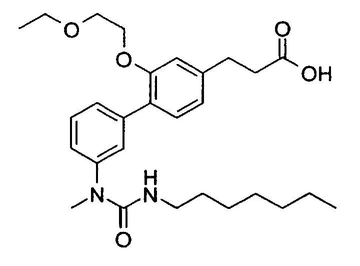

### a. Methyl 3-[2-(2-ethoxyethoxy)-3'-(3-heptyl-1-methylureido)biphenyl-4-yl]propanoate

In a manner similar to that of Example (25a), by reaction of 500 µL (4.43 mmol, 5.4 eq) of 2-bromoethyl ethyl ether and 350 mg (0.82 mmol, 1 eq) of methyl 3-[3'-(3-heptyl-1-methylureido)-2-hydroxybiphenyl-4-yl]propanoate (prepared in Example 15f) in 10 ml of methyl ethyl ketone in the presence of 500 mg (3.61 mmol, 4.4 eq) of potassium carbonate at 90°C for 12 hours, methyl 3-[2-(2-ethoxyethoxy)-3'-(3-heptyl-1-methylureido)biphenyl-4-yl]propanoate is obtained in oil form and is used in the following reaction without further purification.

### b. 3-[2-(2-Ethoxyethoxy)-3'-(3-heptyl-1-methylureido)biphenyl-4-yl]propanoic acid

In a manner similar to that of Example (19g), by reaction of 400 mg (10 mmol, 14 eq) of sodium hydroxide and methyl 3-[2-(2-ethoxyethoxy)-3'-(3-heptyl-1-methylureido)biphenyl-4-yl]propanoate, obtained in the preceding step, in 10 ml of a tetrahydrofuran/methanol mixture (8/2), and after crystallization from pentane and isopropyl ether, 245 mg of 3-[2-(2-ethoxyethoxy)-3'-(3-heptyl-1-methylureido)biphenyl-4-yl]propanoic acid are obtained in the form of a white powder (m.p. = 63°C). Yield = 62% over the two steps.

¹H NMR (CDCl₃, 400 MHz) : 0.85 (t, 3H); 1.17 (t, 3H); 1.21-1.27 (m, 8H); 1.40 (m, 2H); 2.73 (t, 2H); 3.01 (t, 2H); 3.16 (q, 2H); 3.29 (s, 3H); 3.46-3.51 (q, 2H); 3.70 (m, 2H); 4.13 (m, 2H); 4.47 (t, 1H); 6.87 (s, 1 H); 6.90 (d, 1 H); 7.17 (d, 1 H); 7.24 (s, 1 H); 7.41 (t, 1 H); 7.51 (d, 2H).

### Example 48 : 3-[2-Butoxy-3'-(1-methyl-3-phenylureido)biphenyl-4-yl]propanoic acid

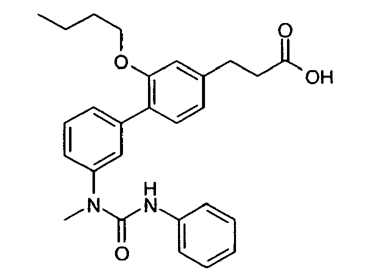

### a. 3-Hydroxy-4-iodobenzoic acid

21.0 g (0.52 mol, 1.05 eq) of sodium hydroxide and then 78.7 g (0.52 mol, 1.05 eq) of sodium iodide are added to a solution of 69.1 g (0.5 mol, 1 eq) of 3-hydroxybenzoic acid in 700 ml of methanol. The reaction mixture is cooled to 0°C and potassium hypochlorite solution (0.52 mol, 1.05 eq) is then added dropwise. The reaction medium is stirred at 0-5°C for 2 hours and then at room temperature overnight. The methanol is evaporated off and the reaction medium is then acidified with concentrated hydrochloric acid solution. The precipitated product is filtered off, washed with water and dried. 121 g of 3-hydroxy-4-iodobenzoic acid are obtained in the form of an off-white solid. Yield = 92 %

### b. Methyl 3-hydroxy-4-iodobenzoate

59 ml (1.10 mol, 2.4 eq) of sulphuric acid are added to a solution of 121 g (0.458 mol, 1 eq) of methyl 3-hydroxy-4-iodobenzoic acid in 700 ml of methanol. The reaction mixture is refluxed for 6 days. The methanol is evaporated off and the reaction medium is then poured into water and extracted with ethyl acetate. The organic phases are combined, washed with water and dried over sodium sulfate. The solvent is concentrated and the solid obtained is filtered off and dried. 88.56 g of methyl 3-hydroxy-4-iodobenzoate are obtained in the form of white crystals. Yield = 70%

### c. Methyl 3-butoxy-4-iodobenzoate

In a manner similar to that of Example (25a), by reaction of 21.5 mL (0.189 mol, 1.5 eq) of 1-iodobutane and 35.03 g (0.126 mol, 1 eq) of methyl 3-hydroxy-4-iodobenzoate in 350 ml of methyl ethyl ketone in the presence of 52.24 g (0.378 mol, 3 eq) of potassium carbonate at 85°C for 2 hours 30 minutes, and after washing with heptane, 41.78 g of methyl 3-butoxy-4-iodobenzoate are obtained in the form of white crystals. Yield = 99%

### d. (3-Butoxy-4-iodophenyl)methanol

8.17 g (0.375 mol, 3 eq) of lithium borohydride are added to a solution of 41.78 g (0.125 mol, 1 eq) of methyl 3-butoxy-4-iodobenzoate in 210 ml of tetrahydrofuran. The reaction medium is heated at 60°C for 2 hours and then hydrolysed cautiously in ice-cold saturated ammonium chloride solution. The reaction medium is neutralized with concentrated hydrochloric acid and then extracted with ethyl acetate. The organic phases are washed with water and dried over magnesium sulfate. The solvent is evaporated off and 38.31 g of (3-butoxy-4-iodophenyl)methanol are obtained in the form of a whitish oil. Yield = 100%

### e. 3-Butoxy-4-iodobenzaldehyde

89.5 g (0.875 mol, 7 eq) of manganese dioxide are added to a solution of 38.30 g (0.125 mol, 1 eq) of (3-butoxy-4-iodophenyl)methanol in 250 mL of dichloromethane. The reaction medium is stirred at room temperature for 18 hours and then filtered through silica gel. The solvent is evaporated off and 29.61 g of 3-butoxy-4-iodobenzaldehyde are obtained in the form of an orange oil. Yield = 78%

### f. Methyl (E)-3-(3-butoxy-4-iodophenyl)acrylate

65.08 g (0.195 mol, 2 eq) of methyl triphenylphosphoranylideneacetate are added to a solution of 29.60 g (0.097 mol, 1 eq) of methyl-3-butoxy-4-iodobenzaldehyde in 360 ml of toluene. The reaction mixture is refluxed for 2 hours. The solvent is evaporated off and the oil obtained is chromatographed on silica gel (50/50 heptane/dichloromethane). 30.47 g of methyl (E)-3-(3-butoxy-4-iodophenyl)acrylate are obtained in the form of pale yellow crystals. Yield = 87%

### g. Methyl (E)-3-(2-Butoxy-3'-methylaminobiphenyl-4-yl)acrylate

In a manner similar to that of Example (18d), by reaction of 28 mg (1 mol%) of palladium acetate, 88 mg (2 mol%) of dicyclohexylbiphenylphosphine, 4.5 g .(12.5 mmol, 1.0 eq) of methyl (E)-3-(3-butoxy-4-iodophenyl)acrylate and 3.79 g (16.2 mmol, 1.3 eq) of methyl[3-(4,4,5,5-tetramethyl[1,3,2]dioxaborolan-2-yl)phenyl]amine (prepared in Example 7a) in 72 mL of a mixture of dimethylformamide/2M potassium phosphate solution (5/1) at 90°C for 2 hours, 3.67 g of methyl (E)-3-(2-butoxy-3'-methylaminobiphenyl-4-yl)acrylate are obtained in the form of crystals. Yield = 86%

### h. Methyl 3-(2-butoxy-3'-methylaminobiphenyl-4-yl)propanoate

A solution of 3.63 g (10.7 mmol) of methyl (E)-3-(2-butoxy-3'-methylaminobiphenyl-4-yl)acrylate in 70 ml of methanol is stirred for 4 hours at room temperature in the presence of 363 mg (10% by mass) of 10% palladium-on-charcoal under a hydrogen atmosphere. The palladium is filtered off and the solvent is evaporated off. 3.39 g of methyl 3-(2-butoxy-3'-methylaminobiphenyl-4-yl)propanoate are obtained in the form of a yellowish oil. Yield = 93%.

### i. Methyl 3-[2-butoxy-3'-(1-methyl-3-phenylureido)biphenyl-4-yl]propanoate

In a manner similar to that of Example (1d), by reaction of 165 µl (1.5 mmol, 1.5 eq) of phenyl isocyanate and 342 mg (1.0 mmol, 1 eq) of methyl 3-(2-butoxy-3'-methylaminobiphenyl-4-yl)propanoate, 437 mg of methyl 3-[2-butoxy-3'-(1-methyl-3-phenylureido)biphenyl-4-yl]propanoate are obtained in the form of a yellow paste. Yield = 95%

### i. 3-[2-Butoxy-3'-(1-methyl-3-phenylureido)biphenyl-4-yl]propanoic acid

In a manner similar to that of Example (19g), by reaction of 187 mg (4.7 mmol, 5 eq) of sodium hydroxide and 437 mg (0.93 mmol, 1 eq) of methyl 3-[2-butoxy-3'-(1-methyl-3-phenylureido)biphenyl-4-yl]propanoate in 5 ml of methanol, 305 mg of 3-[2-butoxy-3'-(1-methyl-3-phenylureido)biphenyl-4-yl]propanoic acid are obtained in the form of white crystals (m.p. = 134-136°C). Yield = 73%

¹H NMR (CDCl₃, 400 MHz): 0.84 (t, 3H) ; 1.33-1.39 (m, 2H) ; 1.62-1.67 (m, 2H) ; 2.73 (t, 2H); 2.99 (t, 2H); 3.37 (s, 3H); 3.95 (t, 2H); 6.34 (s, 1H); 6.84 (s, 1H); 6.85 (d, 1H); 6.98 (t, 1 H); 7.2-7.3 (m, 5H); 7.50 (m, 2H); 7.57 (s, 1H).

### Example 49: 3-[2-(2-Ethoxyethoxy)-3'-(1-methyl-3-pentylureido)biphenyl-4-yl]propanoic acid

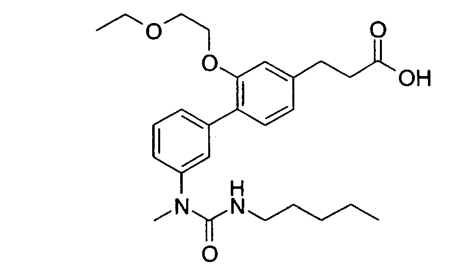

### a. Methyl 3-[2-(2-ethoxyethoxy)-3'-(1-methyl-3-pentylureido)biphenyl-4-yl]propanoate

In a manner similar to that of Example (25a), by reaction of 230 mg (1 mmol, 1.5 eq) of 2-bromoethyl ethyl ether and 400 mg (1.0 mmol, 1 eq) of methyl 3-[2-hydroxy-3'-(1-methyl-3-pentylureido)biphenyl-4-yl]propanoate (prepared as in Example 46d) in 10 ml of methyl ethyl ketone in the presence of 415 mg (3.0 mmol, 3 eq) of potassium carbonate and 60 mg (0.4 mmol, 0.4 eq) of sodium iodide at 90°C for 48 hours, 450 mg of methyl 3-[2-(2-ethoxyethoxy)-3'-(1-methyl-3-pentylureido)biphenyl-4-yl]propanoate are obtained in the form of a yellow oil. Yield = 96%

### b. 3-[2-(2-Ethoxyethoxy)-3'-(1-methyl-3-pentylureido)biphenyl-4-yl]propanoic acid

In a manner similar to that of Example (19g), by reaction of 181 mg (4.5 mmol, 5 eq) of sodium hydroxide and 427 mg (0.90 mmol, 1 eq) of methyl 3-[2-(2-ethoxyethoxy)-3'-(1-methyl-3-pentylureido)biphenyl-4-yl]propanoate in 5 ml of methanol, 304 mg of 3-[2-(2-ethoxyethoxy)-3'-(1-methyl-3-pentylureido)biphenyl-4-yl]propanoic acid are obtained in the form of a white powder (m.p. = 61-62°C). Yield = 73%

¹H NMR (CDCl₃, 400 MHz) : 0.84 (t, 3H); 1.15 (t, 3H); 1.20-1.27 (m, 4H); 1.37-1.42 (m, 2H); 2.72 (t, 2H); 2.99 (t, 2H); 3.46-3.51 (q, 2H); 3.70 (t, 2H); 4.13 (t, 2H); 4.49 (t, 1H); 6.87 (s, 1 H); 6.90 (d, 1 H); 7.15 (d, 1 H); 7.17 (s, 1H); 7.41 (t, 3H); 7.50 (s, 1 H); 7.51 (d, 1H).

### Example 50: 3-[2-(2-Diethylaminoethoxy)-3'-(3-heptyl-1-methylureido)biphenyl-4-yl]propanoic acid hydrochloride

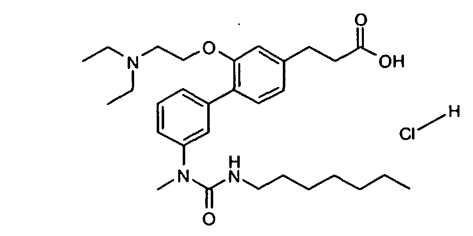

### a. Methyl 3-[2-(2-diethylaminoethoxy)-3'-(3-heptyl-1-methylureido)biphenyl-4-yl]propanoate

A solution of 500 mg (1.17 mmol, 1 eq) of methyl 3-[3'-(3-heptyl-1-methylureido)-2-hydroxybiphenyl-4-yl]propanoate (prepared in Example 15f) in 5 ml of tetrahydrofuran in the presence of 150 mg (1.29 mmol, 1.1 eq) of 2-diethylaminoethanol, 460 mg (1.76 mmol, 1.5 eq) of triphenylphosphine and 280 µL (1.76 mmol, 1.5 eq) of diethyl azodicarboxylate (DEAD) is stirred at room temperature for 24 hours. The reaction medium is concentrated and the residue is then chromatographed on silica gel (40/60 heptane/ethyl acetate). 490 mg of methyl 3-[2-(2-diethylaminoethoxy)-3'-(3-heptyl-1-methylureido)biphenyl-4-yl]propanoate are obtained.
Yield = 80%

### b. 3-[2-(2-Diethylaminoethoxy)-3'-(3-heptyl-1-methylureido)biphenyl-4-yl]propanoic acid hydrochloride

220 mg (9.3 mmol, 10 eq) of lithium hydroxide are added to a solution of 490 mg (0.93 mmol) of methyl 3-[2-(2-diethylaminoethoxy)-3'-(3-heptyl-1-methylureido)biphenyl-4-yl]propanoate in 7 ml of 5/1/1 tetrahydrofuran/methanol/water. The reaction mixture is stirred for 4 hours at room temperature. The reaction medium is hydrolysed with water and extracted with ethyl acetate. The organic phases are combined, washed with water and dried over sodium sulfate. The solvents are evaporated off and the residue is then chromatographed on silica gel (90/10 dichloromethane/methanol), purified on a preparative thin-layer silica plate and then placed in hydrochloride form. 14 mg of 3-[2-(2-diethylaminoethoxy)-3'-(3-heptyl-1-methylureido)biphenyl-4-yl]propanoic acid hydrochloride are obtained in the form of a colourless oil. Yield = 3%
¹H NMR (CDCl₃, 400 MHz) : 0.77 (t, 3H); 0.93 (t, 3H); 1.16 (m, 8H); 1.32 (m, 2H); 2.48 (t, 2H); 2.63-2.68 (q, 4H); 2.87 (m, 4H); 3.04-3.08 (q, 2H) ; 3.19 (s, 3H); 4.11 (m, 2H); 4.36 (t, 1H); 6.77 (d, 1 H); 6.82 (s, 1 H); 7.07 (d, 2H); 7.30 (d, 3H).

### Example 51 : 3-[3'-(3-Heptyl-1-methylureido)-2-(2-morpholin-4-ylethoxy)biphenyl-4-yl]propanoic acid

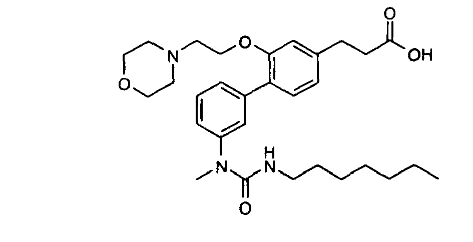

### a. Methyl 3-[3'-(3-Heptyl-1-methylureido)-2-(2-morpholin-4-ylethoxy)biphenyl-4-yl]propanoate

In a manner similar to that of Example (50a), by reaction of 500 mg (1.17 mmol, 1 eq) of methyl 3-[3'-(3-heptyl-1-methylureido)-2-hydroxybiphenyl-4-yl]propanoate (prepared in Example 15f) in 5 ml of tetrahydrofuran, 170 mg (1.29 mmol, 1.1 eq) of N-(2-hydroxyethyl)morpholine, 460 mg (1.76 mmol, 1.5 eq) of triphenylphosphine and 280 µL (1.76 mmol, 1.5 eq) of diethyl azodicarboxylate (DEAD), 420 mg of methyl 3-[3'-(3-heptyl-1-methylureido)-2-(2-morpholin-4-ylethoxy)biphenyl-4-yl]propanoate are obtained. Yield = 68%

### b. 3-[3'-(3-Heptyl-1-methylureido)-2-(2-morpholin-4-ylethoxy)biphenyl-4-yl]propanoic acid

630 mg (1.56 mmol, 2 eq) of sodium hydroxide are added to a solution of 420 mg (0.78 mmol, 1 eq) of methyl 3-[3'-(3-heptyl-1-methylureido)-2-(2-morpholin-4-ylethoxy)biphenyl-4-yl]propanoate in 8 ml of 8/1/1 tetrahydrofuran/methanol/water. The reaction mixture is stirred for 4 hours at room temperature. The reaction medium is hydrolysed with water and acetic acid and extracted with ethyl acetate. The organic phases are combined, washed with water and dried over sodium sulfate. The solvents are evaporated off and the residue is then chromatographed on silica gel (40/60 heptane/ethyl acetate) and then on preparative HPLC. 10 mg of 3-[3'-(3-heptyl-1-methylureido)-2-(2-morpholin-4-ylethoxy)biphenyl-4-yl]propanoic acid are obtained in oil form. Yield = 2%

¹H NMR (CDCl₃, 400 MHz) : 0.79 (t, 3H); 1.18 (m, 8H); 1.37 (m, 2H); 2.57 (t, 2H); 2.94 (t, 2H); 3.02-3.14 (q, 2H); 3.22 (s, 3H); 3.31 (m, 2H); 3.73 (m, 4H); 4.38 (t, 2H); 4.43 (t, 1 H); 6.85 (s, 1 H); 6.87 (d, 1H); 7.10-7.17 (m, 2H); 7.24 (d, 1 H); 7.33-7.37 (m, 3H).

### Example 52 : 3-[2-Amino-3'-(1-methyl-3-pentylureido)biphenyl-4-yl]propanoic acid

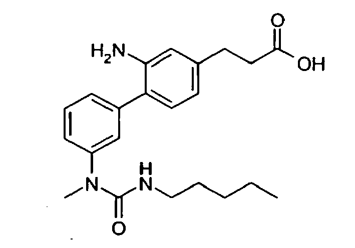

### a. Methyl (E)-3-(4-chloro-3-nitrophenyl)acrylate

A solution of 5.0 g (0.027 mol, 1 eq) of 4-chloro-3-nitrobenzaldehyde in 150 ml of toluene in the presence of 18.0 g (0.054 mol, 2 eq) of triphenylphospharanylidene is refluxed for 1 hour 30 minutes. The cooled reaction medium is hydrolysed by addition of 100 mL of water and the phases are then separated by settling. The organic phase is dried over sodium sulfate and evaporated. The residue is chromatographed on silica gel (70/30 heptane/ethyl acetate) and 6.3 g of methyl (E)-3-(4-chloro-3-nitrophenyl)acrylate are obtained in the form of a pale yellow solid. Yield = 97%

### b. Methyl (E)-3-[3'-(1-methyl-3-pentylureido)-2-nitrobiphenyl-4-yl]acrylate

In a manner similar to that of Example (18d), by reaction of 38 mg (2 mol%) of palladium acetate, 118 mg (4 mol%) of dicyclohexylbiphenylphosphine, 2.0 g (8.28 mmol, 1.0 eq) of methyl (E)-3-(4-chloro-3-nitrophenyl)acrylate and 2.6 g (9.9 mmol, 1.2 eq) of 3-(1-methyl-3-pentylureido)phenylboronic acid in 25 mL of a mixture of dimethylformamide/2M potassium phosphate solution (4/1), 2.90 g of methyl (E)-3-[3'-(1-methyl-3-pentylureido)-2-nitrobiphenyl-4-yl]acrylate are obtained in the form of a yellow oil. Yield = 82%

### c. Methyl 3-[2-amino-3'-(1-methyl-3-pentylureido)biphenyl-4-yl]propanoate

In a manner similar to that of Example (18e), by reaction of 300 mg (10% by mass) of 10% palladium-on-charcoal and 2.90 g (6.8 mmol, 1 eq) of methyl (E)-3-[3'-(1-methyl-3-pentylureido)-2-nitrobiphenyl-4-yl]acrylate in 50 ml of methanol, and after purification by chromatography on silica gel (50/50 heptane/ethyl acetate), 2.57 g of methyl 3-[2-amino-3'-(1-methyl-3-pentylureido)biphenyl-4-yl]propanoate are obtained in the form of a yellow oil. Yield = 95%

### d. 3-[2-Amino-3'-(1-methyl-3-pentylureido)biphenyl-4-yl]propanoic acid

A solution of 123.9 mg (0.311 mmol, 1 eq) of methyl 3-[2-amino-3'-(1-methyl-3-pentylureido)biphenyl-4-yl]propanoate in 3 ml of tetrahydrofuran in the presence of 550 µL (0.623 mmol, 2 eq) of 1N lithium hydroxide solution is stirred at room temperature for 4 hours. The reaction medium is concentrated, hydrolysed with water, acidified with acetic acid and extracted with ethyl acetate. The organic phase is dried over sodium sulfate and evaporated. The residue is taken up in heptane and then recrystallized from acetonitrile. 302.8 mg of 3-[2-amino-3'-(1-methyl-3-pentylureido)biphenyl-4-yl]propanoic acid are obtained in the form of a white powder (m.p. = 84.9°C) Yield = 75%

¹H NMR (CDCl₃, 400 MHz) : 0.85 (t, 3H); 1.19-1.31 (m, 4H); 1.31-1.46 (m, 2H); 2.70 (t, 2H); 2.92 (t, 2H); 3.15-3.20 (q, 2H) ; 3.29 (s, 3H); 4.45 (t, 1 H); 6.65 (s, 1 H); 6.69 (d, 1 H); 7.03 (d, 1 H); 7.22 (d, 1H); 7.34 (s, 1 H); 7.39 (d, 1 H); 7.47 (t, 1 H).

### Example 53: 3-[2-Butylamino-3'-(1-methyl-3-pentylureido)biphenyl-4-yl]propanoic acid hydrochloride

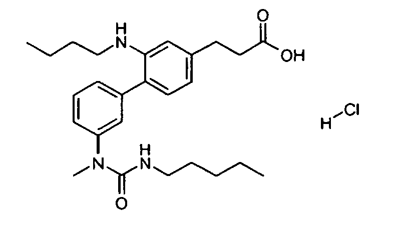

### a. Methyl 3-[2-butylamino-3'-(1-methyl-3-pentylureido)biphenyl-4-yl]propanoate

A solution of 200 mg (0.5 mmol, 1 eq) of methyl 3-[2-amino-3'-(1-methyl-3-pentylureido)biphenyl-4-yl]propanoate (prepared in Example 52c) and 60 µL (0.55 mmol, 1.1 eq) of 1-iodobutane in 2 ml of dimethylformamide in the presence of 170 µL (10.5 mmol, 2.1 eq) of diisopropylethylamine is heated at 80°C for 16 hours. The reaction medium is hydrolysed with water and then extracted with ethyl acetate. The organic phases are combined, washed with saturated sodium chloride solution and dried over sodium sulfate. The solvent is evaporated off and the residue is chromatographed on silica gel (70/30 heptane/ethyl acetate). 114.8 mg of methyl 3-[2-butylamino-3'-(1-methyl-3-pentylureido)biphenyl-4-yl]propanoate are obtained in the form of a yellow oil. Yield = 51%

### b. 3-[2-Butylamino-3'-(1-methyl-3-pentylureido)biphenyl-4-yl]propanoic acid hydrochloride

In a manner similar to that of Example (52d), by reaction of 114.8 mg (0.25 mmol, 1 eq) of methyl 3-[2-butylamino-3'-(1-methyl-3-pentylureido)biphenyl-4-yl]propanoate and 500 µL (0.50 mmol, 2 eq) of 1N lithium hydroxide solution in 2 ml of tetrahydrofuran, the product is obtained and is precipitated in hydrochloride form by addition of a solution of hydrogen chloride in ethyl acetate. The solid obtained is recrystallized from acetonitrile and 44 mg of 3-[2-butylamino-3'-(1-methyl-3-pentylureido)biphenyl-4-yl]propanoic acid hydrochloride are obtained in the form of a white powder (m.p. = 163.9°C). Yield = 37%

¹H NMR (CDCl₃, 400 MHz) : 0.79 (t, 3H); 0.82 (t, 3H); 1.18-1.28 (m, 6H); 1.41-1.49 (m, 2H); 1.63-1.69 (m, 2H); 2.70 (t, 2H); 3.02 (t, 2H); 3.08-3.16 (m, 4H) ; 3.29 (s, 3H); 5.35 (m, 1 H); 7.24-7.36 (m, 5H); 7.50 (t, 1H); 7.72 (s, 1 H).

### Example 54 : 3-[2-Benzylamino-3'-(1-methyl-3-pentylureido)biphenyl-4-yl]propanoic acid hydrochloride

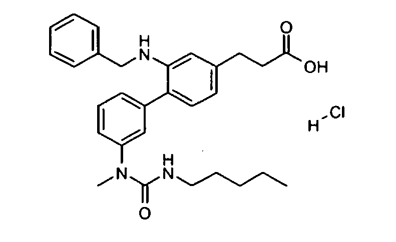

### a. Methyl 3-[2-benzylamino-3'-(1-methyl-3-pentylureido)biphenyl-4-yl]propanoate

A solution of 200 mg (0.5 mmol, 1 eq) of methyl 3-[2-amino-3'-(1-methyl-3-pentylureido)biphenyl-4-yl]propanoate (prepared in Example 52c) and 72 µL (0.70 mmol, 1.4 eq) of benzaldehyde in 4 ml of methanol in the presence of 63 mg (1.0 mmol, 2 eq) of sodium cyanoborohydride is stirred at room temperature for 16 hours. The reaction medium is hydrolysed with ammonium chloride solution and then extracted with ethyl acetate. The organic phases are combined, washed with saturated sodium chloride solution and dried over sodium sulfate. The solvent is evaporated off and the residue is chromatographed on silica gel (70/30 heptane/ethyl acetate). 144 mg of methyl 3-[2-benzylamino-3'-(1-methyl-3-pentylureido)biphenyl-4-yl]propanoate are obtained in the form of a colourless oil. Yield = 59%

### b. 3-[2-Benzylamino-3'-(1-methyl-3-pentylureido)biphenyl-4-yl]propanoic acid hydrochloride

In a manner similar to that of Example (52d), by reaction of 144 mg (0.30 mmol, 1 eq) of methyl 3-[2-benzylamino-3'-(1-methyl-3-pentylureido)biphenyl-4-yl]propanoate and 590 µL (0.60 mmol, 2 eq) of 1 N lithium hydroxide solution in 2 ml of tetrahydrofuran, 42 mg of 3-[2-benzylamino-3'-(1-methyl-3-pentylureido)biphenyl-4-yl]propanoic acid hydrochloride are obtained in the form of a white powder (m.p. = 105°C). Yield = 28%

¹H NMR (CDCl₃, 400 MHz) : 0.84 (t, 3H); 1.20-1.30 (m, 8H); 1.41-1.49 (m, 2H); 2.65 (t, 2H); 2.95 (t, 2H); 3.17 (m, 2H) ; 3.22 (s, 3H); 4.32 (s, 2H); 4.95 (m, 1 H); 6.98 (m, 2H); 7.07 (d, 2H); 7.20-7.28 (m, 7H); 7.39 (t, 1H).

### Example 55 : 3-[2-Diethylamino-3'-(1-methyl-3-pentylureido)biphenyl-4-yl]propanoic acid hydrochloride

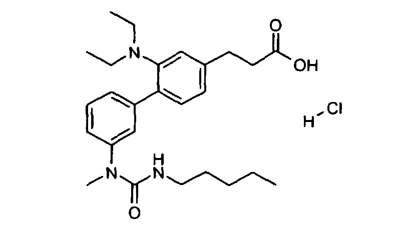

### a. Methyl 3-[2-diethylamino-3'-(1-methyl-3-pentylureido)biphenyl-4-yl]propanoate

In a manner similar to that of Example (54a), by reaction of 300 mg (0.75 mmol, 1 eq) of methyl 3-[2-amino-3'-(1-methyl-3-pentylureido)biphenyl-4-yl]propanoate (prepared in Example 52c) and 100 µL (1.70 mmol, 2.4 eq) of acetaldehyde in 6 ml of methanol in the presence of 95 mg (1.5 mmol, 2 eq) of sodium cyanoborohydride, 279.3 mg of methyl 3-[2-diethylamino-3'-(1-methyl-3-pentylureido)biphenyl-4-yl]propanoate are obtained in the form of a colourless oil. Yield = 82%

### b. 3-[2-Diethylamino-3'-(1-methyl-3-pentylureido)biphenyl-4-yl]propanoic acid hydrochloride

In a manner similar to that of Example (52d), by reaction of 279 mg (0.62 mmol, 1 eq) of methyl 3-[2-diethylamino-3'-(1-methyl-3-pentylureido)biphenyl-4-yl]propanoate and 1.23 mL (1.24 mmol, 2 eq) of 1 N lithium hydroxide solution in 3 ml of tetrahydrofuran, 214 mg of 3-[2-diethylamino-3'-(1-methyl-3-pentylureido)biphenyl-4-yl]propanoic acid hydrochloride are obtained in the form of a white powder (m.p. = 183.2°C) Yield = 73%

¹H NMR (CDCl₃, 400 MHz) : 0.84 (t, 3H); 1.15 (t, 6H); 1.22-1.31 (m, 4H); 1.49-1.54 (m, 2H); 2.70 (t, 2H); 3.07 (t, 2H); 3.14 (m, 2H); 3.25 (s, 3H); 3.77 (m, 2H); 6.65 (m, 1H); 7.03 (s, 1 H); 7.07 (d, 1 H); 7.30 (d, 2H); 7.38 (d, 2H); 7.52 (t, 1 H); 11.2 (m, 1 H).

### Example 56: 3-[3'-(1-Methyl-3-pentylureido)-2-propylaminobiphenyl-4-yl]propanoic acid hydrochloride

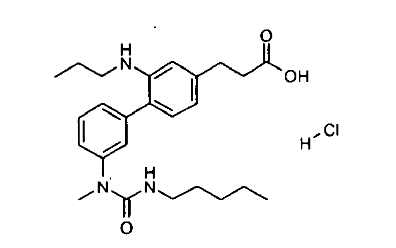

### a. Methyl 3-[3'-(1-methyl-3-pentylureido)-2-propylaminobiphenyl-4-yl]propanoate

In a manner similar to that of Example (54a), by reaction of 300 mg (0.75 mmol, 1 eq) of methyl 3-[2-amino-3'-(1-methyl-3-pentylureido)biphenyl-4-yl]propanoate (prepared in Example 52c) and 77 µL (1.70 mmol, 1.4 eq) of propionaldehyde in 6 ml of methanol in the presence of 95 mg (1.5 mmol, 2 eq) of sodium cyanoborohydride, 207.3 mg of methyl 3-[3'-(1-methyl-3-pentylureido)-2-propylaminobiphenyl-4-yl]propanoate are obtained in the form of a colourless oil. Yield = 62%

### b. 3-[3'-(1-Methyl-3-pentylureido)-2-propylaminobiphenyl-4-yl]propanoic acid hydrochloride

In a manner similar to that of Example (52d), by reaction of 207 mg (0.47 mmol, 1 eq) of methyl 3-[3'-(1-methyl-3-pentylureido)-2-propylaminobiphenyl-4-yl]propanoate and 0.94 mL (0.94 mmol, 2 eq) of 1 N lithium hydroxide solution in 4 ml of tetrahydrofuran, 178.4 mg of 3-[3'-(1-methyl-3-pentylureido)-2-propylaminobiphenyl-4-yl]propanoic acid hydrochloride are obtained in the form .of a white powder (m.p. = 172.8°C). Yield = 82%
¹H NMR (CDCl₃, 400 MHz) : 0.79-0.84 (2t, 6H); 1.18-1.28 (m, 8H); 1.41-1.47 (m, 2H); 1.67-1.73 (m, 2H); 2.69 (t, 2H); 3.02 (t, 2H); 3.06 (m, 2H) ; 3.14 (t, 2H); 3.29 (s, 3H); 5.45 (m, 1H); 7.24-7.34 (m, 5H); 7.50 (t, 1 H); 7.68 (s, 1H).

### Example 57: 3-[2-(4-Fluorobenzylamino)-3'-(1-methyl-3-pentylureido)biphenyl-4-yl]propanoic acid hydrochloride

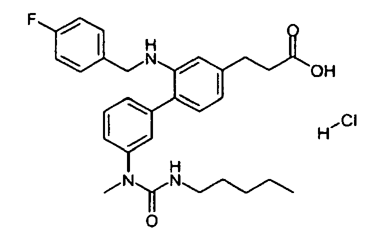

### a. Methyl 3-[2-(4-Fluorobenzvlamino)-3'-(1-methyl-3-pentylureido)biphenyl-4-yl]propanoate

In a manner similar to that of Example (54a), by reaction of 200 mg (0.5 mmol, 1 eq) of methyl 3-[2-amino-3'-(1-methyl-3-pentylureido)biphenyl-4-yl]propanoate (prepared in Example 52c) and 76 µL (0.70 mmol, 1.4 eq) of 4-fluorobenzaldehyde in 4 ml of methanol in the presence of 63 mg (1.0 mmol, 2 eq) of sodium cyanoborohydride, 220.3 mg of methyl 3-[2-(4-fluorobenzylamino)-3'-(1-methyl-3-pentylureido)biphenyl-4-yl]propanoate are obtained in the form of a colourless oil. Yield = 87%

### b. 3-[2-(4-Fluorobenzylamino)-3'-(1-methyl-3-pentylureido)biphenyl-4-yl]propanoic acid hydrochloride

In a manner similar to that of Example (52d), by reaction of 220 mg (0.47 mmol, 1 eq) of methyl 3-[2-(4-fluorobenzylamino)-3'-(1-methyl-3-pentylureido)biphenyl-4-yl]propanoate and 0.87 mL (0.87 mmol, 2 eq) of 1N lithium hydroxide solution in 4 ml of tetrahydrofuran, 98 mg of 3-[2-(4-fluorobenzylamino)-3'-(1-methyl-3-pentylureido)-biphenyl-4-yl]propanoic acid hydrochloride are obtained in the form of a white powder (m.p. = 169.6°C). Yield = 43%

¹H NMR (CD₃OD, 400 MHz) : 0.88 (t, 3H); 1.29-1.33 (m, 4H); 1.49-1.54 (m, 2H); 2.68 (m, 2H); 3.01 (m, 2H); 3.18 (m, 2H); 3.25 (s, 3H); 4.49 (s, 2H); 6.84 (s, 1H); 7.01-7.07 (m, 3H); 7.15 (m, 2H); 7.16 (d, 1 H); 7.33-7.37 (m, 3H); 7.50 (m, 1H).

### Example 58: 3-[2-Butylamino-3'-(3-heptyl-1-methylureido)biphenyl-4-yl]propanoic acid hydrochloride

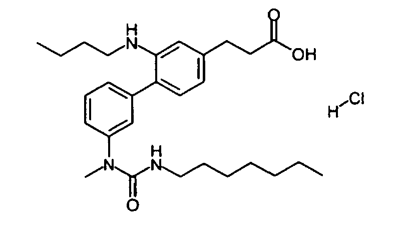

### a. 1-(3-Bromophenyl)-3-heptyl-1-methylurea

In a manner similar to that of Example (46a), by reaction of 50.0 g (0.354 mol, 1 eq) of heptyl isocyanate and 65.9 g (0.354 mol, 1 eq) of (3-bromophenyl)methylamine (prepared in Example 7c) in 300 mL of dichloromethane in the presence of 20 mL (0.143 mol, 0.4 eq) of triethylamine, 113 g of 1-(3-bromophenyl)-3-heptyl-1-methylurea are obtained. Yield = 97%

### b. 3-(3-Heptyl-1-methylureido)phenylboronic acid

In a manner similar to that of Example (18a), by reaction of 113 g (0.345 mol, 1 eq) of 1-(3-bromophenyl)-3-heptyl-1-methylurea in 1.13 L of tetrahydrofuran, 127 mL (0.38 mol, 1.1 eq) of methyllithium, 530 mL (0.76 mol, 2.2 eq) of a 1.7M solution of tert-butyllithium in pentane and 97 mL (0.904 mol, 2.2 eq) of trimethyl borate, and after purification by chromatography on silica gel (50/50 heptane/ethyl acetate) and crystallization from ethyl acetate/heptane, 36.0 g of 3-(3-heptyl-1-methylureido)phenylboronic acid are obtained in the form of a pinkish powder. Yield = 36%

### c. Methyl (E)-3-[3'-(3-heptyl-1-methylureido)-2-nitrobiphenyl-4-yl]acrylate

In a manner similar to that of Example (18d), by reaction of 56 mg (2 mol%) of palladium acetate, 174 mg (4 mol%) of dicyclohexylbiphenylphosphine, 3.0 g (12.0 mmol, 1.0 eq) of methyl (E)-3-(4-chloro-3-nitrophenyl)acrylate (prepared in Example 52a) and 4.4 g (15.0 mmol, 1.2 eq) of 3-(3-heptyl-1-methylureido)phenylboronic acid in 48 mL of a mixture of dimethylformamide/2M potassium phosphate solution (5/1), 4.06 g of methyl (E)-3-[3'-(3-heptyl-1-methylureido)-2-nitrobiphenyl-4-yl]acrylate are obtained in the form of a pale yellow powder. Yield = 72%

### d. Methyl 3-[2-amino-3'-(3-heptyl-1-methylureido)biphenyl-4-yl]propanoate

In a manner similar to that of Example (18e), by reaction of 400 mg (10% by mass) of 10% palladium-on-charcoal and 4.0 g (8.8 mmol, 1 eq) of methyl (E)-3-[3'-(3-heptyl-1-methylureido)-2-nitrobiphenyl-4-yl]acrylate in 80 ml of methanol, and after purification by chromatography on silica gel (50/50 heptane/ethyl acetate), 3.81 g of methyl 3-[2-amino-3'-(3-heptyl-1-methylureido)biphenyl-4-yl]propanoate are obtained in the form of a colourless oil. Yield = 100%

### e. Methyl 3-[2-butylamino-3'-(3-heptyl-1-methylureido)biphenyl-4-yl]propanoate

In a manner similar to that of Example (54a), by reaction of 400 mg (0.94 mmol, 1 eq) of methyl 3-[2-amino-3'-(3-heptyl-1-methylureido)biphenyl-4-yl]propanoate and 120 µL (1.30 mmol, 1.4 eq) of butyraldehyde in 8 ml of methanol in the presence of 120 mg (1.9 mmol, 2 eq) of sodium cyanoborohydride, 335.5 mg of methyl 3-[2-butylamino-3'-(3-heptyl-1-methylureido)biphenyl-4-yl]propanoate are obtained in the form of a colourless oil. Yield = 74%

### f. 3-[2-Butylamino-3'-(3-heptyl-1-methylureido)biphenyl-4-yl]propanoic acid hydrochloride

In a manner similar to that of Example (52d), by reaction of 155 mg (0.32 mmol, 1 eq) of methyl 3-[2-butylamino-3'-(3-heptyl-1-methylureido)biphenyl-4-yl]propanoate and 0.64 mL (0.64 mmol, 2 eq) of 1N lithium hydroxide solution in 2 ml of tetrahydrofuran, 67 mg of 3-[2-butylamino-3'-(3-heptyl-1-methylureido)biphenyl-4-yl]propanoic acid hydrochloride are obtained in the form of a white powder (m.p. = 132.4°C) Yield = 42%

¹H NMR (CDCl₃, 400 MHz) : 0.79 (t, 3H); 0.83 (t, 3H); 1.16-1.28 (m, 10H); 1.45 (m, 2H); 1.63-1.70 (m, 2H); 2.70 (t, 2H); 3.02-3.10 (m, 4H); 3.10 (t, 2H); 3.30 (s, 3H); 5.25 (m, 1 H); 7.23-7.34 (m, 4H); 7.38 (d, 1 H); 7.51 (t, 1H); 7.75 (s, 1 H).

### Example 59 : 3-[2-Benzylamino-3'-(3-heptyl-1-methylureido)biphenyl-4-yl]propanoic acid hydrochloride

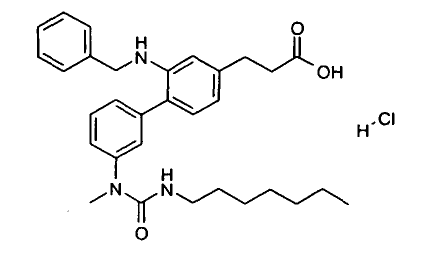

### a. Methyl 3-[2-benzylamino-3'-(3-heptyl-1-methylureido)biphenyl-4-yl]propanoate

In a manner similar to that of Example (54a), by reaction of 200 mg (0.5 mmol, 1 eq) of methyl 3-[2-amino-3'-(3-heptyl-1-methylureido)biphenyl-4-yl]propanoate (prepared in Example 58d) and 67 µL (0.66 mmol, 1.4 eq) of benzaldehyde in 4 ml of methanol in the presence of 59 mg (0.94 mmol, 2 eq) of sodium cyanoborohydride, 126.8 mg of methyl 3-[2-benzylamino-3'-(3-heptyl-1-methylureido)biphenyl-4-yl]propanoate are obtained in the form of a colourless oil. Yield = 52%

### b. 3-[2-Benzvlamino-3'-(3-heptyl-1-methylureido)biphenyl-4-yl]propanoic acid hydrochloride

In a manner similar to that of Example (52d), by reaction of 126 mg (0.25 mmol, 1 eq) of methyl 3-[2-benzylamino-3'-(3-heptyl-1-methylureido)biphenyl-4-yl]propanoate and 0.98 mL (0.98 mmol, 2 eq) of 1 N lithium hydroxide solution in 2 ml of tetrahydrofuran, 57 mg of 3-[2-benzylamino-3'-(3-heptyl-1-methylureido)biphenyl-4-yl]propanoic acid hydrochloride are obtained in the form of a white powder (m.p. = 145.3°C). Yield = 43%

¹H NMR (CDCl₃, 400 MHz) : 0.84 (t, 3H); 1.24-1.27 (m, 8H); 1.49 (m, 2H); 2.63 (t, 2H); 2.97 (t, 2H); 3.18 (m, 2H); 3.20 (s, 3H); 4.32 (s, 2H); 5.5 (m, 1H); 6.70 (s, 1H); 6.80 (d, 1 H); 7.09-7.36 (m, 9H); 7.42 (s, 1 H).

### Example 60 : 3-[2-Diethylamino-3'-(3-heptyl-1-methylureido)biphenyl-4-yl]propanoic acid hydrochloride

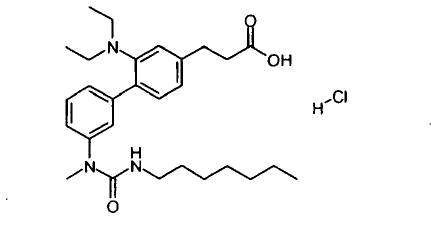

### a. Methyl 3-[2-diethylamino-3'-(3-heotyl-1-methylureido)biphenyl-4-yl]propanoate

In a manner similar to that of Example (54a), by reaction of 300 mg (0.7 mmol, 1 eq) of methyl 3-[2-amino-3'-(3-heptyl-1-methylureido)biphenyl-4-yl]propanoate (prepared in Example 58d) and 60 µL (1.06 mmol, 1.5 eq) of acetaldehyde in 6 ml of methanol in the presence of 89 mg (1.4 mmol, 2 eq) of sodium cyanoborohydride, 210 mg of methyl 3-[2-diethylamino-3'-(3-heptyl-1-methylureido)biphenyl-4-yl]propanoate are obtained in the form of a colourless oil. Yield = 62%

### b. 3-[2-Diethylamino-3'-(3-heptyl-1-methylureido)biphenyl-4-yl]propanoic acid hydrochloride

In a manner similar to that of Example (52d), by reaction of 210 mg (0.44 mmol, 1 eq) of methyl 3-[2-diethylamino-3'-(3-heptyl-1-methylureido)biphenyl-4-yl]propanoate and 0.87 mL (0.87 mmol, 2 eq) of 1 N lithium hydroxide solution in 4 ml of tetrahydrofuran, 132 mg of 3-[2-diethylamino-3'-(3-heptyl-1-methylureido)biphenyl-4-yl]propanoic acid hydrochloride are obtained in the form of a white powder (m.p. = 178.6°C). Yield = 60%

¹H NMR (CDCl₃, 400 MHz) : 0.84 (t, 3H); 1.13 (t, 3H); 1.23-1.28 (m, 8H); 1.50 (m, 2H); 2.74 (t, 2H); 3.08 (t, 2H); 3.23 (s, 3H); 3.27 (m, 2H) ; 3.75 (m, 2H); 6.7 (m, 1 H); 7.00 (s, 1 H); 7.08 (d, 1 H); 7.28 (d, 2H); 7.37 (d, 2H); 7.51 (t, 1H).

### Example 61 : 3-[3'-(3-Heptyl-1-methylureido)-2-propylaminobiphenyl-4-yl]propanoic acid hydrochloride

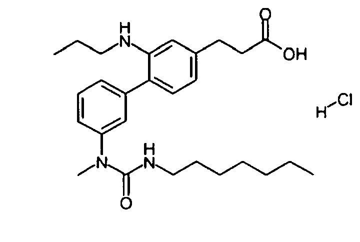

### a. Methyl 3-[3'-(3-heptyl-1-methylureido)-2-propylaminobiphenyl-4-yl]propanoate

In a manner similar to that of Example (54a), by reaction of 300 mg (0.7 mmol, 1 eq) of methyl 3-[2-amino-3'-(3-heptyl-1-methylureido)biphenyl-4-yl]propanoate (prepared in Example 58d) and 72 µL (0.99 mmol, 1.4 eq) of propionaldehyde in 6 ml of methanol in the presence of 89 mg (1.4 mmol, 2 eq) of sodium cyanoborohydride, 131.6 mg of methyl 3-[3'-(3-heptyl-1-methylureido)-2-propylaminobiphenyl-4-yl]propanoate are obtained in the form of a colourless oil. Yield = 40%

### b. 3-[3'-(3-Heotyl-1-methylureido)-2-propylaminobiphenyl-4-yl]propanoic acid hydrochloride

In a manner similar to that of Example (52d), by reaction of 131 mg (0.44 mmol, 1 eq) of methyl 3-[3'-(3-heptyl-1-methylureido)-2-propylaminobiphenyl-4-yl]propanoate and 0.56 mL (0.56 mmol, 2 eq) of 1N lithium hydroxide solution in 2 ml of tetrahydrofuran, 102 mg of 3-[3'-(3-heptyl-1-methylureido)-2-propylaminobiphenyl-4-yl]propanoic acid hydrochloride are obtained in the form of a white powder (m.p. = 140.8°C). Yield = 74%

¹H NMR (CDCl₃, 400 MHz) : 0.79-0.86 (2t, 6H); 1.25 (m, 8H); 1.45 (m, 2H); 1.67-1.72 (m, 2H); 2.70 (t, 2H); 3.02 (t, 2H); 3.05 (m, 2H); 3.15 (m, 2H); 3.29 (s, 3H); 5.35 (m, 1H); 7.23-7.34 (m, 5H); 7.50 (t, 1 H); 7.67 (s, 1H).

### Example 62 : 3-[2-(4-Fluorobenzylamino)-3'-(3-heptyl-1-methylureido)biphenyl-4-yl]-propanoic acid hydrochloride

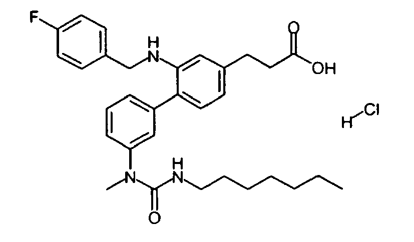

### a. Methyl 3-[2-(4-fluorobenzylamino)-3'-(3-heptyl-1-methylureido)biphenyl-4-yl]propanoate

In a manner similar to that of Example (54a), by reaction of 200 mg (0.47 mmol, 1 eq) of methyl 3-[2-amino-3'-(3-heptyl-1-methylureido)biphenyl-4-yl]propanoate (prepared in Example 58d) and 71 µL (0.99 mmol, 1.4 eq) of 4-fluorobenzaldehyde in 4 ml of methanol in the presence of 59 mg (1.4 mmol, 2 eq) of sodium cyanoborohydride, 207.1 mg of methyl 3-[2-(4-fluorobenzylamino)-3'-(3-heptyl-1-methylureido)biphenyl-4-yl]propanoate are obtained in the form of a colourless oil. Yield = 83%

### b. 3-[2-(4-Fluorobenzylamino)-3'-(3-heptyl-1-methylureido)biphenyl-4-yl]propanoic acid hydrochloride

In a manner similar to that of Example (52d), by reaction of 207 mg (0.38 mmol, 1 eq) of methyl 3-[2-(4-fluorobenzylamino)-3'-(3-heptyl-1-methylureido)biphenyl-4-yl]propanoate and 0.78 mL (0.78 mmol, 2 eq) of 1 N lithium hydroxide solution in 4 ml of tetrahydrofuran, 154 mg of 3-[2-(4-fluorobenzylamino)-3'-(3-heptyl-1-methylureido)-biphenyl-4-yl]propanoic acid hydrochloride are obtained in the form of a white powder. Crude yield = 73%

¹H NMR (CD₃OD, 400 MHz) : 0.80 (t, 3H); 1.14-1.20 (m, 8H); 1.39 (m, 2H); 2.59 (m, 2H); 3.02 (m, 2H); 3.06 (m, 2H); 3.20 (s, 3H); 4.24 (s, 2H); 6.91 (m, 1H); 7.18-7.29 (m, 8H); 7.41 (t, 1H); 7.46 (m, 1 H).

### EXAMPLE 63 - CROSS-CURVE PPAR TRANSACTIVATION TESTS

The activation of PPAR receptors with an agonist (activator) in HeLN cells leads to the expression of a reporter gene, luciferase, which, in the presence of a substrate, generates light. The modulation of the PPAR receptors is measured by quantifying the luminescence produced after incubating the cells in the presence of a reference agonist. The ligands displace the agonist from its site. The measurement of the activity is performed by quantifying the light produced. This measurement makes it possible to determine the modulatory activity of the compounds according to the invention by determining the constant that represents the affinity of the molecule for the PPAR receptor. Since this value can fluctuate depending on the basal activity and the expression of the receptor, it is referred to as Kd apparent (KdApp in nM).

To determine this constant, "cross curves" of the test product against a reference agonist are produced in a 96-well plate: 10 concentrations of the test product plus a concentration 0 are arranged in a line, and 7 concentrations of the agonist plus a concentration 0 are arranged in a column. This represents 88 measurement points for 1 product and 1 receptor. The remaining 8 wells are used for repeatability controls.

In each well, the cells are in contact with a concentration of the test product and a concentration of the reference agonist, 2-(4-{2-[3-(2,4-difluorophenyl)-1-heptylureido]ethyl}phenylsulfanyl)-2-methylpropionic acid for PPARα, {2-methyl-4-[4-methyl-2-(4-trifluoromethylphenyl)thiazol-5-ylmethylsulfanyl]phenoxy}acetic acid for PPARδ and 5-{4-[2-(methylpyrid-2-ylamino)ethoxy]benzyl}thiazolidine-2,4-dione for PPARγ. Measurements are also taken for total agonist controls with the same products.

The HeLN cell lines used are stable transfectants containing the plasmids ERE-βGlob-Luc-SV-Neo (reporter gene) and PPAR (α, δ, γ) Gal-hPPAR. These cells are inoculated into 96-well plates at a rate of 10 000 cells per well in 100 µl of DMEM medium without phenol red and supplemented with 10% of defatted calf serum. The plates are then incubated at 37°C and 7% CO₂ for 16 hours.

The various dilutions of the test products and of the reference ligand are added at a rate of 5 µl per well. The plates are then incubated for 18 hours at 37°C and 7% CO₂. The culture medium is removed by turning over and 100 µl of a 1:1 PBS/luciferin mixture are added to each well. After 5 minutes, the plates are read by the luminescence detector.

These cross curves make it possible to determine the AC50 values (concentration at which 50% activation is observed) of the reference ligand at various concentrations of test product. These AC50 values are used to calculate the Schild regression by plotting a straight line corresponding to the Schild equation (*"quantitation in receptor pharmacology" Terry P. Kenakin, Receptors and Channels, 2001, 7, 371-385*), which allows the Kd app values (in nM) to be obtained.

Transactivation results:

| Compounds | PPAR alpha Kd app (nM) | PPARs delta Kd app (in nM) | PPAR gamma Kd app (in nM) |
|---|---|---|---|
| Reference 1 : 2-(4-{2-[3-(2,4-Difluorophenyl)-1-heptylureido]-ethyl}phenylsulfanyl)-2-methyl-propionic acid | 200 | n.a. | n.a |
| Reference 2 : {2-Methyl-4-[4-methyl-2-(4-trifluoromethylphenyl-thiazol-5-ylmethysulfanyl]phen-oxy}acetic acid | n.a. | 10 | n.a |
| Reference 3 : 5-{4-[2-(Methylpyrid-2-ylamino)ethoxy]benzyl}-thiazolidine-2,4-dione | n.a. | n.a | 30 |
| Example 2 | n.a. | 500 | 2 |
| Example 3 | 2000 | 2000 | 4 |
| Example 13 | 9999 | 2000 | 2 |
| Example 14 | 500 | 500 | 2 |
| Example 15 | 2000 | 2000 | 2 |
| Example 16 | 1000 | n.a. | 1 |
| Example 17 | 500 | n.a. | 1 |
| Example 18 | 2000 | 250 | 4 |
| Example 24 | 500 | 2000 | 4 |
| Example 25 | 500 | 120 | 0.25 |
| Example 26 | 250 | 1000 | 0.25 |
| Example 33 | n.a. | 1000 | 4 |
| Example 34 | 500 | 8 | 2 |
| Example 35 | nd | 1000 | 1 |
| Example 37 | 250 | 2000 | 2 |
| Example 38 | 250 | n.a. | 1 |
| Example 40 | 8000 | n.a. | 1 |
| Example 41 | 60 | 2000 | 0.5 |
| Example 42 | 4000 | n.a. | 1 |
| Example 43 | 2000 | n.a. | 2 |
| Example 44 | 1000 | n.a. | 1 |
| Example 45 | 120 | 8000 | 0.25 |
| Example 46 | 30 | 8000 | 0.25 |
| Example 47 | 1000 | n.a. | 2 |
| Example 48 | 2000 | 8000 | 2 |
| Example 49 | 2000 | n.a. | 2 |

| | | | |
|---|---|---|---|
| n.a means not active ; nd means not determined | | | |

These results show the affinity of the compounds for the PPAR receptors and more particularly the specificity of the affinity of the compounds of the invention for the PPARγ subtype, compared with the affinity of the compounds for the PPARα subtype or for the PPARδ subtype.

### EXAMPLE 64 - RAT SEBOCYTE CULTURE TESTS

Sebocytes in culture obtained from rat preputial gland constitute a pertinent model for evaluating sebaceous function modulatory compounds as described by Rosenfield et al. (Mechanisms of androgen induction of sebocyte differentiation. Rosenfield R.L., Deplewski D., Kentsis A., Ciletti N., Dermatology. 1998;196(1):43-6). This model has especially been used to characterize PPAR compounds with potential in dermatology (Rat preputial sebocyte differentiation involves peroxisome proliferator-activated receptors. Rosenfield R.L., Kentsis A., Deplewski D., Ciletti N., J. Invest. Dermatol. 1999 Feb;112(2):226-32). These sebocytes have functional similarities close to those of human skin sebocytes (response to androgens and to antiandrogens, accumulation of specific sebum lipids, expression of specific markers of the sebaceous gland). The data resulting from this test are considered as pertinent of potential activity on the human sebaceous gland.

### Protocol for culturing sebocytes obtained from rat preputial glands :

The preputial glands are obtained from 10-week-old male rats. They are extracted under anaesthesia with isoflurane, before euthanasing the animals. The glands are then digested enzymatically with trypsin (0.25% trypsin + collagenase H 1.5 mg/ml/gland). The cells are cultured for 24 hours in 24-well plates (37°C, 5% CO₂) in DMEM culture medium supplemented with various factors, especially foetal calf serum (10%), glutamine (1%), a cocktail of antibiotics and antimycotic agents and insulin (5 µg/ml). The cells are then placed in Cellgro medium supplemented with various factors (Mediatech^{®}) and the test compounds are then added in response doses (duplicate). After incubation for 5 days, 10 µl of radiolabelled (¹⁴C) acetate are added to the culture medium (0.2 µCi/µl, Amersham). The cells are incubated for 24 hours and then washed and recovered after digestion with trypsin. The radiolabelled lipids are then extracted using a methanol/dichloromethane mixture (1/2) and placed on a silica TLC plate (Merck^{®}) using a loading robot. The lipids are then separated and quantified after revelation using a Phosphorimager (analysis by image analysis software). For each compound, the AC50 (concentration for which 50% of the effect of the compound is obtained) is determined. The results are expressed as nanomolar concentration for the following specific sebaceous gland lipids : Triglycerides and/or Cholesterol esters.

### Results on sebocytes :

| | Triglycerides |
|---|---|
| Compounds | AC50 (nM) |
| Example 26 | 0.005 |
| Example 38 | 0.006 |
| Example 44 | 0.008 |
| Example 41 | 0.008 |
| Example 16 | 0.008 |

### EXAMPLE 65 - COMPOSITIONS

Various concrete formulations based on the compounds according to the invention are illustrated in this example.

### A- ORAL ROUTE

| (a) 0.2 g tablet | |
|---|---|
| - Compound of Example 1 | 0.001 g |
| - Starch | 0.114 g |
| - Dicalcium phosphate | 0.020 g |
| - Silica | 0.020 g |
| - Lactose | 0.030 g |
| - Talc | 0.010 g |
| - Magnesium stearate | 0.005 g |
| (b) Drinkable suspension in 5 ml ampules | |
| - Compound of Example 5 | 0.001 g |
| - Glycerol | 0.500 g |
| - 70% sorbitol | 0.500 g |
| - Sodium saccharinate | 0.010 g |
| - Methyl parahydroxybenzoate | 0.040 g |
| - Flavouring | qs |
| - Purified water qs | 5 ml |
| (c) 0.2 g tablet | |
| - Compound of Example 2 | 0.050 g |
| - Lactose monohydrate | 0.132 g |
| - Crosspovidone | 0.007 g |
| - Povidone | 0.005 g |
| - Aerosil 200 | 0.004 g |
| - Magnesium stearate | 0.002g |
| (d) Drinkable suspension in 10 ml ampules | |
| - Compound of Example 4 | 0.200 g |
| - Glycerol | 1.000 g |
| - 70% sorbitol | 1.000 g |
| - Sodium saccharinate | 0.010 g |
| - Methyl parahydroxybenzoate | 0.080 g |
| - Flavouring | qs |
| - Purified water qs | 10 ml |

### B- TOPICAL ROUTE

| (a) Ointment | |
|---|---|
| - Compound of Example 6 | 0.020 g |
| - Isopropyl myristate | 81.700 g |
| - Fluid petroleum jelly oil | 9.100 g |
| - Silica ("Aerosil 200" sold by Degussa) | 9.180 g |
| | |

| (b) Ointment | |
|---|---|
| - Compound of Example 2 | 0.300 g |
| - White petroleum jelly codex qs | 100 g |
| | |

| (c) Nonionic water-in-oil cream | |
|---|---|
| - Compound of Example 1 | 0.100 g |
| - Mixture of emulsifying lanolin alcohols, waxes and oils | |
| ("Anhydrous Eucerin" sold by BDF) | 39.900 g |
| - Methyl parahydroxybenzoate | 0.075 g |
| - Propyl parahydroxybenzoate | 0.075 g |
| - Sterile demineralized water qs | 100 g |
| | |

| (d) Lotion | |
|---|---|
| - Compound of Example 3 | 0.100 g |
| - Polyethylene glycol (PEG 400) | 69.900 g |
| - 95% ethanol | 30.000 g |
| | |

| (e) Hydrophobic ointment | |
|---|---|
| - Compound of Example 5 | 0.300 g |
| - Isopropyl myristate | 36.400 g |
| - Silicone oil ("Rhodorsil 47 V 300" sold by | |
| Rhône-Poulenc) | 36.400 g |
| - Beeswax | 13.600 g |
| - Silicone oil ("Abil 300.000 cSt" sold by Goldschmidt) qs | 100 g |
| | |

| (f) Nonionic oil-in-water cream | |
|---|---|
| - Compound of Example 2 | 1.000 g |
| - Cetyl alcohol | 4.000 g |
| - Glyceryl monostearate | 2.500 g |
| - PEG 50 stearate | 2.500 g |
| - Shea butter | 9.200 g |
| - Propylene glycol | 2.000 g |
| - Methyl parahydroxybenzoate | 0.075 g |
| - Propyl parahydroxybenzoate | 0.075 g |
| - Sterile demineralized water qs | 100 g |

## Claims

1. Compounds **characterized in that** they correspond to formula (I) below: 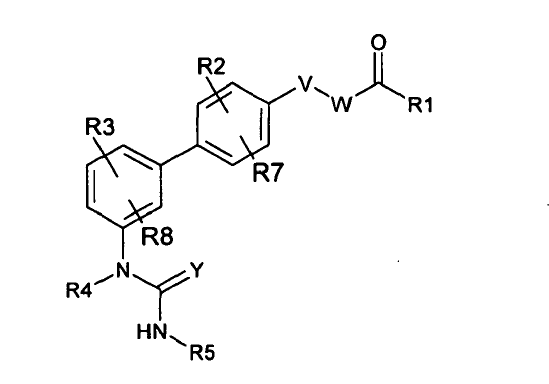 in which:
- **R1** represents a hydroxyl radical, a radical -OR6 or a hydroxylamine radical; R6 being defined below,
- **R2** and **R3,** which may be identical or different, represent a hydrogen atom, a halogen atom, an alkyl radical of 1 to 12 carbon atoms, a hydroxyl radical, an alkoxy radical, a polyether radical, an aralkyl radical, an aryl radical, an amino radical that may be substituted with one or two radicals, which may be identical or different, chosen from an alkyl radical of 1 to 12 carbon atoms and an aralkyl radical;
- **R4** represents a hydrogen atom or a lower alkyl radical containing from 1 to 4 carbon atoms;
- **R5** represents an alkyl radical containing from 1 to 12 carbon atoms, an aryl radical, an aralkyl radical, a heteroaryl radical or a heterocyclic radical;
- **R6** represents an alkyl, aryl or aralkyl radical;
- **R7** and **R8,** which may be identical or different, represent a hydrogen atom, a halogen atom, an alkyl radical of 1 to 12 carbon atoms, a hydroxyl radical, an alkoxy radical, a polyether radical, an aralkyl radical, an aryl radical, an amino radical that may be substituted with one or two radicals, which may be identical or different, chosen from an alkyl radical of 1 to 12 carbon atoms or an aralkyl radical;
- **Y** represents an oxygen or sulfur atom;
- **V-W** represents a sequence CH₂-CH₂ or CH=CH
wherein:
- "alkyl radical of 1 to 12 carbon atoms" means a linear, branched or cyclic, saturated or unsaturated carbon-based chain that may be interrupted with a hetero atom and that may be substituted with one or more radicals chosen from a halogen atom, a hydroxyl radical, an alkoxy radical and a heterocyclic radical.
- "aryl radical" means a phenyl, biphenyl, cinnamyl or naphthyl radical that may be substituted with a halogen atom, a CF3 radical, an alkyl radical of 1 to 12 carbon atoms, an alkoxy radical, a nitro function, a polyether radical, an aryl radical, a benzoyl radical, an alkyl ester group, a carboxylic acid, a hydroxyl radical optionally protected with an acetyl or benzoyl group or an amino radical optionally protected with an acetyl or benzoyl group or optionally substituted with at least one alkyl containing from 1 to 12 carbon atoms, an aralkoxy radical, a phenoxy radical or an amide radical H2NCO.
- "aralkyl radical" means an alkyl radical of 1 to 12 carbon atoms substituted with an aryl radical or with a heteroaryl radical.
- "alkoxy radical" means an oxygen atom substituted with an alkyl radical of 1 to 12 carbon atoms
- "aralkoxy radical" means an oxygen atom substituted with an aralkyl radical.
- "polyether radical" means a radical containing from 1 to 7 carbon atoms interrupted with at least one oxygen atoms.
- "heteroaryl radical" means an aryl radical interrupted with one or more hetero atoms, optionally substituted with at least one halogen, an alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms,
and also the salts of the compounds of formula (I).

2. Compounds according to Claim 1, **characterized in that** they are in the form of salts of an alkali metal or alkaline-earth metal or salts of an organic amine.

3. Compounds according to Claim 1, **characterized in that**, when they contain an amine function, they are in the form of mineral acid salts or organic acid salts.

4. Compounds according to one of Claims 1, 2 and 3, **characterized in that** the alkyl radicals containing from 1 to 12 carbon atoms are chosen from methyl, ethyl, propyl, isopropyl, butyl, tert-butyl, isoamyl, amyl, hexyl, heptyl, octyl, decyl, cyclohexyl, methylcyclohexyl, methylcyclobutyl, methylcyclopentyl and methylcyclohexyl radicals.

5. Compounds according to any one of Claims 1 to 4, **characterized in that** the lower alkyl radicals containing from 1 to 4 carbon atoms are chosen from methyl, ethyl, propyl, methylcyclopropyl, isopropyl, tert-butyl and n-butyl radicals.

6. Compounds according to any one of Claims 1 to 5, **characterized in that** the aryl radicals are chosen from phenyl, biphenyl, cinnamyl and naphthyl radicals that may be substituted with a halogen atom, a CF₃ radical, an alkyl radical of 1 to 12 carbon atoms, an alkoxy radical, a nitro function, a polyether radical, an aryl radical, a benzoyl radical, an alkyl ester group, a carboxylic acid, a hydroxyl radical optionally protected with an acetyl or benzoyl group or an amino radical optionally protected with an acetyl or benzoyl group or optionally substituted with at least one alkyl containing from 1 to 12 carbon atoms, an aralkoxy radical, a phenoxy radical or an amide radical H2NCO.

7. Compounds according to any one of Claims 1 to 6, **characterized in that** the aralkyl radicals are alkyl radicals of 1 to 12 carbon atoms substituted with an aryl radical or with a heteroaryl radical.

8. Compounds according to any one of Claims 1 to 7, **characterized in that** the halogen atom is chosen from fluorine, chlorine, bromine and iodine atoms.

9. Compounds according to any one of Claims 1 to 8, **characterized in that** the alkoxy radicals are chosen from methoxy, ethoxy, isopropyloxy, n-propyloxy, tert-butoxy, n-butoxy, n-pentyloxy, n-hexyloxy, cyclopropylmethoxy, cyclobutylmethoxy, cyclopentylmethoxy and cyclohexylmethoxy radicals.

10. Compounds according to any one of Claims 1 to 9, **characterized in that** the polyether radicals are chosen from radicals containing from 1 to 7 carbon atoms interrupted with at least one oxygen atom, and preferably radicals such as methoxyethoxy, ethoxyethoxy or methoxyethoxyethoxy radicals.

11. Compounds according to any one of Claims 1 to 10, **characterized in that** the heteroaryl radical is chosen from the group consisting of a pyridyl, furyl, thienyl, isoxazolyl, oxadiazolyl, oxazolyl, isothiazolyl, quinazolinyl, benzothiadiazolyl, benzimidazole, indolyl or benzofuran radical, optionally substituted with at least one halogen, an alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms.

12. Compounds according to any one of Claims 1 to 11, **characterized in that** the heterocyclic radical is chosen from a morpholino, pyrrolidino, piperidino, piperazino, 2-oxopiperid-1-yl or 2-oxopyrrolidin-1-yl radical, optionally substituted with at least one alkyl radical containing from 1 to 12 carbon atoms, an alkoxy radical, an aryl radical, a nitro function, a polyether radical, a heteroaryl radical, a benzoyl radical, an alkyl ester group, a carboxylic acid, a hydroxyl optionally protected with an acetyl or benzoyl group or an amino radical optionally protected with an acetyl or benzoyl group or optionally substituted with at least one alkyl containing from 1 to 12 carbon atoms.

13. Compounds according to Claim 1, **characterized in that** they are taken, alone or as mixtures, from the group consisting of:
- (E)-3-[3'-(3-heptyl-1-methylureido)biphenyl-4-yl]acrylic acid
- 3-[3'-(3-heptyl-1-methylureido)biphenyl-4-yl]propanoic acid
- 3-[3-fluoro-3'-(3-heptyl-1-methylureido)biphenyl-4-yl]propanoic acid
- 3-(3'-{3-[2-(4-fluorophenyl)ethyl]-1-methylureido}biphenyl-4-yl)propanoic acid
- 3-[3'-(1-methyl-3-pentylureido)biphenyl-4-yl]propanoic acid
- 3-[3'-(3-heptyl-1-methylureido)biphenyl-4-yl]-N-hydroxypropionamide
- 3-[3'-(1-methyl-3-naphthalen-2-ylureido)biphenyl-4-yl]propanoic acid
- 3-[3'-(3-heptyl-1-methylthioureido)biphenyl-4-yl]propanoic acid
- (E)-3-[2-fluoro-3'-(3-heptyl-1-methylureido)biphenyl-4-yl]acrylic acid
- 3-[2-fluoro-3'-(3-heptyl-1-methylureido)biphenyl-4-yl]propanoic acid
- 3-[3'-(3-hexyl-1-methylureido)biphenyl-4-yl]propanoic acid
- 3-[3'-(3-octyl-1-methylureido)biphenyl-4-yl]propanoic acid
- 3-{3'-[3-(4-benzyloxyphenyl)-1-methylureido]biphenyl-4-yl}propanoic acid
- 3-{3'-[3-(4-butylphenyl)-1-methylureido]biphenyl-4-yl}propanoic acid
- 3-[3'-(3-heptyl-1-methylureido)-2-(2-methoxyethoxy)biphenyl-4-yl]propanoic acid
- 3-[3'-(3-heptyl-1-methylureido)-2-(3-methylbutoxy)biphenyl-4-yl]propanoic acid
- 3-[2-(3-chloropropoxy)-3'-(3-heptyl-1-methylureido)biphenyl-4-yl]propanoic acid
- 3-[3'-(3-heptyl-1-methylureido)-2-methoxybiphenyl-4-yl]propanoic acid
- 3-[3'-(3-heptyl-1-methylureido)-2-methylbiphenyl-4-yl]propanoic acid
- 3-[3'-[1-methyl-3-(3-phenylpropyl)ureido]biphenyl-4-yl]propanoic acid
- (E)-3-[5'-(3-heptyl-1-methylureido)-2'-methylbiphenyl-4-yl]acrylic acid
- 3-[5'-(3-heptyl-1-methylureido)-2'-methylbiphenyl-4-yl]propanoic acid
- (E)-3-[3-fluoro-3'-(3-heptyl-1-methylureido)biphenyl-4-yl]acrylic acid
- (E)-3-[2-benzyloxy-3'-(3-heptyl-1-methylureido)biphenyl-4-yl]acrylic acid
- 3-[3'-(3-heptyl-1-methylureido)-2-propoxybiphenyl-4-yl]propanoic acid
- 3-[2-butoxy-3'-(3-heptyl-1-methylureido)biphenyl-4-yl]propanoic acid
- (E)-3-[3'-(1-ethyl-3-heptylureido)biphenyl-4-yl]acrylic acid
- (E)-3-[3'-(3-heptyl-1-propylureido)biphenyl-4-yl]acrylic acid
- (E)-3-[3,5-difluoro-3'-(3-heptyl-1-methylureido)biphenyl-4-yl]acrylic acid
- 3-[3'-(1-ethyl-3-heptylureido)biphenyl-4-yl]propanoic acid
- 3-[3'-(3-heptyl-1-propylureido)biphenyl-4-yl]propanoic acid
- 3-[3,5-difluoro-3'-(3-heptyl-1-methylureido)biphenyl-4-yl]propanoic acid
- 3-{3'-[3-(4-butoxyphenyl)-1-methylureido]biphenyl-4-yl}propanoic acid
- 3-{3'-[3-(4-butoxyphenyl)-1-ethylureido]biphenyl-4-yl}propanoic acid
- 3-[2-cyclopropylmethoxy-3'-(3-heptyl-1-methylureido)biphenyl-4-yl]propanoic acid
- 3-[2-ethoxy-3'-(3-heptyl-1-methylureido)biphenyl-4-yl]propanoic acid
- 3-[3'-(3-heptyl-1-methylureido)-2-(3,3,3-trifluoropropoxy)biphenyl-4-yl]propanoic acid
- 3-[3'-(3-heptyl-1-methylureido)-2-(4,4,4-trifluorobutoxy)biphenyl-4-yl]propanoic acid
- 3-[3'-(3-heptyl-1-methylureido)-2-(3-hydroxypropoxy)biphenyl-4-yl]propanoic acid
- 3-[3'-(3-heptyl-1-methylureido)-2-(4-hydroxybutoxy)biphenyl-4-yl]propanoic acid
- 3-[2-butoxy-3'-(1-methyl-3-pentylureido)biphenyl-4-yl]propanoic acid
- 3-[2-benzyloxy-3'-(3-heptyl-1-methylureido)biphenyl-4-yl]propanoic acid
- 3-[2-(3-fluorobenzyloxy)-3'-(3-heptyl-1-methylureido)biphenyl-4-yl]propanoic acid
- 3-[2-(4-fluorobenzyloxy)-3'-(3-heptyl-1-methylureido)biphenyl-4-yl]propanoic acid
- 3-[3'-(3-heptyl-1-methylureido)-2-pentyloxybiphenyl-4-yl]propanoic acid
- 3-[3'-(1-methyl-3-pentylureido)-2-pentyloxybiphenyl-4-yl]propanoic acid
- 3-[2-(2-ethoxyethoxy)-3'-(3-heptyl-1-methylureido)biphenyl-4-yl]propanoic acid
- 3-[2-butoxy-3'-(1-methyl-3-phenylureido)biphenyl-4-yl]propanoic acid
- 3-[2-(2-ethoxyethoxy)-3'-(1-methyl-3-pentylureido)biphenyl-4-yl]propanoic acid
- 3-[2-(2-diethylaminoethoxy)-3'-(3-heptyl-1-methylureido)biphenyl-4-yl]propanoic acid hydrochloride
- 3-[3'-(3-heptyl-1-methylureido)-2-(2-morpholin-4-ylethoxy)biphenyl-4-yl]propanoic acid
- 3-[2-amino-3'-(1-methyl-3-pentylureido)biphenyl-4-yl]propanoic acid
- 3-[2-butylamino-3'-(1-methyl-3-pentylureido)biphenyl-4-yl]propanoic acid hydrochloride
- 3-[2-benzylamino-3'-(1-methyl-3-pentylureido)biphenyl-4-yl]propanoic acid hydrochloride
- 3-[2-diethylamino-3'-(1-methyl-3-pentylureido)biphenyl-4-yl]propanoic acid hydrochloride
- 3-[3'-(1-methyl-3-pentylureido)-2-propylaminobiphenyl-4-yl]propanoic acid hydrochloride
- 3-[2-(4-fluorobenzylamino)-3'-(1-methyl-3-pentylureido)biphenyl-4-yl]propanoic acid hydrochloride
- 3-[2-butylamino-3'-(3-heptyl-1-methylureido)biphenyl-4-yl]propanoic acid hydrochloride
- 3-[2-benzylamino-3'-(3-heptyl-1-methylureido)biphenyl-4-yl]propanoic acid hydrochloride
- 3-[2-diethylamino-3'-(3-heptyl-1-methylureido)biphenyl-4-yl]propanoic acid hydrochloride
- 3-[3'-(3-heptyl-1-methylureido)-2-propylaminobiphenyl-4-yl]propanoic acid hydrochloride
- 3-[2-(4-fluorobenzylamino)-3'-(3-heptyl-1-methylureido)biphenyl-4-yl]propanoic acid hydrochloride
- 3-[3'-(1-methyl-3-pentylureido)biphenyl-4-yl]acrylic acid
- 3-[3'-(3-pentyl-1-propylureido)biphenyl-4-yl]acrylic acid
- 3-[4'-fluoro-3'-(1-methyl-3-pentylureido)biphenyl-4-yl]propanoic acid
- ethyl 3-[3'-(1-Methyl-3-pentylureido)biphenyl-4-yl]acrylate
- 3-[3'-(1-methyl-3-naphthalen-2-ylureido)biphenyl-4-yl]acrylic acid
- 3-{3'-[3-(4-fluorophenyl)-1-methylureido]biphenyl-4-yl}acrylic acid
- 3-(3'-{3-[2-(4-fluorophenyl)ethyl]-1-methylureido}biphenyl-4-yl)acrylic acid
- 3-[3'-(3-benzyl-1-methylureido)biphenyl-4-yl]propanoic acid
- 3-[3'-(3-cyclopropylmethyl-1-methylureido)biphenyl-4-yl]propanoic acid
- 3-[3'-(3-cyclohexyl-1-methylureido)biphenyl-4-yl]propanoic acid
- 3-[3'-(3-cyclohexyl-1-methylureido)biphenyl-4-yl]acrylic acid
- 3-[3'-(3-cyclopropylmethyl-1-methylureido)biphenyl-4-yl]acrylic acid
- ethyl 4-{3-[4'-(2-carboxyethyl)biphenyl-3-yl]-3-methylureido}piperidine-1-carboxylate
- 3-[3'-(1-methyl-3-pyrid-3-ylureido)biphenyl-4-yl]acrylic acid
- 3-[3'-(1-methyl-3-pyrid-3-ylureido)biphenyl-4-yl]propanoic acid
- 3-{3'-[3-(6-methoxypyrid-3-yl)-1-methylureido]biphenyl-4-yl}acrylic acid
- 3-[3'-(1-methyl-3-propylureido)biphenyl-4-yl]acrylic acid
- 3-[3'-(3-hexyl-1-methylthioureido)biphenyl-4-yl]propanoic acid
- 3-[3'-(3-hexyl-1-methylthioureido)biphenyl-4-yl]acrylic acid
- methyl 3-[3'-(3-Heptyl-1-methylthioureido)biphenyl-4-yl]acrylate
- 3-[2-methyl-3'-(1-methyl-3-pentylureido)biphenyl-4-yl]propanoic acid
- 3-[3-hydroxy-3'-(1-methyl-3-pentylureido)biphenyl-4-yl]propanoic acid
- 3-[3-methoxymethoxy-3'-(1-methyl-3-pentylureido)biphenyl-4-yl]propanoic acid
- 3-[3'-(1-methyl-3-pentylureido)-2-trifluoromethylbiphenyl-4-yl]propanoic acid
- 3-[3'-(3-heptyl-1-methylureido)-3-methoxybiphenyl-4-yl]propanoic acid
- 3-{3'-[1-methyl-3-(4-propylphenyl)ureido]biphenyl-4-yl}propanoic acid
- 3-{3'-[1-methyl-3-(4-propylphenyl)ureido]biphenyl-4-yl}acrylic acid
- phenyl 3-{3'-[1-methyl-3-(4-propylphenyl)ureido]biphenyl-4-yl}acrylate
- benzyl 3-{3'-[1-methyl-3-(4-propylphenyl)ureido]biphenyl-4-yl}acrylate
- 3-[3'-(3-pentylureido)biphenyl-4-yl]acrylic acid
- N-hydroxy-3-{3'-[1-methyl-3-(3-phenylpropyl)ureido]biphenyl-4-yl}propionamide
- 3-[3'-(3-heptyl-1-methylureido)-2-hydroxybiphenyl-4-yl]propanoic acid
- 3-[2-cyclohexylmethoxy-3'-(3-heptyl-1-methylureido)biphenyl-4-yl]propionic acid
- 3-[2-cyclopentylmethoxy-3'-(3-heptyl-1-methylureido)biphenyl-4-yl]propionic acid
- 3-[2-cyclobutylmethoxy-3'-(3-heptyl-1-methylureido)biphenyl-4-yl]-N-hydroxypropionamide
- 3-[3'-(3-heptyl-1-methylureido)-2-(3-trifluoromethylbenzyloxy)biphenyl-4-yl]propanoic acid
- 3-[3'-(3-heptyl-1-methylureido)-2-(4-trifluoromethylbenzyloxy)biphenyl-4-yl]propanoic acid
- 3-[2-(3-carbamoylbenzyloxy)-3'-(3-heptyl-1-methylureido)biphenyl-4-yl]propanoic acid
- 3-[3'-(3-heptyl-1-methylureido)-2-(2-piperazin-1-ylethoxy)biphenyl-4-yl]propanoic acid
- 3-[3'-(3-heptyl-1-methylureido)-2-(2-pyrrolidin-1-ylethoxy)biphenyl-4-yl]propanoic acid
- 3-(2-(3-methoxybenzyloxy)-3'-{3-[2-(3-methoxyphenyl)ethyl]-1-methylureido}biphenyl-4-yl)propanoic acid
- 3-(2-(4-tert-butylbenzyloxy)-3'-{1-methyl-3-[2-(3-phenoxyphenyl)ethyl]-ureido}biphenyl-4-yl)propanoic acid
- 3-{2-(3,5-dimethoxybenzyloxy)-3'-[1-methyl-3-(3-phenoxyphenyl)ureido]biphenyl-4-yl}propanoic acid
- 3-[3'-[1-methyl-3-(4-phenoxyphenyl)ureido]-2-(3-trifluoromethylbenzyloxy)biphenyl-4-yl]propanoic acid
- 3-(2-(3-isopropoxybenzyloxy)-3'-{3-[2-(4-methoxyphenyl)ethyl]-1-methylureido}biphenyl-4-yl)propanoic acid
- 3-(2'-(3-methoxybenzyloxy)-5'-{3-[2-(3-methoxyphenyl)ethyl]-1-methylureido}biphenyl-4-yl)propanoic acid
- 3-[2'-cyclohexylmethoxy-5'-(1-methyl-3-pentylureido)biphenyl-4-yl]propanoic acid
- 3-[4'-ethoxy-3'-(1-methyl-3-pentylureido)-2-propoxybiphenyl-4-yl]propanoic acid
- 3-[3,5-dimethoxy-3'-(1-methyl-3-pentylureido)biphenyl-4-yl]propanoic acid
- 3-(3,5-diethoxy-3'-{3-[2-(3-methoxyphenyl)ethyl]-1-methylureido}biphenyl-4-yl)propanoic acid
- 3-(3'-{3-[2-(4-methoxyphenyl)ethyl]-1-methylureido}-3-propoxybiphenyl-4-yl)propanoic acid
- 3-[3-cyclopropylmethoxy-3'-(1-methyl-3-pentylureido)biphenyl-4-yl]propanoic acid
- 3-[3-ethoxy-4'-fluoro-3'-(1-methyl-3-pentylureido)biphenyl-4-yl]propanoic acid
- 3-[3'-(1-methyl-3-pentylureido)-3-(4,4,4-trifluorobutoxy)biphenyl-4-yl]propanoic acid
- 3-[3-benzyloxy-3'-(1-methyl-3-pentylureido)biphenyl-4-yl]propanoic acid
- 3-[3'-{3-[2-(4-methoxyphenyl)ethyl]-1-methylureido}-3-(3-trifluoromethylbenzyloxy)biphenyl-4-yl]propanoic acid
- 3-(3'-{3-[2-(3-methoxyphenyl)ethyl]-1-methylureido}-3,5-dipropylbiphenyl-4-yl)propanoic acid
- 3-[3-(2,2-dimethylpropyl)-3'-(1-methyl-3-pentylureido)biphenyl-4-yl]propanoic acid
- 3-[3,5-dimethyl-3'-(1-methyl-3-pentylureido)biphenyl-4-yl]propanoic acid
- 3-[4"-methoxy-3-(1-methyl-3-pentylureido)[1,1';3',1"]terphenyl-4'-yl]propanoic acid
- 3-[3"-methoxy-3-(1-methyl-3-pentylureido)[1,1';2',1"]terphenyl-4'-yl]propanoic acid
- 3-(3-{3-[2-(3-methoxyphenyl)ethyl]-1-methylureido}-3"-trifluoromethyl[1,1';2',1"]terphenyl-4'-yl)propanoic acid
- 3-{3'-(3-butyl-1-methylureido)-2-[2-(3-isopropoxyphenyl)ethyl]biphenyl-4-yl}propanoic acid
- 3-{3'-(1-methyl-3-pentylureido)-2-[(pyrid-3-ylmethyl)amino]biphenyl-4-yl}propanoic acid
- 3-[3-(2-methoxyethylamino)-3'-(1-methyl-3-pentylureido)biphenyl-4-yl]propanoic acid
- methyl 3-[3,5-diethyl-3'-(1-methyl-3-pentylureido)biphenyl-4-yl]propanoate
- methyl 3-[3'-[1-methyl-3-(3-phenoxyphenyl)ureido]-2-(3-trifluoromethylbenzyloxy)-biphenyl-4-yl]propanoate
- methyl 3-[3'-[3-(2-biphenyl-4-ylethyl)-1-methylureido]-2-(3-methoxybenzyloxy)biphenyl-4-yl]propanoate
- ethyl 3-[3'-{3-[2-(3-methoxyphenyl)ethyl]-1-methylureido}-2-(4,4,4-trifluorobutoxy)-biphenyl-4-yl]propanoate.

14. Compounds according to Claim 1, 2 or 3, **characterized in that** they have at least one of the following characteristics:
- R1 represents a hydroxyl or hydroxylamine radical;
- R2 and R7 represent an alkoxy or aryloxy radical, an alkylamino radical or a polyether radical;
- R3 and R8 represent a hydrogen atom;
- R4 represents a lower alkyl radical of 1 to 4 carbon atoms;
- R5 represents an alkyl radical of 3 to 8 carbon atoms;
- Y is an oxygen atom;
- the bond V-W is CH2-CH2 or CH=CH.

15. Compounds according to Claim 14, **characterized in that** they have all of the following characteristics:
- R1 represents a hydroxyl or hydroxylamine radical;
- R2 and R7 represent an alkoxy or aryloxy radical, an alkylamino radical or a polyether radical;
- R3 and R8 represent a hydrogen atom;
- R4 represents a lower alkyl radical of 1 to 4 carbon atoms;
- R5 represents an alkyl radical of 3 to 8 carbon atoms;
- Y is an oxygen atom;
- the bond V-W is CH2-CH2 or CH=CH.

16. Cosmetic composition, **characterized in that** it comprises, in a physiologically acceptable support, at least one of the compounds as defined in any one of Claims 1 to 15.

17. Composition according to Claim 16, **characterized in that** the concentration of compound(s) according to one of Claims 1 to 15 is between 0.001% and 3% by weight relative to the total weight of the composition.

18. Cosmetic use of a composition as defined in either of Claims 16 and 17, for body or hair hygiene.

19. Compounds according to any one of Claims 1 to 15 as medicaments.

20. Use of a compound according to any one of Claims 1 to 15 in the manufacture of a composition for regulating and/or restoring skin lipid metabolism.

21. Use of a compound according to any one of Claims 1 to 15 in the manufacture of a composition for treating:
- dermatological complaints associated with a keratinization disorder relating to cell differentiation and proliferation, especially for treating common acne, comedones, polymorphs, acne rosacea, nodulocystic acne, acne conglobata, senile acne, and secondary acnes such as solar acne, medication-related acne or occupational acne;
- ichthyosis, ichthyosiform conditions, Darier's disease, palmoplantar keratoderma, leukoplakia and leukoplakiform conditions, and cutaneous or mucous (buccal) lichen;
- dermatological complaints with an inflammatory immunoallergic component, with or without cell proliferation disorder, especially cutaneous, mucous or ungual psoriasis, psoriatic rheumatism, cutaneous atopy, such as eczema, respiratory atopy, or gingival hypertrophy;
- dermal or epidermal proliferations, whether benign or malignant, and whether of viral origin or otherwise, especially common warts, flat warts and verruciform epidermodysplasia, oral or florid papillomatoses, T lymphoma;
- proliferations that may be induced by ultraviolet radiation, especially basal cell and spinal cell epithelioma;
- precancerous skin lesions, especially keratoacanthomas;
- immune dermatoses, especially lupus erythematosus;
- immune bullous diseases;
- collagen diseases, especially scleroderma;
- dermatological or general complaints with an immunological component;
- skin disorders caused by exposure to UV radiation, photoinduced or chronological ageing of the skin, actinic pigmentations and keratosis, or any pathology associated with chronological or actinic ageing, especially xerosis;
- sebaceous function disorders, especially the hyperseborrhoea of acne or simple seborrhoea;
- cicatrization disorders or stretchmarks;
- pigmentation disorders, such as hyperpigmentation, melasma, hypopigmentation or vitiligo;
- lipid metabolism complaints, such as obesity, hyperlipidaemia, or non-insulin-dependent diabetes;
- inflammatory complaints such as arthritis;
- cancerous or precancerous conditions;
- alopecia of various origins, especially alopecia caused by chemotherapy or radiation;
- disorders of the immune system, such as asthma, type I sugar diabetes, multiple sclerosis or other selective dysfunctions of the immune system; and
- complaints of the cardiovascular system, such as arteriosclerosis or hypertension.

22. Pharmaceutical composition, **characterized in that** it comprises, in a physiologically acceptable support, at least one of the compounds as defined in any one of Claims 1 to 15.

23. Composition according to Claim 22, **characterized in that** the concentration of compound(s) according to one of Claims 1 to 15 is between 0.001% and 10% by weight relative to the total weight of the composition.

24. Composition according to Claim 22, **characterized in that** the concentration of compound(s) according to one of Claims 1 to 15 is between 0.01% and 1 % by weight relative to the total weight of the composition.

## Patentansprüche

1. Verbindungen, **dadurch gekennzeichnet, dass** sie der nachstehenden Formel (I) 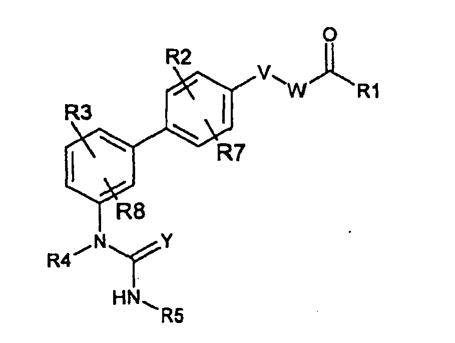 entsprechen,
worin bedeuten:
- **R1** eine Hydroxylgruppe, eine Gruppe -OR6 oder eine Hydroxylamingruppe,
wobei R6 wie unten definiert ist,
- **R2** und **R3,** die gleich oder verschieden sein können, ein Wasserstoffatom, eine Halogenatom, eine Alkylgruppe mit 1 bis 12 Kohlenstoffatomen, eine Hydroxylgruppe, eine Alkoxygruppe, eine Polyethergruppe, eine Aralkylgruppe, eine Arylgruppe, eine Aminogruppe, die mit einer oder zwei Gruppen substituiert sein kann, die gleich oder verschieden sein können und unter einer Alkylgruppe mit 1 bis 12 Kohlenstoffatomen und einer Aralkylgruppe ausgewählt sind,
- **R4** ein Wasserstoffatom oder eine niedere Alkylgruppe mit 1 bis 4 Kohlenstoffatomen,
- **R5** eine Alkylgruppe mit 1 bis 12 Kohlenstoffatomen, eine Arylgruppe, eine Aralkylgruppe, eine Heteroarylgruppe oder eine heterocyclische Gruppe,
- **R6** eine Alkylgruppe, eine Arylgruppe oder eine Aralkylgruppe,
- **R7** und **R8,** die gleich oder verschieden sein können, ein Wasserstoffatom, ein Halogenatom, eine Alkylgruppe mit 1 bis 12 Kohlenstoffatomen, eine Hydroxylgruppe, eine Alkoxygruppe, eine Polyethergruppe, eine Aralkylgruppe, eine Arylgruppe, eine Aminogruppe, die mit einer oder zwei Gruppen substituiert sein kann, die gleich oder verschieden sein können und unter einer Alkylgruppe mit 1 bis 12 Kohlenstoffatomen oder einer Aralkylgruppe ausgewählt sind,
- **Y** ein Sauerstoffatom oder ein Schwefelatom,
- **V-W** eine Gruppierung CH₂-CH₂ oder CH=CH,
wobei folgende Definitionen gelten:
- "Alkylgruppe mit 1 bis 12 Kohlenstoffatomen" bedeutet eine geradkettige, verzweigte oder cyclische, gesättigte oder ungesättigte Kohlenstoffkette, die durch ein Heteroatom unterbrochen und mit einer oder mehreren Gruppen substituiert sein kann, die unter einem Halogenatom, einer Hydroxylgruppe, einer Alkoxygruppe und einer heterocyclischen Gruppe ausgewählt sind;
- "Arylgruppe" bedeutet eine Phenylgruppe, eine Biphenylgruppe, eine Cinnamylgruppe oder eine Naphthylgruppe, die substituiert sein kann mit einem Halogenatom, einer Gruppe CF₃, einer Alkylgruppe mit 1 bis 12 Kohlenstoffatomen, einer Alkoxygruppe, einer Nitrofunktion, einer Polyethergruppe, einer Arylgruppe, einer Benzoylgruppe, einer Alkylestergruppe, einer Carboxylgruppe, einer Hydroxylgruppe, die gegebenenfalls mit einer Acetylgruppe oder einer Benzoylgruppe geschützt ist, oder einer Aminogruppe, die gegebenenfalls mit einer Acetylgruppe oder einer Benzoylgruppe geschützt oder wahlweise mit mindestens einer Alkylgruppe mit 1 bis 12 Kohlenstoffatomen, einer Aralkoxygruppe, einer Phenoxygruppe oder einer Amidgruppe H₂NCO substituiert ist;
- "Aralkylgruppe" bedeutet eine Alkylgruppe mit 1 bis 12 Kohlenstoffatomen, die mit einer Arylgruppe oder einer Heteroarylgruppe substituiert ist;
- "Alkoxygruppe" bedeutet ein Sauerstoffatom, das mit einer Alkylgruppe mit 1 bis 12 Kohlenstoffatomen substituiert ist;
- "Aralkoxygruppe" bedeutet ein Sauerstoffatom, das mit einer Aralkylgruppe substituiert ist;
- "Polyethergruppe" bedeutet eine Gruppe mit 1 bis 7 Kohlenstoffatomen, die durch mindestens ein Sauerstoffatom unterbrochen ist;
- "Heteroarylgruppe" bedeutet eine Arylgruppe, die durch ein oder mehrere Heteroatome unterbrochen ist und die wahlweise substituiert ist mit mindestens einem Halogenatom, einer Alkylgruppe mit 1 bis 12 Kohlenstoffatomen, einer Alkoxygruppe, einer Arylgruppe, einer Nitrofunktion, einer Polyethergruppe, einer Heteroarylgruppe, einer Benzoylgruppe, einer Alkylestergruppe, einer Carboxylgruppe, einer Hydroxylgruppe, die gegebenenfalls mit einer Acetylgruppe oder einer Benzoylgruppe geschützt ist, oder einer Aminogruppe, die wahlweise mit einer Acetylgruppe oder einer Benzoylgruppe geschützt ist oder wahlweise mit mindestens einer Alkylgruppe mit 1 bis 12 Kohlenstoffatomen substituiert ist,
sowie die Salze der Verbindungen der Formel (I).

2. Verbindungen nach Anspruch 1, **dadurch gekennzeichnet, dass** sie in Form von Salzen eines Alkalimetalls oder Erdalkalimetalls oder in Form von Salzen eines organischen Amins vorliegen.

3. Verbindungen nach Anspruch 1, **dadurch gekennzeichnet, dass** sie, wenn sie eine Aminfunktion enthalten, in Form von Salzen mit anorganischen Säuren oder organischen Säuren vorliegen.

4. Verbindungen nach einem der Ansprüche 1, 2 und 3, **dadurch gekennzeichnet, dass** die Alkylgruppen, die 1 bis 12 Kohlenstoffatome enthalten, ausgewählt sind unter den Gruppen Methyl, Ethyl, Propyl, Isopropyl, Butyl, tert.-Butyl, Isoamyl, Amyl, Hexyl, Heptyl, Octyl, Decyl, Cyclohexyl, Methylcyclohexyl, Methylcyclobutyl, Methylcyclopentyl und Methylcyclohexyl.

5. Verbindungen nach einem der Ansprüche 1 bis 4, **dadurch gekennzeichnet, dass** die niederen Alkylgruppen, die 1 bis 4 Kohlenstoffatome enthalten, ausgewählt sind unter den Gruppen Methyl, Ethyl, Propyl, Methylcyclopropyl, Isopropyl, tert.-Butyl und n-Butyl.

6. Verbindungen nach einem der Ansprüche 1 bis 5, **dadurch gekennzeichnet, dass** die Arylgruppen unter Phenylgruppen, Biphenylgruppen, Cinnamylgruppen und Naphthylgruppen ausgewählt sind, die substituiert sein können mit einem Halogenatom, einer Gruppe CF₃, einer Alkylgruppe mit 1 bis 12 Kohlenstoffatomen, einer Alkoxygruppe, einer Nitrofunktion, einer Polyethergruppe, einer Arylgruppe, einer Benzoylgruppe, einer Alkylestergruppe, einer Carboxylgruppe, einer Hydroxylgruppe, die gegebenenfalls mit einer Acetylgruppe oder einer Benzoylgruppe geschützt ist, oder einer Aminogruppe, die gegebenenfalls mit einer Acetylgruppe oder einer Benzoylgruppe geschützt ist oder wahlweise mit mindestens einer Alkylgruppe mit 1 bis 12 Kohlenstoffatomen substituiert ist, einer Aralkoxygruppe, einer Phenoxygruppe oder einer Amidgruppe H₂NCO.

7. Verbindungen nach einem der Ansprüche 1 bis 6, **dadurch gekennzeichnet, dass** die Aralkylgruppen Alkylgruppen mit 1 bis 12 Kohlenstoffatomen sind, die mit einer Arylgruppe oder mit einer Heteroarylgruppe substituiert sind.

8. Verbindungen nach einem der Ansprüche 1 bis 7, **dadurch gekennzeichnet, dass** das Halogenatom unter den Atomen Fluor, Chlor, Brom und Iod ausgewählt ist.

9. Verbindungen nach einem der Ansprüche 1 bis 8, **dadurch gekennzeichnet, dass** die Alkoxygruppen ausgewählt sind unter den Gruppen Methoxy, Ethoxy, Isopropyloxy, n-Propyloxy, tert.-Butoxy, n-Butoxy, n-Pentyloxy, n-Hexyloxy, Cyclopropylmethoxy, Cyclobutylmethoxy, Cyclopentylmethoxy und Cyclohexylmethoxy.

10. Verbindungen nach einem der Ansprüche 1 bis 9, **dadurch gekennzeichnet, dass** die Polyethergruppen unter Gruppen ausgewählt sind, die 1 bis 7 Kohlenstoffatome enthalten, die durch mindestens ein Sauerstoffatom unterbrochen sind, und vorzugsweise Gruppen wie Methoxyethoxy, Ethoxyethoxy oder Methoxyethoxyethoxy bedeuten.

11. Verbindungen nach einem der Ansprüche 1 bis 10, **dadurch gekennzeichnet, dass** die Heteroarylgruppe aus der Gruppe ausgewählt ist, die besteht aus den Gruppen Pyridyl, Furyl, Thienyl, Isoxazolyl, Oxadiazolyl, Oxazolyl, Isothiazolyl, Chinazolinyl, Benzothiadiazolyl, Benzimidazol, Indolyl oder Benzofuran, die wahlweise substituiert sind mit mindestens einem Halogen, einer Alkylgruppe mit 1 bis 12 Kohlenstoffatomen, einer Alkoxygruppe, einer Arylgruppe, einer Nitrofunktion, einer Polyethergruppe, einer Heteroarylgruppe, einer Benzoylgruppe, einer Alkylestergruppe, einer Carboxylgruppe, einer Hydroxylgruppe, die gegebenenfalls mit einer Acetylgruppe oder einer Benzoylgruppe geschützt ist, oder einer Aminogruppe, die gegebenenfalls mit einer Acetylgruppe oder einer Benzoylgruppe geschützt oder wahlweise mit mindestens einer Alkylgruppe mit 1 bis 12 Kohlenstoffatomen substituiert ist.

12. Verbindungen nach einem der Ansprüche 1 bis 11, **dadurch gekennzeichnet, dass** die heterocyclische Gruppe ausgewählt ist unter den Gruppen Morpholino, Pyrrolidino, Piperidino, Piperazino, 2-Oxopiperid-1-yl oder 2-Oxopyrrolidin-1-yl, die wahlweise substituiert sind mit mindestens einer Alkylgruppe mit 1 bis 12 Kohlenstoffatomen, einer Alkoxygruppe, einer Arylgruppe, einer Nitrofunktion, einer Polyethergruppe, einer Heteroarylgruppe, einer Benzoylgruppe, einer Alkylestergruppe, einer Carboxylgruppe, einer Hydroxylgruppe, die gegebenenfalls mit einer Acetylgruppe oder einer Benzoylgruppe substituiert ist, oder einer Aminogruppe, die gegebenenfalls mit einer Acetylgruppe oder einer Benzoylgruppe geschützt oder wahlweise mit mindestens einer Alkylgruppe mit 1 bis 12 Kohlenstoffatomen substituiert ist.

13. Verbindungen nach Anspruch 1, **dadurch gekennzeichnet, dass** sie allein oder in Form von Gemischen aus der Gruppe ausgewählt sind, die besteht aus:
- (E)-3-[3'-(3-Heptyl-1-methylureido)-biphenyl-4-yl]-acrylsäure,
- 3-[3'-(3-Heptyl-1-methylureido)-biphenyl-4-yl]-propionsäure
- 3-[3-Fluor-3'-(3-heptyl-1-methylureido)-biphenyl-4-yl]-propionsäure
- 3-(3'-{3-[2-(4-Fluorphenyl)-ethyl]-1-methylureido}-biphenyl-4-yl)-propionsäure
- 3-[3'-(1-Methyl-3-pentylureido)-biphenyl-4-yl]-propionsäure
- 3-[3'-(3-heptyl-1-methylureido)-biphenyl-4-yl]-N-hydroxypropionamid
- 3-[3'-(1-Methyl-3-naphthalin-2-ylureido)-biphenyl-4-yl]-propionsäure
- 3-[3'-(3-Heptyl-1-methylthioureido)-biphenyl-4-yl]-propionsäure
- (E)-3-[2-Fluor-3'-(3-heptyl-1-methylureido)-biphenyl-4-yl]-acrylsäure
- 3-[2-Fluor-3'-(3-heptyl-1-methylureido)-biphenyl-4-yl]-propionsäure
- 3-[3'-(3-Hexyl-1-methylureido)-biphenyl-4-yl]-propionsäure
- 3-[3'-(3-Octyl-1-methylureido)-biphenyl-4-yl]-propionsäure
- 3-{3'-[3-(4-Benzyloxyphenyl)-1-methylureido]-biphenyl-4-yl}-propionsäure
- 3-{3'-[3-(4-Butylphenyl)-1-methylureido]-biphenyl-4-yl}-propionsäure
- 3-[3'-(3-Heptyl-1-methylureido)-2-(2-methoxyethoxy)-biphenyl-4-yl]-propionsäure
- 3-[3'-(3-Heptyl-1-methylureido)-2-(3-methylbutoxy)-biphenyl-4-yl]-propionsäure
- 3-[2-(3-Chlorpropoxy)-3'-(3-heptyl-1-methylureido)-biphenyl-4-yl]-propionsäure
- 3-[3'-(3-Heptyl-1-methylureido)-2-methoxybiphenyl-4-yl]-propionsäure
- 3-[3'-(3-Heptyl-1-methylureido)-2-methylbiphenyl-4-yl]-propionsäure
- 3-[3'-[1-Methyl-3-(3-phenylpropyl)-ureido]-biphenyl-4-yl]-propionsäure
- (E)-3-[5'-(3-Heptyl-1-methylureido)-2'-methylbiphenyl-4-yl]-acrylsäure
- 3-[5'-(3-Heptyl-1-methylureido)-2'-methylbiphenyl-4-yl]-propionsäure
- (E)-3-[3-Fluor-3'-(3-heptyl-1-methylureido)-biphenyl-4-yl]-acrylsäure
- (E)-3-[2-Benzyloxy-3'-(3-heptyl-1-methylureido)-biphenyl-4-yl]-acrylsäure
- 3-[3'-(3-Heptyl-1-methylureido)-2-propoxybiphenyl-4-yl]-propionsäure
- 3-[2-Butoxy-3'-(3-heptyl-1-methylureido)-biphenyl-4-yl]-propionsäure
- (E)-3-[3'-(1-Ethyl-3-heptylureido)-biphenyl-4-yl]-acrylsäure
- (E)-3-[3'-(3-Heptyl-1-propylureido)-biphenyl-4-yl]-acrylsäure
- (E)-3-[3,5-Difluor-3'-(3-heptyl-1-methylureido)-biphenyl-4-yl]-acrylsäure
- 3-[3'-(1-Ethyl-3-heptylureido)-biphenyl-4-yl]-propionsäure
- 3-[3'-(3-Heptyl-1-propylureido)-biphenyl-4-yl]-propionsäure
- 3-[3,5-Difluor-3'-(3-heptyl-1-methylureido)-biphenyl-4-yl]-propionsäure
- 3-{3'-[3-(4-Butoxyphenyl)-1-methylureido]-biphenyl-4-yl}-propionsäure
- 3-{3'-[3-(4-Butoxyphenyl)-1-ethylureido]-biphenyl-4-yl}-propionsäure
- 3-[2-Cyclopropylmethoxy-3'-(3-heptyl-1-methylureido)-biphenyl-4-yl]-propionsäure
- 3-[2-Ethoxy-3'-(3-heptyl-1-methylureido)-biphenyl-4-yl]-propionsäure
- 3-[3'-(3-Heptyl-1-methylureido)-2-(3,3,3-trifluorpropoxy)-biphenyl-4-yl]-propionsäure
- 3-[3'-(3-Heptyl-1-methylureido)-2-(4,4,4-trifluorbutoxy)-biphenyl-4-yl]-propionsäure
- 3-[3'-(3-Heptyl-1-methylureido)-2-(3-hydroxypropoxy)-biphenyl-4-yl]-propionsäure
- 3-[3'-(3-Heptyl-1-methylureido)-2-(4-hydroxybutoxy)-biphenyl-4-yl]-propionsäure
- 3-[2-Butoxy-3'-(1-methyl-3-pentylureido)-biphenyl-4-yl]-propionsäure
- 3-[2-Benzyloxy-3'-(3-heptyl-1-methylureido)-biphenyl-4-yl]-propionsäure
- 3-[2-(3-Fluorbenzyloxy)-3'-(3-heptyl-1-methylureido)-biphenyl-4-yl]-propionsäure
- 3-[2-(4-Fluorbenzyloxy)-3'-(3-heptyl-1-methylureido)-biphenyl-4-yl]-propionsäure
- 3-[3'-(3-Heptyl-1-methylureido)-2-pentyloxybiphenyl-4-yl]-propionsäure
- 3-[3'-(1-Methyl-3-pentylureido)-2-pentyloxybiphenyl-4-yl]-propionsäure
- 3-[2-(2-Ethoxyethoxy)-3'-(3-heptyl-1-methylureido)-biphenyl-4-yl]-propionsäure
- 3-[2-Butoxy-3'-(1-methyl-3-phenylureido)-biphenyl-4-yl]-propionsäure
- 3-[2-(2-Ethoxyethoxy)-3'-(1-methyl-3-pentylureido)-biphenyl-4-yl]-propionsäure
- 3-[2-(2-Diethylaminoethoxy)-3'-(3-heptyl-1-methylureido)-biphenyl-4-yl]-propionsäure-hydrochlorid
- 3-[3'-(3-Heptyl-1-methylureido)-2-(2-morpholin-4-ylethoxy)-biphenyl-4-yl]-propionsäure
- 3-[2-Amino-3'-(1-methyl-3-pentylureido)-biphenyl-4-yl]-propionsäure
- 3-[2-Butylamino-3'-(1-methyl-3-pentylureido)-biphenyl-4-yl]-propionsäure-hydrochlorid
- 3-[2-Benzylamino-3'-(1-methyl-3-pentylureido)-biphenyl-4-yl]-propionsäure-hydrochlorid
- 3-[2-Diethylamino-3'-(1-methyl-3-pentylureido)-biphenyl-4-yl]-propionsäure-hydrochlorid
- 3-[3'-(1-Methyl-3-pentylureido)-2-propylaminobiphenyl-4-yl]-propionsäure-hydrochlorid
- 3-[2-(4-Fluorbenzylamino)-3'-(1-methyl-3-pentylureido)-biphenyl-4-yl]-propionsäure-hydrochlorid
- 3-[2-Butylamino-3'-(3-heptyl-1-methylureido)-biphenyl-4-yl]-propionsäure-hydrochlorid
- 3-[2-Benzylamino-3'-(3-heptyl-1-methylureido)-biphenyl-4-yl]-propionsäure-hydrochlorid
- 3-[2-Diethylamino-3'-(3-heptyl-1-methylureido)-biphenyl-4-yl]-propionsäure-hydrochlorid
- 3-[3'-(3-Heptyl-1-methylureido)-2-propylamino-biphenyl-4-yl]-propionsäure-hydrochlorid
- 3-[2-(4-Fluorbenzylamino)-3'-(3-heptyl-1-methylureido)-biphenyl-4-yl]-propionsäure-hydrochlorid
- 3-[3'-(1-Methyl-3-pentylureido)-biphenyl-4-yl]-acrylsäure
- 3-[3'-(3-Pentyl-1-propylureido)-biphenyl-4-yl]-acrylsäure
- 3-[4'-Fluor-3'-(1-methyl-3-pentylureido)-biphenyl-4-yl]-propionsäure
- Ethyl-3-[3'-(1-methyl-3-pentylureido)-biphenyl-4-yl]-acrylat
- 3-[3'-(1-Methyl-3-naphthalin-2-ylureido)-biphenyl-4-yl]-acrylsäure
- 3-{3'-[3-(4-Fluorphenyl)-1-methylureido]-biphenyl-4-yl}-acrylsäure
- 3-(3'-{3-[2-(4-Fluorphenyl)-ethyl]-1-methylureido}-biphenyl-4-yl)-acrylsäure
- 3-[3'-(3-Benzyl-1-methylureido)-biphenyl-4-yl]-propionsäure
- 3-[3'-(3-Cyclopropylmethyl-1-methylureido)-biphenyl-4-yl]-propionsäure
- 3-[3'-(3-Cyclohexyl-1-methylureido)-biphenyl-4-yl]-propionsäure
- 3-[3'-(3-Cyclohexyl-1-methylureido)-biphenyl-4-yl]-acrylsäure
- 3-[3'-(3-Cyclopropylmethyl-1-methylureido)-biphenyl-4-yl]-acrylsäure
- Ethyl-4-{3-[4'-(2-carboxyethyl)-biphenyl-3-yl]-3-methylureido}-piperidin-1-carboxylat
- 3-[3'-(1-Methyl-3-pyrid-3-ylureido)-biphenyl-4-yl]-acrylsäure
- 3-[3'-(1-Methyl-3-pyrid-3-ylureido)-biphenyl-4-yl]-propionsäure
- 3-{3'-[3-(6-Methoxypyrid-3-yl)-1-methylureido]-biphenyl-4-yl}-acrylsäure
- 3-[3'-(1-Methyl-3-propylureido)-biphenyl-4-yl]-acrylsäure
- 3-[3'-(3-Hexyl-1-methylthioureido)-biphenyl-4-yl]-propionsäure
- 3-[3'-(3-Hexyl-1-methylthioureido)-biphenyl-4-yl]-acrylsäure
- Methyl-3-[3'-(3-heptyl-1-methylthioureido)-biphenyl-4-yl]-acrylat
- 3-[2-Methyl-3'-(1-methyl-3-pentylureido)-biphenyl-4-yl]-propionsäure
- 3-[3-Hydroxy-3'-(1-methyl-3-pentylureido)-biphenyl-4-yl]-propionsäure
- 3-[3-Methoxymethoxy-3'-(1-methyl-3-pentylureido)-biphenyl-4-yl]-propionsäure
- 3-[3'-(1-Methyl-3-pentylureido)-2-trifluoromethyl-biphenyl-4-yl]-propionsäure
- 3-[3'-(3-Heptyl-1-methylureido)-3-methoxy-biphenyl-4-yl]-propionsäure
- 3-(3'-[1-Methyl-3-(4-propylphenyl)-ureido]-biphenyl-4-yl}-propionsäure
- 3-{3'-[1-Methyl-3-(4-propylphenyl)-ureido]-biphenyl-4-yl}-acrylsäure
- Phenyl-3-{3'-[1-methyl-3-(4-propylphenyl)-ureido]-biphenyl-4-yl}-acrylat
- Benzyl-3-{3'-[1-methyl-3-(4-propylphenyl)-ureido]-biphenyl-4-yl}-acrylat
- 3-[3'-(3-Pentylureido)-biphenyl-4-yl]-acrylsäure
- N-Hydroxy-3-{3'-[1-methyl-3-(3-phenylpropyl)-ureido]-biphenyl-4-yl}-propionamid
- 3-[3'-(3-Heptyl-1-methylureido)-2-hydroxybiphenyl-4-yl]-propionsäure
- 3-[2-Cyclohexylmethoxy-3'-(3-heptyl-1-methylureido)-biphenyl-4-yl]-propionsäure
- 3-[2-Cyclopentylmethoxy-3'-(3-heptyl-1-methylureido)-biphenyl-4-yl]-propionsäure
- 3-[2-Cyclobutylmethoxy-3'-(3-heptyl-1-methylureido)-biphenyl-4-yl]-N-hydroxypropionamid
- 3-[3'-(3-Heptyl-1-methylureido)-2-(3-trifluormethylbenzyloxy)-biphenyl-4-yl]-propionsäure
- 3-[3'-(3-Heptyl-1-methylureido)-2-(4-trifluormethylbenzyloxy)-biphenyl-4-yl]-propionsäure
- 3-[2-(3-Carbamoylbenzyloxy)-3'-(3-heptyl-1-methylureido)-biphenyl-4-yl]-propionsäure
- 3-[3'-(3-Heptyl-1-methylureido)-2-(2-piperazin-1-ylethoxy)-biphenyl-4-yl]-propionsäure
- 3-[3'-(3-Heptyl-1-methylureido)-2-(2-pyrrolidin-1-ylethoxy)-biphenyl-4-yl]-propionsäure
- 3-(2-(3-Methoxybenzyloxy)-3'-{3-[2-(3-methoxyphenyl)-ethyl]-1-methylureido}-biphenyl-4-yl)-propionsäure
- 3-(2-(4-tert.-Butylbenzyloxy)-3'-{1-methyl-3-[2-(3-phenoxyphenyl)-ethyl]-ureido}-biphenyl-4-yl)-propionsäure
- 3-{2-(3,5-Dimethoxybenzyloxy)-3'-[1-methyl-3-(3-phenoxyphenyl)-ureido]-biphenyl-4-yl}-propionsäure
- 3-[3'-[1-Methyl-3-(4-phenoxyphenyl)-ureido]-2-(3-trifluormethylbenzyloxy)-biphenyl-4-yl]-propionsäure
- 3-(2-(3-Isopropoxybenzyloxy)-3'-{3-[2-(4-methoxyphenyl)-ethyl]-1-methylureido}-biphenyl-4-yl)-propionsäure
- 3-(2'-(3-Methoxybenzyloxy)-5'-{3-[2-(3-methoxyphenyl)-ethyl]-1-methylureido}-biphenyl-4-yl)-propionsäure
- 3-[2'-Cyclohexylmethoxy-5'-(1-methyl-3-pentylureido)-biphenyl-4-yl]-propionsäure
- 3-[4'-Ethoxy-3'-(1-methyl-3-pentylureido)-2-propoxybiphenyl-4-yl]-propionsäure
- 3-[3,5-Dimethoxy-3'-(1-methyl-3-pentylureido)-biphenyl-4-yl]-propionsäure
- 3-(3,5-Diethoxy-3'-{3-[2-(3-methoxyphenyl)-ethyl]-1-methylureido}-biphenyl-4-yl)-propionsäure
- 3-(3'-{3-[2-(4-Methoxyphenyl)-ethyl]-1-methylureido}-3-propoxybiphenyl-4-yl)-propionsäure
- 3-[3-Cyclopropylinethoxy-3'-(1-methyl-3-pentylureido)-biphenyl-4-yl]-propionsäure
- 3-[3-Ethoxy-4'-fluor-3'-(1-methyl-3-pentylureido)-biphenyl-4-yl]-propionsäure
- 3-[3'-(1-Methyl-3-pentylureido)-3-(4,4,4-trifluorbutoxy)-biphenyl-4-yl]-propionsäure
- 3-[3-Benzyloxy-3'-(1-methyl-3-pentylureido)-biphenyl-4-yl]-propionsäure
- 3-[3'-{3-[2-(4-Methoxyphenyl)-ethyl]-1-methylureido}-3-(3-trifluormethylbenzyloxy)-biphenyl-4-yl]-propionsäure
- 3-(3'-{3-[2-(3-Methoxyphenyl)-ethyl]-1-methylureido}-3,5-dipropylbiphenyl-4-yl)-propionsäure
- 3-[3-(2,2-Dimethylpropyl)-3'-(1-methyl-3-pentylureido)-biphenyl-4-yl]-propionsäure
- 3-[3,5-Dimethyl-3'-(1-methyl-3-pentylureido)-biphenyl-4-yl]-propionsäure
- 3-[4"-Methoxy-3-(1-methyl-3-pentylureido)-[1,1'; 3',1"]terphenyl-4'-yl]-propionsäure
- 3-[3"-Methoxy-3-(1-methyl-3-pentylureido)-[1,1';2',1 "]terphenyl-4'-yl]-propionsäure
- 3-(3-{3-[2-(3-Methoxyphenyl)-ethyl]-1-methylureido}-3"-trifluormethyl-[1,1';2',1"]terphenyl-4'-yl)-propionsäure
- 3-{3'-(3-Butyl-1-methylureido)-2-[2-(3-isopropoxyphenyl)-ethyl]-biphenyl-4-yl}-propionsäure
- 3-{3'-(1-Methyl-3-pentylureido)-2-[(pyrid-3-ylmethyl)-amino]-biphenyl-4-yl}-propionsäure
- 3-[3-(2-Methoxyethylamino)-3'-(1-methyl-3-pentylureido)-biphenyl-4-yl]-propionsäure
- Methyl-3-[3,5-diethyl-3'-(1-methyl-3-pentylureido)-biphenyl-4-yl]-propionat
- Methyl-3-[3'-[1-methyl-3-(3-phenoxyphenyl)-ureido]-2-(3-trifluormethylbenzyloxy)-biphenyl-4-yl]-propionat
- Methyl-3-[3'-[3-(2-biphenyl-4-ylethyl)-1-methylureido]-2-(3-methoxybenzyloxy)-biphenyl-4-yl]-propionat
- Ethyl-3-[3'-{3-[2-(3-methoxyphenyl)-ethyl]-1-methylureido}-2-(4,4,4-trifluorobutoxy)-biphenyl-4-yl]-propionat.

14. Verbindungen nach Anspruch 1, 2 oder 3, **dadurch gekennzeichnet, dass** sie mindestens eine der folgenden Eigenschaften aufweisen:
- R1 bedeutet eine Hydroxylgruppe oder eine Hydroxylamingruppe;
- R2 und R7 bedeuten eine Alkoxygruppe oder eine Aryloxygruppe, eine Alkylaminogruppe oder eine Polyethergruppe;
- R3 und R8 bedeuten ein Wasserstoffatom;
- R4 bedeutet eine niedere Alkylgruppe mit 1 bis 4 Kohlenstoffatomen;
- R5 bedeutet eine Alkylgruppe mit 3 bis 8 Kohlenstoffatomen;
- Y ist ein Sauerstoffatom;
- die Gruppierung V-W ist CH₂-CH₂ oder CH=CH.

15. Verbindungen nach Anspruch 14, **dadurch gekennzeichnet, dass** sie sämtliche nachstehenden Eigenschaften aufweisen:
- R1 bedeutet eine Hydroxylgruppe oder eine Hydroxylamingruppe;
- R2 und R7 bedeuten eine Alkoxygruppe oder eine Aryloxygruppe, eine Alkylaminogruppe oder eine Polyethergruppe;
- R3 und R8 bedeuten ein Wasserstoffatom;
- R4 bedeutet eine niedere Alkylgruppe mit 1 bis 4 Kohlenstoffatomen;
- R5 bedeutet eine Alkylgruppe mit 3 bis 8 Kohlenstoffatomen;
- Y ist ein Sauerstoffatom;
- die Gruppierung V-W ist CH₂-CH₂ oder CH=CH.

16. Kosmetische Zusammensetzung, **dadurch gekennzeichnet, dass** sie in einem physiologisch akzeptablen Träger mindestens eine der Verbindungen enthält, die in einem der Ansprüche 1 bis 15 definiert sind.

17. Zusammensetzung nach Anspruch 16, **dadurch gekennzeichnet, dass** die Konzentration der Verbindung(en) nach einem der Ansprüche 1 bis 15 0,001 bis 3 Gew.-% beträgt, bezogen auf das Gesamtgewicht der Zusammensetzung.

18. Kosmetische Verwendung einer Zusammensetzung, wie sie in einem der Ansprüche 16 und 17 definiert ist, zur Körper- oder Haarhygiene.

19. Verbindungen nach einem der Ansprüche 1 bis 15 als Arzneimittel.

20. Verwendung einer Verbindung nach einem der Ansprüche 1 bis 15 bei der Herstellung einer Zusammensetzung zur Regulierung und/oder Wiederherstellung des Hautlipidmetabolismus.

21. Verwendung einer Verbindung nach einem der Ansprüche 1 bis 15 bei der Herstellung einer Zusammensetzung zur Behandlung von:
- dermatologischen Beschwerden, die mit einer Störung der Keratinisierung in Verbindung stehen und sich auf die Zelldifferenzierung und Zellproliferation beziehen, insbesondere zur Behandlung von gemeiner Akne, Komedonen, polymorphen Formen, Acne rosacea, nodulocystischer Akne, Acne conglobata, seniler Akne, sowie von sekundären Aknen wie Solarakne, medikationsabhängiger Akne und Berufsakne;
- Ichthyosis, ichthyosiformen Zuständen, Darier-Krankheit, palmoplantarem Keratoderma, Leukoplakie und leukoplakiformen Zuständen sowie Lichen der Haut oder der Schleimhäute (bukkal);
- dermatologischen Beschwerden mit einer entzündlichen immunoallergischen Komponente mit oder ohne Störung der Zellproliferation, insbesondere von kutaner, mukosaler oder ungualer Psoriasis, psoriatischem Rheumatismus, Hautatopien, wie Ekzemen, respiratorischer Atopie oder gingivaler Hypertrophie;
- dermalen oder epidermalen Proliferationen, und zwar benignen oder malignen Proliferationen viralen oder nichtviralen Ursprungs, insbesondere von gemeinen Warzen, Flachwarzen und verruciformen Epidermodysplasien, oralen oder floriden Papillomatosen und T-Lymphoma;
- Proliferationen, die durch Ultraviolettstrahlung induziert sein können, insbesondere von Basalzell-Epitheliomen und Spinalzell-Epitheliomen;
- präkanzerösen Hautläsionen, insbesondere Keratoakanthomen;
- Immundermatosen, insbesondere Lupus erythematodes;
- bullösen Immunerkrankungen;
- Collagen-Erkrankungen, insbesondere Skleroderma;
- dermatologischen oder allgemeinen Beschwerden mit einer immunologischen Komponente;
- Hautstörungen, die durch UV-Strahlungsexposition hervorgerufen sind, von lichtinduzierter oder altersbedingter Hautalterung, aktinischen Pigmentationen und Keratosen oder beliebigen Pathologien, die mit der altersbedingten oder aktinischen Alterung in Verbindung stehen, insbesondere von Xerosen;
- Störungen der Talgdrüsenfunktion, insbesondere der hyperseborrhöischen Akne oder einfacher Seborrhoe;
- Störungen der Narbenbildung oder von Schwangerschaftsstreifen;
- Störungen der Pigmentierung, wie Hyperpigmentation, Melasma, Hypopigmentation oder Vitiligo;
- Krankheiten des Lipidstoffwechsels, wie Fettsucht, Hyperlipidämie oder nicht-insulinabhängigem Diabetes;
- entzündlichen Krankheiten wie Arthritis;
- kanzerösen oder präkanzerösen Zuständen;
- Alopezie verschiedenen Ursprungs, insbesondere von durch Chemotherapie oder Bestrahlung hervorgerufener Alopezie;
- Störungen des Immunsystems, wie Asthma, Typ-I-Diabetes, multipler Sklerose oder anderen selektiven Dysfunktionen des Immunsystems sowie
- Erkrankungen des kardiovaskulären Systems, wie Arteriosklerose oder Hypertension.

22. Pharmazeutische Zusammensetzung, **dadurch gekennzeichnet, dass** sie in einem physiologisch akzeptablen Träger mindestens eine der in einem der Ansprüche 1 bis 15 definierten Verbindungen enthält.

23. Zusammensetzung nach Anspruch 22, **dadurch gekennzeichnet, dass** die Konzentration der Verbindung(en) nach einem der Ansprüche 1 bis 15 0,001 bis 10 Gew.-% beträgt, bezogen auf das Gesamtgewicht der Zusammensetzung.

24. Zusammensetzung nach Anspruch 22, **dadurch gekennzeichnet, dass** die Konzentration der Verbindung(en) nach einem der Ansprüche 1 bis 15 0,01 bis 1 Gew.-% beträgt, bezogen auf das Gesamtgewicht der Zusammensetzung.

## Revendications

1. Composés **caractérisés en ce qu'**ils correspondent à la formule (I) ci-dessous : 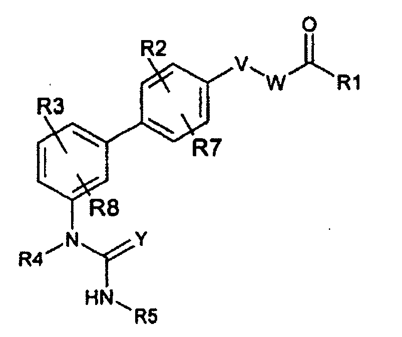 dans laquelle :
- **R1** représente un radical hydroxyle, un radical -OR6 ou un radical hydroxylamine ; R6 étant défini ci-dessous,
- **R2** et **R3,** qui peuvent être identiques ou différents, représentent un atome d'hydrogène, un atome d'halogène, un radical alkyle renfermant de 1 à 12 atomes de carbone, un radical hydroxyle, un radical alcoxy, un radical polyéther, un radical aralkyle, un radical aryle, un radical amino qui peut être substitué par un ou deux radicaux, qui peuvent être identiques ou différents, choisis parmi un radical alkyle renfermant de 1 à 12 atomes de carbone, et un radical aralkyle ;
- **R4** représente un atome d'hydrogène ou un radical alkyle inférieur renfermant de 1 à 4 atomes de carbone ;
- **R5** représente un radical alkyle renfermant de 1 à 12 atomes de carbone, un radical aryle, un radical aralkyle, un radical hétéroaryle ou un radical hétérocyclique ;
- **R6** représente un radical alkyle, un radical aryle ou un radical aralkyle ;
- **R7** et **R8,** qui peuvent être identiques ou différents, représentent un atome d'hydrogène, un atome d'halogène, un radical alkyle renfermant de 1 à 12 atomes de carbone, un radical hydroxyle, un radical alcoxy, un radical polyéther, un radical aralkyle, un radical aryle, un radical amino qui peut être substitué par un ou deux radicaux, qui peuvent être identiques ou différents, choisis parmi un radical alkyle renfermant de 1 à 12 atomes de carbone ou un radical aralkyle ;
- **Y** représente un atome d'oxygène ou un atome de soufre ;
- **-V-W** représente une séquence CH₂-CH₂ ou CH=CH
où :
- un « radical alkyle renfermant de 1 à 12 atomes de carbone » signifie une chaîne à base carbonée linéaire, ramifiée ou cyclique, saturée ou insaturée, qui peut être interrompue par un hétéroatome et qui peut être substituée par un ou plusieurs radicaux choisis parmi un atome d'halogène, un radical hydroxyle, un radical alcoxy et un radical hétérocyclique,
- un « radical aryle » signifie un radical phényle, un radical biphényle, un radical cinnamyle ou un radical naphtyle qui peut être substitué par un atome d'halogène, un radical CF₃, un radical alkyle renfermant de 1 à 12 atomes de carbone, un radical alcoxy, une fonction nitro, un radical polyéther, un radical aryle, un radical benzoyle, un groupe ester alkylique, un groupe acide carboxylique, un radical hydroxyle éventuellement protégé par un groupe acétyle ou un groupe benzoyle, ou un radical amino éventuellement protégé par un groupe acétyle ou un groupe benzoyle ou éventuellement substitué par au moins un groupe alkyle renfermant de 1 à 12 atomes de carbone, un radical aralcoxy, un radical phénoxy ou un radical amide H₂NCO,
- un « radical aralkyle » signifie un radical alkyle renfermant de 1 à 12 atomes de carbone, substitué par un radical aryle ou par un radical hétéroaryle,
- un « radical alcoxy » signifie un atome d'oxygène substitué par un radical alkyle renfermant de 1 à 12 atomes de carbone,
- un « radical aralcoxy » signifie un atome d'oxygène substitué par un radical aralkyle,
- un « radical polyéther » signifie un radical renfermant de 1 à 7 atomes de carbone, interrompu par au moins un atome d'oxygène,
- un « radical hétéroaryle » signifie un radical aryle interrompu par un ou plusieurs hétéroatomes, éventuellement substitué par au moins un atome d'halogène, un radical alkyle renfermant de 1 à 12 atomes de carbone, un radical alcoxy, un radical aryle, une fonction nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle, ou un radical amino éventuellement protégé par un groupe acétyle ou un groupe benzoyle ou éventuellement substitué par au moins un groupe alkyle renfermant de 1 à 12 atomes de carbone,
ainsi que les sels des composés de formule (I).

2. Composés selon la revendication 1, **caractérisés en ce qu'**ils sont sous la forme de sels d'un métal alcalin ou d'un métal alcalino-terreux ou de sels d'une amine organique.

3. Composés selon la revendication 1, **caractérisés en ce que,** lorsqu'ils renferment une fonction amine, ils sont sous la forme de sels d'acides minéraux ou de sels d'acides organiques.

4. Composés selon l'une des revendications 1, 2 et 3, **caractérisés en ce que** les radicaux alkyle renfermant de 1 à 12 atomes de carbone sont choisis parmi des radicaux méthyle, éthyle, propyle, isopropyle, butyle, tert-butyle, isoamyle, amyle, hexyle, heptyle, octyle, décyle, cyclohexyle, méthylcyclohexyle, méthylcyclobutyle, méthylcyclopentyle et méthylcyclohexyle.

5. Composés selon l'une quelconque des revendications 1 à 4, **caractérisés en ce que** les radicaux alkyle inférieurs renfermant de 1 à 4 atomes de carbone sont choisis parmi des radicaux méthyle, éthyle, propyle, méthylcyclopropyle, isopropyle, tert-butyle et n-butyle.

6. Composés selon l'une quelconque des revendications 1 à 5, **caractérisés en ce que** les radicaux aryle sont choisis parmi des radicaux phényle, biphényle, cinnamyle et naphtyle qui peuvent être substitués par un atome d'halogène, un radical CF₃, un radical alkyle renfermant de 1 à 12 atomes de carbone, un radical alcoxy, une fonction nitro, un radical polyéther, un radical aryle, un radical benzoyle, un groupe ester alkylique, un groupe acide carboxylique, un radical hydroxyle éventuellement protégé par un groupe acétyle ou un groupe benzoyle, ou un radical amino éventuellement protégé par un groupe acétyle ou un groupe benzoyle ou éventuellement substitué par au moins un groupe alkyle renfermant de 1 à 12 atomes de carbone, un radical aralcoxy, un radical phénoxy ou un radical amide H₂NCO.

7. Composés selon l'une quelconque des revendications 1 à 6, **caractérisés en ce que** les radicaux aralkyle sont des radicaux alkyle renfermant de 1 à 12 atomes de carbone, substitués par un radical aryle ou par un radical hétéroaryle.

8. Composés selon l'une quelconque des revendications 1 à 7, **caractérisés en ce que** l'atome d'halogène est choisi parmi des atomes de fluor, de chlore, de brome et d'iode.

9. Composés selon l'une quelconque des revendications 1 à 8, **caractérisés en ce que** les radicaux alcoxy sont choisis parmi des radicaux méthoxy, éthoxy, isopropyloxy, n-propyloxy, tert-butoxy, n-butoxy, n-pentyloxy, n-hexyloxy, cyclopropylméthoxy, cyclobutylméthoxy, cyclopentylméthoxy et cyclohexylméthoxy.

10. Composés selon l'une quelconque des revendications 1 à 9, **caractérisés en ce que** les radicaux polyéther sont choisis parmi des radicaux renfermant de 1 à 7 atomes de carbone, interrompus par au moins un atome d'oxygène, et de préférence des radicaux tels que des radicaux méthoxyéthoxy, éthoxyéthoxy ou méthoxyéthoxyéthoxy.

11. Composés selon l'une quelconque des revendications 1 à 10, **caractérisés en ce que** le radical hétéroaryle est choisi parmi le groupe constitué d'un radical pyridyle, d'un radical furyle, d'un radical thiényle, d'un radical isoxazolyle, d'un radical oxadiazolyle, d'un radical oxazolyle, d'un radical isothiazolyle, d'un radical quinazolinyle, d'un radical benzothiadiazolyle, d'un radical benzimidazole, d'un radical indolyle ou d'un radical benzofurane, éventuellement substitué par au moins un atome d'halogène, un radical alkyle renfermant de 1 à 12 atomes de carbone, un radical alcoxy, un radical aryle, une fonction nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle, ou un radical amino éventuellement protégé par un groupe acétyle ou un groupe benzoyle ou éventuellement substitué par au moins un groupe alkyle renfermant de 1 à 12 atomes de carbone.

12. Composés selon l'une quelconque des revendications 1 à 11, **caractérisés en ce que** le radical hétérocyclique est choisi parmi un radical morpholino, un radical pyrrolidino, un radical pipéridino, un radical pipérazino, un radical 2-oxopipérid-1-yle ou un radical 2-oxopyrrolidin-1-yle, éventuellement substitué par au moins un radical alkyle renfermant de 1 à 12 atomes de carbone, un radical alcoxy, un radical aryle, une fonction nitro, un radical polyéther, un radical hétéroaryle, un radical benzoyle, un groupe ester alkylique, un groupe acide carboxylique, un groupe hydroxyle éventuellement protégé par un groupe acétyle ou un groupe benzoyle, ou un radical amino éventuellement protégé par un groupe acétyle ou un groupe benzoyle ou éventuellement substitué par au moins un groupe alkyle renfermant de 1 à 12 atomes de carbone.

13. Composés selon la revendication 1, **caractérisés en ce qu'**ils sont pris, seuls ou sous forme de mélanges, parmi le groupe constitué :
- de l'acide (E)-3-[3'-(3-heptyl-1-méthyluréido)biphényl-4-yl]acrylique
- de l'acide 3-[3'-(3-heptyl-1-méthyluréido)biphényl-4-yl]propanoïque
- de l'acide 3-[3-fluoro-3'-(3-heptyl-1-méthyluréido)biphényl-4-yl]propanoïque
- de l'acide 3-(3'-{3-[2-(4-fluorophényl)éthyl]-1-méthyluréido}biphényl-4-yl)propanoïque
- de l'acide 3-[3'-(1-méthyl-3-pentyluréido)biphényl-4-yl]propanoïque
- du 3-[3'-(3-heptyl-1-méthyluréido)biphényl-4-yl]-N-hydroxypropionamide
- de l'acide 3-[3'-(1-méthyl-3-naphtalén-2-yluréido)biphényl-4-yl]propanoïque
- de l'acide 3-[3'-(3-heptyl-1-méthylthiouréido)biphényl-4-yl]propanoïque
- de l'acide (E)-3-[2-fluoro-3'-(3-heptyl-1-méthyluréido)biphényl-4-yl]acrylique
- de l'acide 3-[2-fluoro-3'-(3-heptyl-1-méthyluréido)biphényl-4-yl]propanoïque
- de l'acide 3-[3'-(3-hexyl-1-méthyluréido)biphényl-4-yl]propanoïque
- de l'acide 3-[3'-(3-octyl-1-méthyluréido)biphényl-4-yl]propanoïque
- de l'acide 3-{3'-[3-(4-benzyloxyphényl)-1-méthyluréido]biphényl-4-yl}propanoïque
- de l'acide 3-{3'-[3-(4-butylphényl)-1-méthyluréido]biphényl-4-yl}propanoïque
- de l'acide 3-[3'-(3-heptyl-1-méthyluréido)-2-(2-méthoxyéthoxy)biphényl-4-yl]propanoïque
- de l'acide 3-[3'-(3-heptyl-1-méthyluréido)-2-(3-méthylbutoxy)biphényl-4-yl]propanoïque
- de l'acide 3-[2-(3-chloropropoxy)-3'-(3-heptyl-1-méthyluréido)biphényl-4-yl]propanoïque
- de l'acide 3-[3'-(3-heptyl-1-méthyluréido)-2-méthoxybiphényl-4-yl]propanoïque
- de l'acide 3-[3'-(3-heptyl-1-méthyluréido)-2-méthylbiphényl-4-yl]propanoïque
- de l'acide 3-[3'-[1-méthyl-3-(3-phénylpropyl)uréido]biphényl-4-yl]propanoïque
- de l'acide (E)-3-[5'-(3-heptyl-1-méthyluréido)-2'-méthylbiphényl-4-yl]acrylique
- de l'acide 3-[5'-(3-heptyl-1-méthyluréido)-2'-méthylbiphényl-4-yl]propanoïque
- de l'acide (E)-3-[3-fluoro-3'-(3-heptyl-1-méthyluréido)biphényl-4-yl]acrylique
- de l'acide (E)-3-[2-benzyloxy-3'-(3-heptyl-1-méthyluréido)biphényl-4-yl]acrylique
- de l'acide 3-[3'-(3-heptyl-1-méthyluréido)-2-propoxybiphényl-4-yl]propanoïque
- de l'acide 3-[2-butoxy-3'-(3-heptyl-1-méthyluréido)biphényl-4-yl]propanoïque
- de l'acide (E)-3-[3'-(1-éthyl-3-heptyluréido)biphényl-4-yl]acrylique
- de l'acide (E)-3-[3'-(3-heptyl-1-propyluréido)biphényl-4-yl]acrylique
- de l'acide (E)-3-[3,5-difluoro-3'-(3-heptyl-1-méthyluréido)biphényl-4-yl]acrylique
- de l'acide 3-[3'-(1-éthyl-3-heptyluréido)biphényl-4-yl]propanoïque
- de l'acide 3-[3'-(3-heptyl-1-propyluréido)biphényl-4-yl]propanoïque
- de l'acide 3-[3,5-difluoro-3'-(3-heptyl-1-méthyluréido)biphényl-4-yl]propanoïque
- de l'acide 3-{3'-[3-(4-butoxyphényl)-1-méthyluréido]biphényl-4-yl}propanoïque
- de l'acide 3-{3'-[3-(4-butoxyphényl)-1-éthyluréido]biphényl-4-yl}propanoïque
- de l'acide 3-[2-cyclopropylméthoxy-3'-(3-heptyl-1-méthyluréido)biphényl-4-yl]propanoïque
- de l'acide 3-[2-éthoxy-3'-(3-heptyl-1-méthyluréido)biphényl-4-yl]propanoïque
- de l'acide 3-[3'-(3-heptyl-1-méthyluréido)-2-(3,3,3-trifluoropropoxy)biphényl-4-yl]propanoïque
- de l'acide 3-[3'-(3-heptyl-1-méthyluréido)-2-(4,4,4-trifluorobutoxy)biphényl-4-yl]propanoïque
- de l'acide 3-[3'-(3-heptyl-1-méthyluréido)-2-(3-hydroxypropoxy)biphényl-4-yl]propanoïque
- de l'acide 3-[3'-(3-heptyl-1-méthyluréido)-2-(4-hydroxybutoxy)biphényl-4-yl]propanoïque
- de l'acide 3-[2-butoxy-3'-(1-méthyl-3-pentyluréido)biphényl-4-yl]propanoïque
- de l'acide 3-[2-benzyloxy-3'-(3-heptyl-1-méthyluréido)biphényl-4-yl]propanoïque
- de l'acide 3-[2-(3-fluorobenzyloxy)-3'-(3-heptyl-1-méthyluréido)biphényl-4-yl]propanoïque
- de l'acide 3-[2-(4-fluorobenzyloxy)-3'-(3-heptyl-1-méthyluréido)biphényl-4-yl]propanoïque
- de l'acide 3-[3'-(3-heptyl-1-méthyluréido)-2-pentyloxybiphényl-4-yl]propanoïque
- de l'acide 3-[3'-(1-méthyl-3-pentyluréido)-2-pentyloxybiphényl-4-yl]propanoïque
- de l'acide 3-[2-(2-éthoxyéthoxy)-3'-(3-heptyl-1-méthyluréido)biphényl-4-yl]propanoïque
- de l'acide 3-[2-butoxy-3'-(1-méthyl-3-phényluréido)biphényl-4-yl]propanoïque
- de l'acide 3-[2-(2-éthoxyéthoxy)-3'-(1-méthyl-3-pentyluréido)biphényl-4-yl]propanoïque
- du chlorhydrate de l'acide 3-[2-(2-diéthylaminoéthoxy)-3'-(3-heptyl-1-méthyluréido)biphényl-4-yl]propanoïque
- de l'acide 3-[3'-(3-heptyl-1-méthyluréido)-2-(2-morpholin-4-yléthoxy)biphényl-4-yl]propanoïque
- de l'acide 3-[2-amino-3'-(1-méthyl-3-pentyluréido)biphényl-4-yl]propanoïque
- du chlorhydrate de l'acide 3-[2-butylamino-3'-(1-méthyl-3-pentyluréido)biphényl-4-yl]propanoïque
- du chlorhydrate de l'acide 3-[2-benzylamino-3'-(1-méthyl-3-pentyluréido)biphényl-4-yl]propanoïque
- du chlorhydrate de l'acide 3-[2-diéthylamino-3'-(1-méthyl-3-pentyluréido)biphényl-4-yl]propanoïque
- du chlorhydrate de l'acide 3-[3'-(1-méthyl-3-pentyluréido)-2-propylaminobiphényl-4-yl]propanoïque
- du chlorhydrate de l'acide 3-[2-(4-fluorobenzylamino)-3'-(1-méthyl-3-pentyluréido)biphényl-4-yl]propanoïque
- du chlorhydrate de l'acide 3-[2-butylamino-3'-(3-heptyl-1-méthyluréido)biphényl-4-yl]propanoïque
- du chlorhydrate de l'acide 3-[2-benzylamino-3'-(3-heptyl-1-méthyluréido)biphényl-4-yl]propanoïque
- du chlorhydrate de l'acide 3-[2-diéthylamino-3'-(3-heptyl-1-méthyluréido)biphényl-4-yl]propanoïque
- du chlorhydrate de l'acide 3-[3'-(3-heptyl-1-méthyluréido)-2-propylaminobiphényl-4-yl]propanoïque
- du chlorhydrate de l'acide 3-[2-(4-fluorobenzylamino)-3'-(3-heptyl-1-méthyluréido)biphényl-4-yl]propanoïque
- de l'acide 3-[3'-(1-méthyl-3-pentyluréido)biphényl-4-yl]acrylique
- de l'acide 3-[3'-(3-pentyl-1-propyluréido)biphényl-4-yl]acrylique
- de l'acide 3-[4'-fluoro-3'-(1-méthyl-3-pentyluréido)biphényl-4-yl]propanoïque
- du 3-[3'-(1-méthyl-3-pentyluréido)biphényl-4-yl]acrylate d'éthyle
- de l'acide 3-[3'-(1-méthyl-3-naphtalén-2-yluréido)biphényl-4-yl]acrylique
- de l'acide 3-{3'-[3-(4-fluorophényl)-1-méthyluréido]biphényl-4-yl}acrylique
- de l'acide 3-(3'-{3-[2-(4-fluorophényl)éthyl]-1-méthyluréido}biphényl-4-yl)acrylique
- de l'acide 3-[3'-(3-benzyl-1-méthyluréido)biphényl-4-yl]propanoïque
- de l'acide 3-[3'-(3-cyclopropylméthyl-1-méthyluréido)biphényl-4-yl]propanoïque
- de l'acide 3-[3'-(3-cyclohexyl-1-méthyluréido)biphényl-4-yl]propanoïque
- de l'acide 3-[3'-(3-cyclohexyl-1-méthyluréido)biphényl-4-yl]acrylique
- de l'acide 3-[3'-(3-cyclopropylméthyl-1-méthyluréido)biphényl-4-yl]acrylique
- du 4-{3-[4'-(2-carboxyéthyl)biphényl-3-yl]-3-méthyluréido}pipéridine-1-carboxylate d'éthyle
- de l'acide 3-[3'-(1-méthyl-3-pyrid-3-yluréido)biphényl-4-yl]acrylique
- de l'acide 3-[3'-(1-méthyl-3-pyrid-3-yluréido)biphényl-4-yl]propanoïque
- de l'acide 3-{3'-[3-(6-méthoxypyrid-3-yl)-1-méthyluréido]biphényl-4-yl}acrylique
- de l'acide 3-[3'-(1-méthyl-3-propyluréido)biphényl-4-yl]acrylique
- de l'acide 3-[3'-(3-hexyl-1-méthylthiouréido)biphényl-4-yl]propanoïque
- de l'acide 3-[3'-(3-hexyl-1-méthylthiouréido)biphényl-4-yl]acrylique
- du 3-[3'-(3-heptyl-1-méthylthiouréido)biphényl-4-yl]acrylate de méthyle
- de l'acide 3-[2-méthyl-3'-(1-méthyl-3-pentyluréido)biphényl-4-yl]propanoïque
- de l'acide 3-[3-hydroxy-3'-(1-méthyl-3-pentyluréido)biphényl-4-yl]propanoïque
- de l'acide 3-[3-méthoxyméthoxy-3'-(1-méthyl-3-pentyluréido)biphényl-4-yl]propanoïque
- de l'acide 3-[3'-(1-méthyl-3-pentyluréido)-2-trifluorométhylbiphényl-4-yl]propanoïque
- de l'acide 3-[3'-(3-heptyl-1-méthyluréido)-3-méthoxybiphényl-4-yl]propanoïque
- de l'acide 3-{3'-[1-méthyl-3-(4-propylphényl)uréido]biphényl-4-yl}propanoïque
- de l'acide 3-{3'-[1-méthyl-3-(4-propylphényl)uréido]biphényl-4-yl}acrylique
- du 3-{3'-[1-méthyl-3-(4-propylphényl)uréido]biphényl-4-yl}acrylate de phényle
- du 3-{3'-[1-méthyl-3-(4-propylphényl)uréido]biphényl-4-yl}acrylate de benzyle
- de l'acide 3-[3'-(3-pentyluréido)biphényl-4-yl]acrylique
- du N-hydroxy-3-{3'-[1-méthyl-3-(3-phénylpropyl)uréido]biphényl-4-yl}propionamide
- de l'acide 3-[3'-(3-heptyl-1-méthyluréido)-2-hydroxybiphényl-4-yl]propanoïque
- de l'acide 3-[2-cyclohexylméthoxy-3'-(3-heptyl-1-méthyluréido)biphényl-4-yl]propionique
- de l'acide 3-[2-cyclopentylméthoxy-3'-(3-heptyl-1-méthyluréido)biphényl-4-yl]propionique
- du 3-[2-cyclobutylméthoxy-3'-(3-heptyl-1-méthyluréido)biphényl-4-yl]-N-hydroxypropionamide
- de l'acide 3-[3'-(3-heptyl-1-méthyluréido)-2-(3-trifluorométhylbenzyloxy)biphényl-4-yl]propanoïque
- de l'acide 3-[3'-(3-heptyl-1-méthyluréido)-2-(4-trifluorométhylbenzyloxy)biphényl-4-yl]propanoïque
- de l'acide 3-[2-(3-carbamoylbenzyloxy)-3'-(3-heptyl-1-méthyluréido)biphényl-4-yl]propanoïque
- de l'acide 3-[3'-(3-heptyl-1-méthyluréido)-2-(2-pipérazin-1-yléthoxy)biphényl-4-yl]propanoïque
- de l'acide 3-[3'-(3-heptyl-1-méthyluréido)-2-(2-pyrrolidin-1-yléthoxy)biphényl-4-yl]propanoïque
- de l'acide 3-(2-(3-méthoxybenzyloxy)-3'-{3-[2-(3-méthoxyphényl)éthyl]-1-méthyluréido}biphényl-4-yl)propanoïque
- de l'acide 3-(2-(4-tert-butylbenzyloxy)-3'-{1-méthyl-3-[2-(3-phénoxyphényl)éthyl]uréido}biphényl-4-yl)propanoïque
- de l'acide 3-{2-(3,5-diméthoxybenzyloxy)-3'-[1-méthyl-3-(3-phénoxyphényl)uréido]biphényl-4-yl}propanoïque
- de l'acide 3-[3'-[1-méthyl-3-(4-phénoxyphényl)uréido]-2-(3-trifluorométhylbenzyloxy)biphényl-4-yl]propanoïque
- de l'acide 3-(2-(3-isopropoxybenzyloxy)-3'-{3-[2-(4-méthoxyphényl)éthyl]-1-méthyluréido}biphényl-4-yl)propanoïque
- de l'acide 3-(2'-(3-méthoxybenzyloxy)-5'-{3-[2-(3-méthoxyphényl)éthyl]-1-méthyluréido}biphényl-4-yl)propanoïque
- de l'acide 3-[2'-cyclohexylméthoxy-5'-(1-méthyl-3-pentyluréido)biphényl-4-yl]propanoïque
- de l'acide 3-[4'-éthoxy-3'-(1-méthyl-3-pentyluréido)-2-propoxybiphényl-4-yl]propanoïque
- de l'acide 3-(3,5-diméthoxy-3'-(1-méthyl-3-pentyluréido)biphényl-4-yl]propanoïque
- de l'acide 3-(3,5-diéthoxy-3'-{3-[2-(3-méthoxyphényl)éthyl]-1-méthyluréido}biphényl-4-yl)propanoïque
- de l'acide 3-(3'-{3-[2-(4-méthoxyphényl)éthyl]-1-méthyluréido}-3-propoxybiphényl-4-yl)propanoïque
- de l'acide 3-[3-cyclopropylméthoxy-3'-(1-méthyl-3-pentyluréido)biphényl-4-yl]propanoïque
- de l'acide 3-[3-éthoxy-4'-fluoro-3'-(1-méthyl-3-pentyluréido)biphényl-4-yl]propanoïque
- de l'acide 3-[3'-(1-méthyl-3-pentyluréido)-3-(4,4,4-trifluorobutoxy)biphényl-4-yl]propanoïque
- de l'acide 3-[3-benzyloxy-3'-(1-méthyl-3-pentyluréido)biphényl-4-yl]propanoïque
- de l'acide 3-[3'-{3-[2-(4-méthoxyphényl)éthyl]-1-méthyluréido}-3-(3-trifluorométhylbenzyloxy)biphényl-4-yl]propanoïque
- de l'acide 3-(3'-{3-[2-(3-méthoxyphényl)éthyl]-1-méthyluréido}-3,5-dipropylbiphényl-4-yl)propanoïque
- de l'acide 3-[3-(2,2-diméthylpropyl)-3'-(1-méthyl-3-pentyluréido)biphényl-4-yl]propanoïque
- de l'acide 3-[3,5-diméthyl-3'-(1-méthyl-3-pentyluréido)biphényl-4-yl]propanoïque
- de l'acide 3-[4''-méthoxy-3-(1-méthyl-3-pentyluréido)[1,1';3',1'']terphényl-4'-yl]propanoïque
- de l'acide 3-[3''-méthoxy-3-(1-méthyl-3-pentyluréido)[1,1';2',1'']terphényl-4'-yl]propanoïque
- de l'acide 3-(3-{3-[2-(3-méthoxyphényl)éthyl]-1-méthyluréido}-3"-trifluorométhyl[1,1';2',1"]terphényl-4'-yl)propanoïque
- de l'acide 3-{3'-(3-butyl-1-méthyluréido)-2-[2-(3-isopropoxyphényl)éthyl]biphényl-4-yl}propanoïque
- de l'acide 3-{3'-(1-méthyl-3-pentyluréido)-2-[(pyrid-3-ylméthyl)amino]biphényl-4-yl}propanoïque
- de l'acide 3-[3-(2-méthoxyéthylamino)-3'-(1-méthyl-3-pentyluréido)biphényl-4-yl]propanoïque
- du 3-[3,5-diéthyl-3'-(1-méthyl-3-pentyluréido)biphényl-4-yl]propanoate de méthyle
- du 3-[3'-[1-méthyl-3-(3-phénoxyphényl)uréido]-2-(3-trifluorométhylbenzyloxy)biphényl-4-yl]propanoate de méthyle
- du 3-[3'-[3-(2-biphényl-4-yléthyl)-1-méthyluréido]-2-(3-méthoxybenzyloxy)biphényl-4-yl]propanoate de méthyle
- du 3-[3'-{3-[2-(3-méthoxyphényl)éthyl]-1-méthyluréido}-2-(4,4,4-trifluorobutoxy)biphényl-4-yl]propanoate d'éthyle.

14. Composés selon la revendication 1, 2 ou 3, **caractérisés en ce qu'**ils présentent au moins l'une des caractéristiques suivantes :
- R1 représente un radical hydroxyle ou un radical hydroxylamine ;
- R2 et R7 représentent un radical alcoxy ou un radical aryloxy, un radical alkylamino ou un radical polyéther ;
- R3 et R8 représentent un atome d'hydrogène ;
- R4 représente un radical alkyle inférieur renfermant de 1 à 4 atomes de carbone ;
- R5 représente un radical alkyle renfermant de 3 à 8 atomes de carbone ;
- Y est un atome d'oxygène ;
- la liaison V-W est un groupe CH2-CH2 ou un groupe CH=CH.

15. Composés selon la revendication 14, **caractérisés en ce qu'**ils présentent toutes les caractéristiques suivantes :
- R1 représente un radical hydroxyle ou un radical hydroxylamine ;
- R2 et R7 représentent un radical alcoxy ou un radical aryloxy, un radical alkylamino ou un radical polyéther ;
- R3 et R8 représentent un atome d'hydrogène ;
- R4 représente un radical alkyle inférieur renfermant de 1 à 4 atomes de carbone ;
- R5 représente un radical alkyle renfermant de 3 à 8 atomes de carbone ;
- Y est un atome d'oxygène ;
- la liaison V-W est un groupe CH2-CH2 ou un groupe CH=CH.

16. Composition cosmétique, **caractérisée en ce qu'**elle comprend, dans un support physiologiquement acceptable, au moins l'un des composés tels que définis dans l'une quelconque des revendications 1 à 15.

17. Composition selon la revendication 16,
**caractérisée en ce que** la concentration en composé(s) selon l'une des revendications 1 à 15 est comprise entre 0,001 % et 3 % en poids, par rapport au poids total de la composition.

18. Utilisation cosmétique d'une composition telle que définie dans l'une ou l'autre des revendications 16 et 17, pour l'hygiène du corps ou des cheveux.

19. Composés selon l'une quelconque des revendications 1 à 15 en tant que médicaments.

20. Utilisation d'un composé selon l'une quelconque des revendications 1 à 15 pour la fabrication d'une composition destinée à réguler et/ou à rétablir le métabolisme lipidique de la peau.

21. Utilisation d'un composé selon l'une quelconque des revendications 1 à 15 pour la fabrication d'une composition destinée à traiter :
- des affections dermatologiques associées à un trouble de kératinisation lié à la différenciation et à la prolifération cellulaires, en particulier pour le traitement de l'acné vulgaire, de comédons, de granulocytes, de l'acné rosacée, de l'acné nodulo-kystique, de l'acné conglobata, de l'acné sénile et d'acnés secondaires telles que l'acné solaire, l'acné liée à un médicament ou l'acné professionnelle ;
- une ichtyose, des affections ichtyosiformes, la maladie de Darier, la kératodermie palmoplantaire, la leucoplasie et des affections leucoplasiformes, et le lichen cutané ou muqueux (buccal) ;
- des affections dermatologiques avec une composante immuno-allergique inflammatoire avec
ou sans trouble de la prolifération cellulaire, en particulier le psoriasis cutané, muqueux ou unguéal, le rhumatisme psoriatique, une atopie cutanée, telle que l'eczéma, une atopie respiratoire ou une hypertrophie gingivale ;
- des proliférations dermiques ou épidermiques, qu'elles soient bénignes ou malignes, et qu'elles soient d'origine virale ou autre, en particulier des verrues vulgaires, des verrues planes et une épidermodysplasie verriciforme, des papillomatoses orales ou développées ou un lymphome T ;
- des proliférations qui peuvent être induites par un rayonnement ultraviolet, en particulier un épithélioma de cellules basales et de cellules spinales ;
- des lésions cutanées pré-cancéreuses, en particulier des kératoacanthomes ;
- des dermatoses immunes, en particulier le lupus érythémateux ;
- des maladies bulleuses immunes ;
- des maladies du collagène, en particulier un scléroderme ;
- des affections dermatologiques ou générales avec une composante immunologique ;
- des troubles cutanés causés par une exposition à un rayonnement UV, un vieillissement photo-induit ou chronologique de la peau, des pigmentations actiniques et une kératose, ou toute pathologie associée à un vieillissement chronologique ou actinique, en particulier une xérose ;
- des troubles de la fonction sébacée, en particulier l'hyperséborrhée de l'acné ou une séborrhée simple ;
- des troubles de cicatrisation ou des vergetures ;
- des troubles de pigmentation, telles qu'une hyperpigmentation, un mélasme, une hypopigmentation ou un vitiligo ;
- des affections du métabolisme lipidique, tels que l'obésité, l'hyperlipidémie ou le diabète non insulino-dépendant ;
- des troubles inflammatoires tels que l'arthrite ;
- des affections cancéreuses ou pré-cancéreuses ;
- l'alopécie de diverses origines, en particulier une alopécie causée par une chimiothérapie ou un rayonnement ;
- des troubles du système immunitaire, tels que l'asthme, le diabète sucré du type I, la sclérose en plaques ou d'autres dysfonctionnements sélectifs du système immunitaire ; et
- des affections du système cardiovasculaire, telles que l'artériosclérose ou l'hypertension.

22. Composition pharmaceutique, **caractérisée en ce qu'**elle comprend, dans un support physiologiquement acceptable, au moins l'un des composés tels que définis dans l'une quelconque des revendications 1 à 15.

23. Composition selon la revendication 22, **caractérisée en ce que** la concentration en composé(s) selon l'une des revendications 1 à 15 est comprise entre 0,001 % et 10 % en poids, par rapport au poids total de la composition.

24. Composition selon la revendication 22, **caractérisée en ce que** la concentration en composé(s) selon l'une des revendications 1 à 15 est comprise entre 0,01 % et 1 % en poids, par rapport au poids total de la composition.
